# EUROPEAN PATENT APPLICATION

(11) **EP 2 527 344 A1**
(43) Date of publication of application: **28.11.2012**
(21) Application number: 11382170.6
(22) Date of filing: 25.05.2011
(51) Int. Cl.: C07D 471/02, C07D 473/00, A61K 31/52, A61P 35/00, A61P 37/06

(54) **Pyridin-2(1H)-one derivatives useful as medicaments for the treatment of myeloproliferative disorders, transplant rejection, immune-mediated and inflammatory diseases**

(71) Applicant: Almirall, S.A., 08022 Barcelona (ES)
(72) Inventor: Eastwood, Paul Robert, 08980 Sant Feliú de Llobregat (ES); Gonzales Rodriguez, Jacob, 08980 Sant Feliú de Llobregat (ES); Gomez Castillo, Elena, 08980 Sant Feliú de Llobregat (ES); Bach Taña, Mr Jordi, 08980 Sant Feliú de Llobregat (ES)
(74) Representative: Elzaburu Marquez, Alberto

(57) **Abstract**

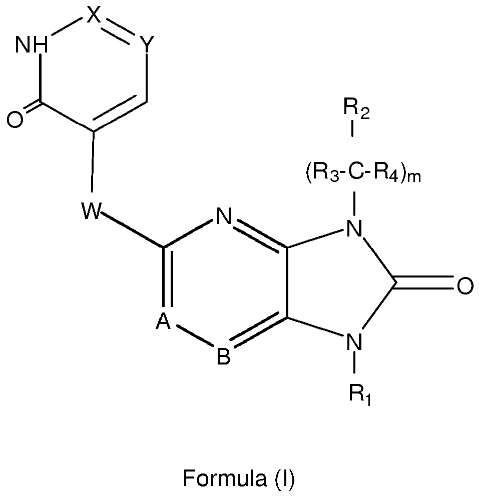

New pyridin-2(1h)-one derivatives having the chemical structure of formula (I) are disclosed; as well as process for their preparation, pharmaceutical compositions comprising them and their use in therapy as inhibitors of Janus Kinases (JAK).

## Description

Cytokines have critical functions in regulating many aspects of immunity and inflammation, ranging from the development and differentiation of immune cells to the suppression of immune responses. Type I and type II cytokine receptors lack intrinsic enzymatic activity capable of mediating signal transduction, and thus require association with tyrosine kinases for this purpose. The JAK family of kinases comprises four different members, namely JAK1, JAK2, JAK3 and TYK2, which bind to type I and type II cytokine receptors for controlling signal transduction (Murray PJ, (2007). The JAK-STAT signalling pathway: input and output integration. J Immunol, 178: 2623). Each of the JAK kinases is selective for the receptors of certain cytokines. In this regard, JAK-deficient cell lines and mice have validated the essential role of each JAK protein in receptor signalling: JAK1 in class II cytokine receptors (IFN and IL-10 family), those sharing the gp130 chain (IL-6 family) and the common gamma chain (IL-2, IL-4, IL-7, IL-9, IL- 15 and IL-21) (Rodig et al. (1998). Disruption of the JAK1 gene demonstrates obligatory and nonredundant roles of the Jaks in cytokine-induced biological response. Cell, 93:373; Guschin et al. (1995). A major role for the protein tyrosine kinase JAK1 in the JAK/STAT signal transduction pathway in response to interleukin-6. EMBO J. 14: 1421; Briscoe et al. (1996). Kinase-negative mutants of JAK1 can sustain intereferon-gamma-inducible gene expression but not an antiviral state. EMBO J. 15:799); JAK2 in hematopoietic factors (Epo, Tpo, GM-CSF, IL-3, IL-5) and type II IFNs (Parganas et al., (1998). JAK2 is essential for signalling through a variety of cytokine receptors. Cell, 93:385); JAK3 in receptors sharing the common gamma chain (IL-2 family) (Park et al., (1995). Developmental defects of lymphoid cells in JAK3 kinase-deficient mice. Immunity, 3:771; Thomis et al., (1995). Defects in B lymphocyte maturation and T lymphocyte activation in mice lacking JAK3. Science, 270:794; Russell et al., (1995). Mutation of JAK3 in a partient with SCID: Essential role of JAK3 in lymphoid development. Science, 270:797); and Tyk2 in the receptors of IL-12, IL-23, IL-13 and type I IFNs (Karaghiosoff et al., (2000). Partial impairment of cytokine responses in Tyk2-deficient mice. Immunity, 13:549; Shimoda et al., (2000). Tyk2 plays a restricted role in IFNg signaling, although it is required for IL-12-mediated T cell function. Immunity, 13:561; Minegishi et al., (2006). Human Tyrosine kinase 2 deficiency reveals its requisite roles in multiple cytokine signals involved in innate and acquired immunity. Immunity, 25:745).

Receptor stimulation leads sequentially to JAK activation by phosphorylation, receptor phosphorylation, STAT protein recruitment and STAT activation and dimerization. The

STAT dimer then functions as a transcription factor, translocating to the nucleus and activating the transcription of multiple response genes. There are seven STAT proteins identified: STAT1, STAT2, STAT3, STAT4, STAT5a, STAT5b and STAT6. Each particular cytokine receptor associates preferentially with a particular STAT protein. Some associations are independent of cell type (ex: IFNg- STAT1) while others may be cell type dependent (Murray PJ, (2007). The JAK-STAT signaling pathway: input and output integration. J Immunol, 178: 2623).

The phenotype of deficient mice has provided insights on the function of each JAK and the cytokine receptors signaling through them. JAK3 associates exclusively with the common gamma chain of the receptors for IL-2, IL-4, IL-7, IL-9, IL-15 and IL-21 cytokines. By virtue of this exclusive association, JAK3 knock out mice and common gamma chain deficient mice have an identical phenotype (Thomis et al., (1995). Defects in B lymphocyte maturation and T lymphocyte activation in mice lacking JAK3. Science, 270:794; DiSanto et al., (1995). Lymphoid development in mice with a targeted deletion of the interleukin 2 receptor gamma chain. PNAS, 92:377). Moreover, this phenotype is shared to a great extent with SCID patients that hold mutations/defects in the common gamma chain or JAK3 genes (O'Shea et al., (2004). JAK3 and the pathogenesis of severe combined immunodeficiency. Mol Immunol, 41: 727). JAK3-deficient mice are viable but display abnormal lymphopoiesis which leads to a reduced thymus size (10-100 fold smaller than wild type). JAK3-deficient peripheral T cells are unresponsive and have an activated/memory cell phenotype (Baird et al., (1998). T cell development and activation in JAK3-deficient mice. J. Leuk. Biol. 63: 669). The thymic defect in these mice strongly resembles that seen in IL-7 and IL-7 receptor knockout mice, suggesting that the absence of IL-7 signaling accounts for this defect in JAK3 -/-mice (von Freeden-Jeffry et al., (1995). Lymphopenia in Interleukin (IL)-7 Gene-deleted Mice Identifies IL-7 as a non-redundant Cytokine. J Exp Med, 181:1519; Peschon et al, (1994). Early lymphocyte expansion is severely impaired in interleukin 7 receptor-deficient mice. J Exp Med, 180: 1955). These mice, like SCID humans, have no NK cells, probably due to the absence of IL-15 signaling, a survival factor for these cells. JAK3 knockout mice, unlike SCID patients, show deficient B cell lymphopoiesis while in human patients, B cells are present in circulation but are not responsive leading to hypoglobulinemia (O'Shea et al., (2004). JAK3 and the pathogenesis of severe combined immunodeficiency. Mol Immunol, 41: 727). This is explained by species-specific differences in IL-7 function in B and T cell development in mice and humans. On the other hand, Grossman et al. (1999. Dysregulated myelopoiesis in mice lacking JAK3. Blood, 94:932-939) have shown that the loss of JAK3 in the T-cell compartment drives the expansion of the myeloid lineages leading to dysregulated myelopoiesis.

JAK2-deficient mice are embrionically lethal, due to the absence of definitive erythropoiesis. Myeloid progenitors fail to respond to Epo, Tpo, IL-3 or GM-CSF, while G-CSF and IL-6 signaling are not affected. JAK2 is not required for the generation, amplification or functional differentiation of lymphoid progenitors (Parganas et al., (1998). JAK2 is essential for signaling through a variety of cytokine receptors. Cell, 93:385).

JAK1-deficient mice die perinatally due to a nursing defect. JAK1 binds exclusively to the gp130 chain shared by the IL-6 cytokine family (i.e. LIF, CNTF, OSM, CT-1) and along with JAK3, is an essential component of the receptors sharing the common gamma chain, by binding to the non-shared receptor subunit. In this regard, JAK1-deficient mice show similar hematopoiesis defects as JAK3-deficient mice. In addition, they show defective responses to neurotrophic factors and to all interferons (class II cytokine receptors) (Rodig et al., (1998). Disruption of the JAK1 gene demonstrates obligatory and non-redundant roles of the JAKs in cytokine-induced biological response. Cell, 93:373).

Finally, Tyk2-deficient mice show an impaired response to IL-12 and IL-23 and only partially impaired to IFN-alpha (Karaghiosoff et al., (2000). Partial impairment of cytokine responses in Tyk2-deficient mice. Immunity, 13:549; Shimoda et al., (2000). Tyk2 plays a restricted role in IFNg signaling, although it is required for IL-12-mediated T cell function. Immunity, 13:561). However, human Tyk2 deficiency demonstrates that Tyk2 is involved in the signaling from IFN-α, IL-6, IL-10, IL-12 and IL-23 (Minegishi et al., (2006). Human Tyrosine kinase 2 deficiency reveals its requisite roles in multiple cytokine signals involved in innate and acquired immunity. Immunity, 25:745).

The role of JAK kinases in transducing the signal from a myriad of cytokines makes them potential targets for the treatment of diseases in which cytokines have a pathogenic role, such as inflammatory diseases, including but not limited to allergies and asthma, chronic obstructive pulmonary disease (COPD), psoriasis, autoimmune diseases such as rheumatoid arthritis, amyotrophic lateral sclerosis and multiple sclerosis, uveitis, transplant rejection, as well as in solid and hematologic malignancies such as myeloproliferative disorders, leukemia and lymphomas.

Inhibition of JAK kinases, especially JAK1 and JAK3, could give rise to potent immunosuppression which could be used therapeutically to prevent transplant rejection. In this regard, the JAK inhibitor CP-690,550 (tasocitinib) has shown efficacy in several animal models of transplantation (heretopic heart transplantation in mice, cardiac allografts implanted in the ear of mice, renal allotransplantation in cynomolgous monkeys, aorta and tracheal transplantation in rats) by prolonging the mean survival time of grafts (West K (2009). CP-690,550, a JAK3 inhibitor as an immunosuppressant for the treatment of rheumatoid arthritis, transplant rejection, psoriasis and other immune-mediated disorders. Curr. Op. Invest. Drugs 10: 491).

In rheumatoid joints, an imbalance between pro and anti-inflammatory cytokine activities favours the induction of autoimmunity, followed by chronic inflammation and tissue destruction. In this regard, the pathogenic role of IL-6 in rheumatoid arthritis (RA) has been validated clinically by the use of the anti-IL-6R antibody tocilizumab. IL-6 activates the transcription factor STAT3, through the use of JAK1 binding to the gp130 receptor chain (Heinrich et al., (2003). Principles of interleukin (IL)-6-type cytokine signaling and its regulation. Biochem J. 374: 1). Constitutive STAT3 mediates the abnormal growth and survival properties of RA synoviocytes (Ivashkiv and Hu (2003). The JAK/STAT pathway in rheumatoid arthritis: pathogenic or protective? Arth & Rheum. 48:2092). Other cytokines that have been implicated in the pathogenesis of arthritis include IL-12 and IL-23, implicated in Th1 and Th17 cell proliferation, respectively; IL-15, and GM-CSF (McInnes and Schett, (2007). Cytokines in the pathogenesis of rheumatoid arthritis. Nature Rew Immunol. 7:429.). The receptors for these cytokines also utilize JAK proteins for signal transduction, making JAK inhibitors potential pleiotropic drugs in this pathology. Consequently, administration of several JAK inhibitors in animal models of murine collagen-induced arthritis and rat adjuvant-induced arthritis has shown to reduce inflammation, and tissue destruction (Milici et al., (2008). Cartilage preservation by inhibition of Janus kinase 3 in two rodent models of rheumatoid arthritis. Arth. Res. 10:R14).

Inflammatory bowel disease (IBD) encloses two major forms of intestinal inflammation: ulcerative colitis and Crohn's disease. Growing evidence has shown that multiple cytokines, including interleukins and interferons, are involved in the pathogenesis of IBD (Strober et al, (2002). The immunology of mucosal models of inflammation. Annu Rev Immunol. 20: 495). Activation of the IL-6/STAT3 cascade in lamina propia T cells has been shown to induce prolonged survival of pathogenic T cells (Atreya et al, (2000). Blockade of interleukin 6 trans signaling suppresses T-cell resistance against apoptosis in chronic intestinal inflammation: Evidence in Crohn's disease and experimental colitis in vivo. Nature Med. 6:583). Specifically, STAT3 has been shown to be constitutively active in intestinal T cells of Crohn's disease patients and a JAK inhibitor has been shown to block the constitutive activation of STAT3 in these cells (Lovato et al, (2003). Constitutive STAT3 activation in intestinal T cells from patients with Crohn's disease. J Biol Chem. 278:16777). These observations indicate that the JAK-STAT pathway plays a pathogenic role in IBD and that a JAK inhibitor could be therapeutic in this setting.

Multiple sclerosis is an autoimmune demyelinating disease characterized by the formation of plaques in the white matter. The role of cytokines in the generation of multiple sclerosis has long been known. Potential therapies include blockade of IFN-g, IL-6, IL-12 and IL-23 (Steinman L. (2008). Nuanced roles of cytokines in three major human brain disorders. J Clin Invest. 118:3557), cytokines that signal through the JAK-STAT pathways. Use of tyrphostin, a JAK inhibitor, has been shown to inhibit IL-12-induced phosphorylation of STAT3, and to reduce the incidence and severity of active and passive experimental autoimmune encephalitis (EAE) (Bright et al., (1999) Tyrphostin B42 inhibits IL-12-induced tyrosine phosphorylation and activation of Janus kinase-2 and prevents experimental allergic encephalomyelitis. J Immunol. 162:6255). Another multikinase inhibitor, CEP701, has been shown to reduce secretion of TNF-alpha, IL-6 and IL-23 as well as the levels of phospho-STAT1, STAT3, and STAT5 in peripheral DCs of mice with EAE, significantly improving the clinical course of EAE in mice (Skarica et al, (2009). Signal transduction inhibition of APCs diminishes Th17 and Th1 responses in experimental autoimmune encephalomyelitis. J. Immunol. 182:4192.).

Psoriasis is a skin inflammatory disease which involves a process of immune cell infiltration and activation that culminates in epithelial remodeling. The current theory behind the cause of psoriasis states the existence of a cytokine network that governs the interaction between immune and epithelial cells (Nickoloff BJ. (2007). Cracking the cytokine code in psoriasis, Nat Med, 13:242). In this regard, IL-23 produced by dendritic cells is found elevated in psoriatic skin, along with IL-12. IL-23 induces the formation of Th17 cells which in turn produce IL-17 and IL-22, the last one being responsible for epidermis thickening. IL-23 and IL-22 induce the phosphorylation of STAT-3, which is found abundantly in psoriatic skin. JAK inhibitors may thus be therapeutic in this setting. In accordance, a JAK1/3 inhibitor, R348, has been found to attenuate psoriasiform skin inflammation in a spontaneous T cell-dependent mouse model of psoriasis (Chang et al., (2009). JAK3 inhibition significantly attenuates psoriasiform skin inflammation on CD18 mutant PL/J mice. J Immunol. 183:2183).

Th2 cytokine-driven diseases such as allergy and asthma could also be a target of JAK inhibitors. IL-4 promotes Th2 differentiation, regulates B-cell function and immunoglobulin class switching, regulates eotaxin production, induces expression of IgE receptor and MHC II on B cells, and stimulates mast cells. Other Th2 cytokines like IL-5 and IL-13 can also contribute to eosinophil recruitment in bronchoalveolar lavage by stimulating eotaxin production. Pharmacological inhibition of JAK has been shown to reduce the expression of IgE receptor and MHCII induced by IL-4 stimulation on B cells (Kudlacz et al., (2008). The JAK3 inhibitor CP-690,550 is a potent anti-inflammatory agent in a murine model of pulmonary eosinophilia. European J. Pharm. 582: 154). Furthermore, JAK3-deficient mice display poor eosinophil recruitment and mucus secretion to the airway lumen upon OVA challenge, as compared to wild type mice (Malaviya et al, (2000). Treatment of allergic asthma by targeting Janus kinase 3-dependent leukotriene synthesis in mast cells with 4-(3', 5'- dibromo-4'-hydroxyphenyl)amino-6,7-dimethoxyquinazoline (WHI-P97). JPET295:912.). In this regard, systemic administration of the CP-690,550 JAK inhibitor in mice has been shown to reduce the eosinophil count as well as the levels of eotaxin and IL13 in BAL in a murine model of pulmonary eosinophilia (Kudlacz et al., (2008). The JAK3 inhibitor CP-690,550 is a potent anti-inflammatory agent in a murine model of pulmonary eosinophilia. European J. Pharm. 582:154).

There is increasing evidence that cytokines play a pathogenetic role in ocular inflammatory disease such as uveitis or dry eye syndrome. Some cytokines implicated in experimental autoimmune uveitis, such as IL-2, IL-6, IL-12 and IFNg, would be amenable to JAK inhibition (Vallochi et al, (2007). The role of cytokines in the regulation of ocular autoimmune inflammation. Cytok Growth Factors Rev. 18:135). In this regard, drugs or biologicals that interfere with IL-2 signaling such as cyclosporine or anti-IL-2 receptor antibody (daclizumab) have shown efficacy in the treatment of keratoconjuctivitis sicca and refractory uveitis, respectively (Lim et al, (2006). Biologic therapies for inflammatory eye disease. Clin Exp Opht 34:365). Similarly, allergic conjunctivitis, a common allergic eye disease characterized by conjuctival congestion, mast cell activation and eosinophil infiltration, could benefit from JAK inhibition. STAT6-deficient mice, showing decreased TH2-mediated immune responses which are normally triggered by IL-4, do not develop the classical early and late phase responses, suggesting that IL-4 pathway abrogation through JAK inhibition may be therapeutic in this setting (Ozaki et al, (2005). The control of allergic conjunctivitis by suppression of cytokine signaling (SOCS)3 and SOCS5 in a murine model. J Immunol, 175:5489).

There is growing evidence of the critical role of STAT3 activity in processes involved in tumorigenesis like cell cycle dysregulation, promotion of uncontrolled growth, induction of survival factors and inhibition of apoptosis (Siddiquee et al., (2008). STAT3 as a target for inducing apoptosis in solid and haematological tumors. Cell Res. 18: 254). Antagonism of STAT3 by means of dominant-negative mutants or antisense oligonucleotides has shown to promote apoptosis of cancer cells, inhibition of angiogenesis and up-regulation of host immunocompetence. Inhibition of constitutively active STAT3 in human tumors by means of JAK inhibitors may provide a therapeutic option to the treatment of this disease. In this regard, the use of the JAK inhibitor tyrphostin has been shown to induce apoptosis of malignant cells and inhibit cell proliferation in vitro and in vivo (Meydan et al., (1996). Inhibition of acute lymphoblastic leukemia by a JAK-2 inhibitor. Nature, 379:645).

Hematological malignancies with dysregulated JAK-STAT pathways may benefit from JAK inhibition. Recent studies have implicated dysregulation of JAK2 kinase activity by chromosomal translocations and mutations within the pseudokinase domain (such as the JAK2V617F mutation) in a spectrum of myeloproliferative diseases (Ihle and Gililand, 2007), including polycythemia vera, myelofibrosis and essential thrombocythemia. In this regard, several JAK inhibitors that tackle JAK2 potently, such as TG-101209 (Pardanani et al., (2007). TG101209, a small molecular JAK2-selective inhibitor potently inhibits myeloproliferative disorder-associated JAK2V617F and MPLW515L/K mutations Leukemia. 21:1658-68), TG101348 (Wernig et al, (2008). Efficacy of TG101348, a selective JAK2 inhibitor, in treatment of a murine model of JAK2V617F-induced polycythemia vera. Cancer Cell, 13: 311), CEP701, (Hexner et al, (2008). Lestaurtinib (CEP701) is a JAK2 inhibitor that suppresses JAK2/STAT5 signaling and the proliferation of primary erythroid cells from patients with myeloproliferative disorders. Blood, 111: 5663), CP-690,550 (Manshouri et al, (2008). The JAK kinase inhibitor CP-690,550 suppresses the growth of human polycythemia vera cells carrying the JAK2V617F mutation. Cancer Sci, 99:1265), and CYT387 (Pardanani et al., (2009). CYT387, a selective JAK1/JAK2 inhibitor: invitro assessment of kinase selectivity and preclinical studies using cell lines and primary cells from polycythemia vera patients. Leukemia, 23:1441) have been proposed for treating myeloproliferative diseases on the basis of their antiproliferative activity on cells carrying the JAK2V617F mutation. Similarly, T-cell leukemia due to human T-cell leukemia virus (HTLV-1) transformation is associated with JAK3 and STAT5 constitutive activation (Migone et al, (1995). Constitutively activated JAK-STAT pathway in T cells transformed with HTLV-I. Science, 269: 79) and JAK inhibitors may be therapeutic in this setting (Tomita et al, (2006). Inhibition of constitutively active JAK-STAT pathway suppresses cell growth of human T-cell leukemia virus type I-infected T cell lines and primary adult T-cell leukemia cells. Retrovirology, 3:22). JAK1-activating mutations have also been identified in adult acute lymphoblastic leukemia of T cell origin (Flex et al, (2008). Somatically acquired JAK1 mutations in adult acute lymphoblastic leukemia. J. Exp. Med. 205:751-8) pointing to this kinase as a target for the development of novel antileukemic drugs.

Conditions in which targeting of the JAK pathway or modulation of the JAK kinases, particularly JAK1, JAK2 and JAK3 kinases, are contemplated to be therapeutically useful for the treatment or prevention of diseases include: neoplastic diseases (e.g. leukemia, lymphomas, solid tumors); transplant rejection, bone marrow transplant applications (e.g., graft- versus-host disease); autoimmune diseases (e.g. diabetes, multiple sclerosis, rheumatoid arthritis, inflammatory bowel disease); respiratory inflammation diseases (e.g. asthma, chronic obstructive pulmonary disease), inflammation-linked ocular diseases or allergic eye diseases (e.g. dry eye, glaucoma, uveitis, diabetic retinopathy, allergic conjunctivitis or age-related macular degeneration) and skin inflammatory diseases (e.g., atopic dermatitis or psoriasis).

In view of the numerous conditions that are contemplated to benefit by treatment involving modulation of the JAK pathway or of the JAK Kinases it is immediately apparent that new compounds that modulate JAK pathways and use of these compounds should provide substantial therapeutic benefits to a wide variety of patients.

Provided herein are novel pyridin-2(1H)-one derivatives for use in the treatment of conditions in which targeting of the JAK pathway or inhibition of JAK kinases can be therapeutically useful.

The compounds described in the present invention are simultaneously potent JAK1, JAK2 and JAK3 inhibitors, i.e. pan-JAK inhibitors. This property makes them useful for the treatment or prevention of pathological conditions or diseases such as myeloproliferative disorders (such as polycythemia vera, essential thrombocythemia or myelofibrosis), leukemia, lymphomas and solid tumors; bone marrow and organ transplant rejection; immune-mediated diseases and inflammatory diseases, including rheumatoid arthritis, multiple sclerosis, inflammatory bowel disease (such as ulcerative colitis or Crohn's disease), inflammation-linked ocular diseases or allergic eye diseases (such as dry eye, uveitis, or allergic conjunctivitis), allergic rhinitis, asthma, chronic obstructive pulmonary disease (COPD), and skin inflammatory diseases (such as atopic dermatitis or psoriasis).

It has now been found that certain pyridin-2(1 H)-one derivatives are novel and potent JAK inhibitors and can therefore be used in the treatment or prevention of these diseases.

Thus the present invention is directed to compounds of formula (I), or a pharmaceutically acceptable salt, or solvate, or N-oxide, or stereoisomer or deuterated derivative thereof: 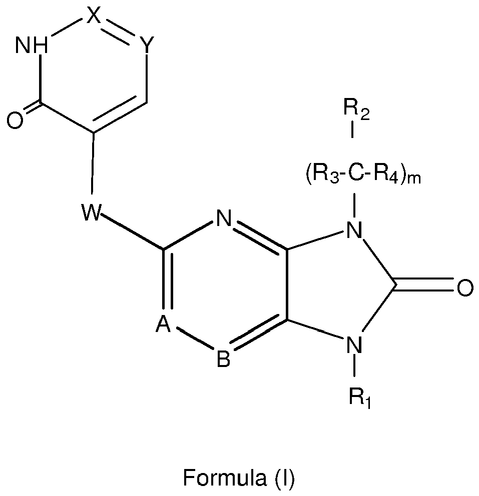 wherein,
m is 0 or an integer from 1 to 3;
X and Y each independently represent a nitrogen atom or a -CR₅ group, wherein at least one of X and Y represents a -CR₅ group;
A and B each independently represent a nitrogen atom or a -CR₆ group, wherein at least one of A and B represents a -CR₆ group;
W represents a linker selected from a -NR₇- group, a -(CR₈R₉)- group, -O- or -S-;
R₁ represents a hydrogen atom, a linear or branched C₁-C₆ alkyl group, a C₁-C₄ haloalkyl group, a C₁-C₄ hydroxyalkyl group, a C₁-C₄ alkoxy group, a C₃-C₁₀ cycloalkyl group, a C₃-C₁₀ cycloalkenyl group, a monocyclic or bicyclic C₆-C₁₄ aryl group, a 5- to 14- membered heteroaryl group containing at least one heteroatom selected from O, S and N, or a 5- to 14- membered heterocyclyl group containing at least one heteroatom selected from O, S and N,
wherein the cycloalkyl, cycloalkenyl, aryl, heteroaryl, and heterocyclyl groups are unsubstituted or substituted by one or more substituents selected from a halogen atom, a cyano group, a linear or branched C₁-C₆ alkyl group, a C₁-C₄ haloalkyl group, a C₁-C₄ hydroxyalkyl group, a C₁-C₄ alkylsulfonyl group, a C₃-C₇ cycloalkyl group, a phenyl group, a pyridyl group, a pyrimidinyl group or a piperidyl group;
R₂ and R₆ each independently represent a hydrogen atom, a halogen atom, a cyano group, a linear or branched C₁-C₆ alkyl group, a C₁-C₄ haloalkyl group, a C₁-C₄ hydroxyalkyl group, a C₃-C₁₀ cycloalkyl group, a C₃-C₁₀ cycloalkenyl group, a monocyclic or bicyclic C₆-C₁₄ aryl group, a 5- to 14- membered heteroaryl group containing at least one heteroatom selected from O, S and N, a 5- to 14- membered heterocyclyl group containing at least one heteroatom selected from O, S and N, or a bicyclyl group which is a monocyclic C₆-C₉ aryl or 5- to 9- membered heteroaryl group fused to a 5- to 9- membered cycloalkyl or heterocyclyl group, said heteroaryl or heterocyclyl group containing at least one heteroatom selected from O, S and N, wherein the cycloalkyl, cycloalkenyl, aryl, heteroaryl, and heterocyclyl groups and bicyclyl group which is a monocyclic C₆-C₉ aryl or 5- to 9- membered heteroaryl group fused to a 5- to 9- membered cycloalkyl or heterocyclyl group, are unsubstituted or substituted by one or more substituents selected from a halogen atom, a cyano group, a linear or branched C₁-C₆ alkyl group, a C₁-C₄ haloalkyl group, a C₁-C₄ hydroxyalkyl group, a C₃-C₇ cycloalkyl group, a monocyclic or bicyclic C₆-C₁₄ aryl group, a 5- to 14- membered heteroaryl group containing at least one heteroatom selected from O, S and N, a 5- to 14-membered heterocyclyl group containing at least one heteroatom selected from O, S and N, a -(CH₂)₁₋₃CN group, a -(CH₂)ₙOR₁₁ group, a -NR₁₀R₁₁ group, a -NR₁₀C(O)-(CH₂)ₙ-R₁₁ group, a -NR₁₀C(O)-(CH₂)ₙ-NR₁₁R₁₂ group, a -C(O)-(CH₂)₁-₃-CN group, a -C(O)-(CH₂)ₙ-R₁₁ group, a -C(O)-(CH₂)ₙ-NR₁₀R₁₁ group, a -S(O)₂(CH₂)ₙR₁₁ group, a -S(O)₂(CH₂)ₙNR₁₀R₁₁ group, or a -NR₁₀S(O)₂(CH₂)ₙNR₁₁R₁₂ group; wherein each n is 0, 1 or 2;
R₃ and R₄ each independently represent a hydrogen atom, a C₁-C₄ haloalkyl group, a C₁-C₄ hydroxyalkyl group or a linear or branched C₁-C₆ alkyl group, which alkyl group is unsubstituted or substituted by one or more substituents selected from a C₁-C₄ alkoxy group, a cyano group, a C₃-C₇ cycloalkyl group, a phenyl group, a pyridyl group, a pyrimidinyl group or a piperidyl group;
R₅ represents a hydrogen atom, a halogen atom, a cyano group, a linear or branched C₁-C₆ alkyl group, a C₁-C₄ haloalkyl group, a C₁-C₄ hydroxyalkyl group, a C₃-C₁₀ cycloalkyl group, a C₃-C₁₀ cycloalkenyl group, a monocyclic or bicyclic C₆-C₄ aryl group, a 5- to 14- membered heteroaryl group containing at least one heteroatom selected from O, S and N, or a 5- to 14- membered heterocyclyl group containing at least one heteroatom selected from O, S and N,
wherein the cycloalkyl, cycloalkenyl, aryl, heteroaryl, and heterocyclyl groups are unsubstituted or substituted by one or more substituents selected from a halogen atom, a cyano group, a linear or branched C₁-C₆ alkyl group, a C₁-C₄ haloalkyl group, a C₁-C₄ hydroxyalkyl group, a C₃-C₇ cycloalkyl, a phenyl group, a pyridyl group, a pyrimidinyl group, a piperidyl group or a -C(O)-(CH₂)ₙ-NR₁₀R₁₁ group; wherein n is 0, 1 or 2;
R₇, R₈ and R₉ each independently represent a hydrogen atom, a C₁-C₄ haloalkyl group, a C₁-C₄ hydroxyalkyl group or a linear or branched C₁-C₆ alkyl group, which alkyl group is unsubstituted or substituted by one or more substituents selected from a C₁-C₄ alkoxy group, a cyano group, a C₃-C₇ cycloalkyl group, a phenyl group, a pyridyl group, a pyrimidinyl group or a piperidyl group;
R₁₀, R₁₁ and R₁₂ each independently represent a hydrogen atom, a C₁-C₄ haloalkyl group, a C₁-C₄ hydroxyalkyl group or a linear or branched C₁-C₆ alkyl group, which alkyl group is unsubstituted or substituted by one or more substituents selected from a C₁-C₄ alkoxy group, a cyano group, a C₃-C₇ cycloalkyl group, a phenyl group, a pyridyl group, a pyrimidinyl group or a piperidyl group.

The invention further provides synthetic processes and intermediates described herein, which are useful for preparing said compounds.

The invention is also directed to a compound of the invention as described herein for use in the treatment of the human or animal body by therapy.

The invention also provides a pharmaceutical composition comprising the compounds of the invention and a pharmaceutically-acceptable diluent or carrier.

The invention is also directed to the compounds of the invention as described herein, for use in the treatment of a pathological condition or disease susceptible to amelioration by inhibiton of Janus Kinases (JAK), in particular wherein the pathological condition or disease is selected from myeloproliferative disorders, leukemia, lymphoid malignancies and solid tumors; bone marrow and organ transplant rejection; immune-mediated diseases and inflammatory diseases; more in particular wherein the pathological condition or disease is selected from rheumatoid arthritis, multiple sclerosis, inflammatory bowel disease, dry eye, uveitis, allergic conjunctivitis, allergic rhinitis, asthma, chronic obstructive pulmonary disease (COPD), atopic dermatitis and psoriasis.

The invention is also directed to use of the compounds of the invention as described herein, in the manufacture of a medicament for treatment of a pathological condition or disease susceptible to amelioration by inhibiton of Janus Kinases (JAK), in particular wherein the pathological condition or disease is selected from myeloproliferative disorders, leukemia, lymphoid malignancies and solid tumors; bone marrow and organ transplant rejection; immune-mediated diseases and inflammatory diseases; more in particular wherein the pathological condition or disease is selected from rheumatoid arthritis, multiple sclerosis, inflammatory bowel disease, dry eye, uveitis, allergic conjunctivitis, allergic rhinitis, asthma, chronic obstructive pulmonary disease (COPD), atopic dermatitis and psoriasis.

The invention also provides a method of treatment of a pathological condition or disease susceptible to amelioration by inhibiton of Janus Kinases (JAK), in particular wherein the pathological condition or disease is selected from myeloproliferative disorders, leukemia, lymphoid malignancies and solid tumors; bone marrow and organ transplant rejection; immune-mediated diseases and inflammatory diseases, more in particular wherein the pathological condition or disease is selected from rheumatoid arthritis, multiple sclerosis, inflammatory bowel disease, dry eye, uveitis, allergic conjunctivitis, allergic rhinitis, asthma, chronic obstructive pulmonary disease (COPD), atopic dermatitis and psoriasis; comprising administering a therapeutically effective amount of the compounds of the invention or a pharmaceutical composition of the invention to a subject in need of such treatment.

The invention also provides a combination product comprising (i) the compounds of the invention as described herein; and (ii) one or more additional active substances which are known to be useful in the treatment of myeloproliferative disorders (such as polycythemia vera, essential thrombocythemia or mielofibrosis), leukemia, lymphoid malignancies and solid tumors; bone marrow and organ transplant rejection; immune-mediated diseases and inflammatory diseases, more in particular wherein the pathological condition or disease is selected from rheumatoid arthritis, multiple sclerosis, inflammatory bowel disease (such as ulcerative colitis or Crohn's disease), dry eye, uveitis, allergic conjunctivitis, allergic rhinitis, asthma, chronic obstructive pulmonary disease (COPD), atopic dermatitis and psoriasis.

As used herein the term C₁-C₆ alkyl embraces linear or branched radicals having 1 to 6 carbon atoms, preferably 1 to 4 carbon atoms. Examples include methyl, ethyl, n-propyl, i-propyl, n-butyl, sec-butyl, t-butyl, n-pentyl, 1-methylbutyl, 2-methylbutyl, isopentyl, 1-ethylpropyl, 1,1-dimethylpropyl, 1,2-dimethylpropyl, n-hexyl, 1-ethylbutyl, 2-ethylbutyl, 1,1-dimethylbutyl, 1,2-dimethylbutyl, 1,3-dimethylbutyl, 2,2-dimethylbutyl, 2,3-dimethylbutyl, 2-methylpentyl, 3-methylpentyl and iso-hexyl radicals.

When it is mentioned that the alkyl radical may be optionally substituted it is meant to include linear or branched alkyl radical as defined above, which may be unsubstituted or substituted in any position by one or more substituents, for example by 1, 2 or 3 substituents. When two or more substituents are present, each substituent may be the same or different.

As used herein, the term C₁-C₄ haloalkyl group is an alkyl group, for example a C₁-C₄ or C₁-C₂ alkyl group, which is bonded to one or more, preferably 1, 2 or 3 halogen atoms. Preferably, said haloakyl group is chosen from -CCl₃, -CHF₂ and -CF₃.

As used herein, the term C₁-C₄ hydroxyalkyl embraces linear or branched alkyl radicals having 1 to 4 carbon atoms, any one of which may be substituted by one or more, preferably 1 or 2, more preferably 1 hydroxyl radicals. Examples of such radicals include hydroxymethyl, hydroxyethyl, hydroxypropyl, and hydroxybutyl.

As used herein, the term C₁-C₄ alkoxy (or alkyloxy) embraces linear or branched oxycontaining radicals each having alkyl portions of 1 to 4 carbon atoms. Examples of C₁-C₄ alkoxy radicals include methoxy, ethoxy, n-propoxy, i-propoxy, n-butoxy, sec-butoxy or t-butoxy.

As used herein, the term C₁-C₄ alkylsulfonyl embraces radicals containing an optionally substituted, linear or branched alkyl radicals of 1 to 4 carbon atoms attached to a divalent -SO₂- radical.

As used herein, the term C₃-C₁₀ cycloalkyl embraces saturated monocyclic or polycyclic carbocyclic radicals having from 3 to 10 carbon atoms, preferably from 3 to 7 carbon atoms. An optionally substituted C₃-C₁₀ cycloalkyl radical is typically unsubstituted or substituted by 1, 2 or 3 substituents which may be the same or different. When a C₃-C₁₀ cycloalkyl radical carries 2 or more substituents, the substituents may be the same or different. Typically the substituents on a C₃-C₁₀ cycloalkyl group are themselves unsubstituted. Polycyclic cycloalkyl radicals contains two or more fused cycloalkyl groups, preferably two cycloalkyl groups. Typically, polycyclic cycloalkyl radicals are selected from decahydronaphthyl (decalyl), bicyclo[2.2.2]octyl, adamantly, camphyl or bornyl groups.

Examples of monocyclic cycloalkyl groups include cyclopropyl, cyclobutyl, cyclopentyl, cyclohexyl, cycloheptyl, cyclooctyl, cyclononyl and cyclodecyl.

As used herein, the term C₃-C₁₀ cycloalkenyl embraces partially unsaturated carbocyclic radicals having from 3 to 10 carbon atoms, preferably from 3 to 7 carbon atoms. A C₃-C₁₀ cycloalkenyl radical is typically unsubstituted or substituted by 1, 2 or 3 substituents which may be the same or different. When a C₃-C₁₀ cycloalkenyl radical carries 2 or more substituents, the substituents may be the same or different. Typically, the substituents on a cycloalkenyl group are themselves unsubstituted. Examples include cyclobutenyl, cyclopentenyl, cyclohexenyl, cycloheptenyl, cyclooctenyl, cyclononenyl and cyclodecenyl.

As used herein, the term C₆-C₁₄ aryl radical embraces typically a C₆-C₁₄, preferably C₆-C₁₀ monocyclic or bicyclic aryl radical such as phenyl, naphthyl, anthranyl and phenanthryl. Phenyl is preferred. A said optionally substituted C₆-C₁₄ aryl radical is typically unsubstituted or substituted by 1, 2 or 3 substituents which may be the same or different. When a C₆-C₁₄ aryl radical carries 2 or more substituents, the substituents may be the same or different. Unless otherwise specified, the substituents on a C₆-C₁₄ aryl group are typically themselves unsubstituted.

As used herein, the term 5- to 14- membered heteroaryl radical embraces typically a 5-to 14- membered ring system, preferably a 5- to 10- membered ring system, more preferably a 5- to 6- membered ring system, comprising at least one heteroaromatic ring and containing at least one heteroatom selected from O, S and N. A 5- to 14-membered heteroaryl radical may be a single ring or two or more fused rings wherein at least one ring contains a heteroatom.

A said optionally substituted 5- to 14- membered heteroaryl radical is typically unsubstituted or substituted by 1, 2 or 3 substituents which may be the same or different. When a 5- to 14- membered heteroaryl radical carries 2 or more substituents, the substituents may be the same or different. Unless otherwise specified, the substituents on a 5- to 14- membered heteroaryl radical are typically themselves unsubstituted.

Examples include pyridyl, pyrazinyl, pyrimidinyl, pyridazinyl, furyl, benzofuranyl, oxadiazolyl, oxazolyl, isoxazolyl, benzoxazolyl, imidazolyl, benzimidazolyl, thiazolyl, thiadiazolyl, thienyl, pyrrolyl, benzothiazolyl, indolyl, indazolyl, purinyl, quinolyl, isoquinolyl, phthalazinyl, naphthyridinyl, quinoxalinyl, quinazolinyl, quinolizinyl, cinnolinyl, triazolyl, indolizinyl, indolinyl, isoindolinyl, isoindolyl, imidazolidinyl, pteridinyl, thianthrenyl, pyrazolyl, 2H-pyrazolo[3,4-d]pyrimidinyl, 1 H-pyrazolo[3,4-d]pyrimidinyl, thieno[2,3-d] pyrimidinyl and the various pyrrolopyridyl radicals.

As used herein, the term 5- to 14-membered heterocyclyl radical embraces typically a non-aromatic, saturated or unsaturated C₅-C₁₄ carbocyclic ring system, preferably C₅-C₁₀ carbocyclic ring system, more preferably C₅-C₆ carbocyclic ring system, in which one or more, for example 1, 2, 3 or 4 of the carbon atoms preferably 1 or 2 of the carbon atoms are replaced by a heteroatom selected from N, O and S. A heterocyclyl radical may be a single ring or two or more fused rings wherein at least one ring contains a heteroatom. When a 5 to 14-membered heterocyclyl radical carries 2 or more substituents, the substituents may be the same or different.

A said optionally substituted 5- to 14-membered heterocyclyl radical is typically unsubstituted or substituted by 1, 2 or 3 substituents which may be the same or different. Typically, the substituents on a 5 to 14-membered heterocyclyl radical are themselves unsubstituted.

Examples of 5- to 14-membered heterocyclyl radicals include piperidyl, pyrrolidyl, pyrrolinyl, piperazinyl, morpholinyl, thiomorpholinyl, pyrrolyl, pyrazolinyl, pirazolidinyl, quinuclidinyl, triazolyl, pyrazolyl, tetrazolyl, imidazolidinyl, imidazolyl, oxiranyl, thiaranyl, aziridinyl, oxetanyl, thiatanyl, azetidinyl, 4,5-dihydro-oxazolyl, 2-benzofuran-1 (3H)-one, 1,3-dioxol-2-one, tetrahydrofuranyl, 3-aza-tetrahydrofuranyl, tetrahydrothiophenyl, tetrahydropyranyl, tetrahydrothiopyranyl, 1,4-azathianyl, oxepanyl, thiephanyl, azepanyl, 1,4-dioxepnayl, 1,4-oxathiepanyl, 1,4-oxaazepanyl, 1,4-dithiepanyl, 1,4-thiezepanyl, 1,4-diazepanyl, tropanyl, (1S,5R)-3-aza-bicyclo[3.1.0]hexyl, 3,4-dihydro-2H-pyranyl, 5,6-dihydro-2H-pyranyl, 2H-pyranyl, 2,3-hydrobenzofuranyl, 1,2,3,4-tetrahydropyridinyl, 1,2,5,6-tetrahydropyridinyl, isoindolinyl and indolinyl.

Where a 5- to 14-membered heterocyclyl radical carries 2 or more substituents, the substituents may be the same or different.

As used herein, the term bicyclyl group which is a monocyclic C₆-C₉ aryl or 5- to 9-membered heteroaryl group fused to a 5- to 9- membered cycloalkyl or heterocyclyl group typically refers to a moiety containing a bond which is shared between a monocyclic C₆-C₉ aryl or 5- to 9- membered heteroaryl group and a 5- to 9- membered cycloalkyl or heterocyclyl group, wherein said heteroaryl or heterocyclyl group contains at least one heteroatom selected from O, S and N. Typically said bicyclyl group is a phenyl or 5- or 6- membered heteroaryl group fused to a 5- or 6-, preferably 6-, membered cycloalkyl or heterocyclyl group. Typically said heteroaryl or heterocyclyl group contains 1, 2 or 3, preferably 1 or 2, for example 1, heteroatom selected from O, S and N, preferably N. Examples include chromanyl groups or 1,2,3,4-tetrahydronaphthalenyl groups. 1,2,3,4-tetrahydronaphthalenyl groups are preferred.

As used herein, some of the atoms, radicals, moieties, chains and cycles present in the general structures of the invention are "optionally substituted". This means that these atoms, radicals, moieties, chains and cycles can be either unsubstituted or substituted in any position by one or more, for example 1, 2, 3 or 4, substituents, whereby the hydrogen atoms bound to the unsubstituted atoms, radicals, moieties, chains and cycles are replaced by chemically acceptable atoms, radicals, moieties, chains and cycles. When two or more substituents are present, each substituent may be the same or different. The substituents are typically themselves unsubstituted.

As used herein, the term halogen atom embraces chlorine, fluorine, bromine and iodine atoms. A halogen atom is typically a fluorine, chlorine or bromine atom, most preferably chlorine or fluorine. The term halo when used as a prefix has the same meaning.

Compounds containing one or more chiral centre may be used in enantiomerically or diastereoisomerically pure form, in the form of racemic mixtures and in the form of mixtures enriched in one or more stereoisomer. The scope of the invention as described and claimed encompasses the racemic forms of the compounds as well as the individual enantiomers, diastereomers, and stereoisomer-enriched mixtures.

Conventional techniques for the preparation/isolation of individual enantiomers include chiral synthesis from a suitable optically pure precursor or resolution of the racemate using, for example, chiral high pressure liquid chromatography (HPLC). Alternatively, the racemate (or a racemic precursor) may be reacted with a suitable optically active compound, for example, an alcohol, or, in the case where the compound contains an acidic or basic moiety, an acid or base such as tartaric acid or 1-phenylethylamine. The resulting diastereomehc mixture may be separated by chromatography and/or fractional crystallization and one or both of the diastereoisomers converted to the corresponding pure enantiomer(s) by means well known to one skilled in the art. Chiral compounds of the invention (and chiral precursors thereof) may be obtained in enantiomerically-enriched form using chromatography, typically HPLC, on an asymmetric resin with a mobile phase consisting of a hydrocarbon, typically heptane or hexane, containing from 0 to 50% isopropanol, typically from 2 to 20%, and from 0 to 5% of an alkylamine, typically 0.1 % diethylamine. Concentration of the eluate affords the enriched mixture. Stereoisomer conglomerates may be separated by conventional techniques known to those skilled in the art. See, e.g. "Stereochemistry of Organic Compounds" by Ernest L. Eliel (Wiley, New York, 1994).

As used herein, the term pharmaceutically acceptable salt refers to a salt prepared from a base or acid which is acceptable for administration to a patient, such as a mammal. Such salts can be derived from pharmaceutically-acceptable inorganic or organic bases and from pharmaceutically-acceptable inorganic or organic acids.

Pharmaceutically acceptable acids include both inorganic acids, for example hydrochloric, sulphuric, phosphoric, diphosphoric, hydrobromic, hydroiodic and nitric acid; and organic acids, for example citric, fumaric, gluconic, glutamic, lactic, maleic, malic, mandelic, mucic, ascorbic, oxalic, pantothenic, succinic, tartaric, benzoic, acetic, methanesulphonic, ethanesulphonic, benzenesulphonic, p-toluenesulphonic acid, xinafoic (1-hydroxy-2-naphthoic acid), napadisilic (1,5-naphthalenedisulfonic acid) and the like. Particularly preferred are salts derived from fumaric, hydrobromic, hydrochloric, acetic, sulfuric, methanesulfonic, xinafoic, and tartaric acids.

Salts derived from pharmaceutically-acceptable inorganic bases include aluminum, ammonium, calcium, copper, ferric, ferrous, lithium, magnesium, manganic, manganous, potassium, sodium, zinc and the like. Particularly preferred are ammonium, calcium, magnesium, potassium and sodium salts.

Salts derived from pharmaceutically-acceptable organic bases include salts of primary, secondary and tertiary amines, including alkyl amines, arylalkyl amines, heterocyclyl amines, cyclic amines, naturally-occurring amines and the like, such as arginine, betaine, caffeine, choline, N,N'-dibenzylethylenediamine, diethylamine, 2-diethylaminoethanol, 2-dimethylaminoethanol, ethanolamine, ethylenediamine, N-ethylmorpholine, N-ethylpiperidine, glucamine, glucosamine, histidine, hydrabamine, isopropylamine, lysine, methylglucamine, morpholine, piperazine, piperidine, polyamine resins, procaine, purines, theobromine, triethylamine, trimethylamine, tripropylamine, tromethamine and the like.

Other preferred salts according to the invention are quaternary ammonium compounds wherein an equivalent of an anion (X⁻) is associated with the positive charge of the N atom. X⁻ may be an anion of various mineral acids such as, for example, chloride, bromide, iodide, sulphate, nitrate, phosphate, or an anion of an organic acid such as, for example, acetate, maleate, fumarate, citrate, oxalate, succinate, tartrate, malate, mandelate, trifluoroacetate, methanesulphonate and *p*-toluenesulphonate. X⁻ is preferably an anion selected from chloride, bromide, iodide, sulphate, nitrate, acetate, maleate, oxalate, succinate or trifluoroacetate. More preferably X⁻ is chloride, bromide, trifluoroacetate or methanesulphonate.

As used herein, an N-oxide is formed from the tertiary basic amines or imines present in the molecule, using a convenient oxidising agent.

The compounds of the invention may exist in both unsolvated and solvated forms. The term solvate is used herein to describe a molecular complex comprising a compound of the invention and an amount of one or more pharmaceutically acceptable solvent molecules. The term hydrate is employed when said solvent is water. Examples of solvate forms include, but are not limited to, compounds of the invention in association with water, acetone, dichloromethane, 2-propanol, ethanol, methanol, dimethylsulfoxide (DMSO), ethyl acetate, acetic acid, ethanolamine, or mixtures thereof. It is specifically contemplated that in the present invention one solvent molecule can be associated with one molecule of the compounds of the present invention, such as a hydrate. Furthermore, it is specifically contemplated that in the present invention, more than one solvent molecule may be associated with one molecule of the compounds of the present invention, such as a dihydrate. Additionally, it is specifically contemplated that in the present invention less than one solvent molecule may be associated with one molecule of the compounds of the present invention, such as a hemihydrate. Furthermore, solvates of the present invention are contemplated as solvates of compounds of the present invention that retain the biological effectiveness of the non-solvate form of the compounds.

The invention also includes isotopically-labeled compounds of the invention, wherein one or more atoms is replaced by an atom having the same atomic number, but an atomic mass or mass number different from the atomic mass or mass number usually found in nature. Examples of isotopes suitable for inclusion in the compounds of the invention include isotopes of hydrogen, such as ²H and ³H, carbon, such as ¹¹C, ¹³C and ¹⁴C, chlorine, such as ³⁶Cl, fluorine, such as ¹⁸F, iodine, such as ¹²³I and ¹²⁵I, nitrogen, such as ¹³N and ¹⁵N, oxygen, such as ¹⁵O, ¹⁷O and ¹⁸O, phosphorus, such as ³²P, and sulfur, such as ³⁵S. Certain isotopically-labeled compounds of the invention, for example, those incorporating a radioactive isotope, are useful in drug and/or substrate tissue distribution studies. The radioactive isotopes tritium, ³H, and carbon-14, ¹⁴C , are particularly useful for this purpose in view of their ease of incorporation and ready means of detection. Substitution with heavier isotopes such as deuterium, ²H, may afford certain therapeutic advantages resulting from greater metabolic stability, for example, increased in vivo half-life or reduced dosage requirements, and hence may be preferred in some circumstances. Substitution with positron emitting isotopes, such as ¹¹C, ¹⁸F, ¹⁵O and ¹³N, can be useful in Positron Emission Topography (PET) studies for examining substrate receptor occupancy.

Isotopically-labeled compounds of the invention can generally be prepared by conventional techniques known to those skilled in the art or by processes analogous to those described herein, using an appropriate isotopically-labeled reagent in place of the non-labeled reagent otherwise employed.

Preferred isotopically-labeled compounds include deuterated derivatives of the compounds of the invention. As used herein, the term deuterated derivative embraces compounds of the invention where in a particular position at least one hydrogen atom is replaced by deuterium. Deuterium (D or ²H) is a stable isotope of hydrogen which is present at a natural abundance of 0.015 molar %.

Hydrogen deuterium exchange (deuterium incorporation) is a chemical reaction in which a covalently bonded hydrogen atom is replaced by a deuterium atom. Said exchange (incorporation) reaction can be total or partial.

Typically, a deuterated derivative of a compound of the invention has an isotopic enrichment factor (ratio between the isotopic abundance and the natural abundance of that isotope, i.e. the percentage of incorporation of deuterium at a given position in a molecule in the place of hydrogen) for each deuterium present at a site designated as a potential site of deuteration on the compound of at least 3500 (52.5% deuterium incorporation).

In a preferred embodiment, the isotopic enrichment factor is at least 5000 (75% deuterium). In a more preferred embodiment, the isotopic enrichment factor is at least 6333.3 (95% deuterium incorporation). In a most preferred embodiment, the isotopic enrichment factor is at least 6633.3 (99.5% deuterium incorporation). It is understood that the isotopic enrichment factor of each deuterium present at a site designated as a site of deuteration is independent from the other deuteration sites.

The isotopic enrichment factor can be determined using conventional analytical methods known too en ordinary skilled in the art, including mass spectrometry (MS) and nuclear magnetic resonance (NMR).

Prodrugs of the compounds described herein are also within the scope of the invention. Thus certain derivatives of the compounds of the present invention, which derivatives may have little or no pharmacological activity themselves, when administered into or onto the body may be converted into compounds of the present invention having the desired activity, for example, by hydrolytic cleavage. Such derivatives are referred to as 'prodrugs'. Further information on the use of prodrugs may be found in Pro-drugs as Novel Delivery Systems, Vol. 14, ACS Symposium Series (T. Higuchi and W. Stella) and Bioreversible Carriers in Drug Design, Pergamon Press, 1987 (ed. E. B. Roche, American Pharmaceutical Association).

Prodrugs in accordance with the invention can, for example, be produced by replacing appropriate functionalities present in the compounds of the present invention with certain moieties known to those skilled in the art as 'pro-moieties' as described, for example, in Design of Prodrugs by H. Bundgaard (Elsevier, 1985).

In the case of compounds that are solids, it is understood by those skilled in the art that the inventive compounds and salts may exist in different crystalline or polymorphic forms, or in an amorphous form, all of which are intended to be within the scope of the present invention.

Typically, in the compound of formula (I), X and Y each independently represent a nitrogen atom or a -CR₅ group, wherein at least one of X and Y represents a -CR₅ group.

In one embodiment, in the compound of formula (I) X represents a nitrogen atom and Y represents a -CR₅ group.

In other embodiment, in the compound of formula (I) Y represents a nitrogen atom and X represents a -CR₅ group.

In another embodiment, in the compound of formula (I) X and Y independently represent a -CR₅ group.

For the avoidance of doubt, when two -CR₅ groups are present, they may be the same or different.

In one embodiment, in the compound of formula (I) A represents a nitrogen atom and B represents a -CR₆ group.

In other embodiment, in the compound of formula (I) B represents a nitrogen atom and A represents a -CR₆ group.

In another embodiment, in the compound of formula (I) A and B independently represent a -CR₆ group.

For the avoidance of doubt, when two -CR₆ groups are present, they may be the same or different.

Typically, in the compound of formula (I) R₁ represents a hydrogen atom, a linear or branched C₁-C₆ alkyl group, a C₁-C₄ haloalkyl group, a C₁-C₄ hydroxyalkyl group, a C₃-C₇ cycloalkyl group, a phenyl group, a pyridyl group, a pyrimidinyl group or a piperidinyl group.

Preferably, R₁ represents a hydrogen atom, a linear or branched C₁-C₄ alkyl group, a C₁-C₄ haloalkyl group, a C₁-C₄ hydroxyalkyl group, a C₃-C₇ cycloalkyl group, a phenyl group or a pyridyl group.

More preferably R₁ represents a hydrogen atom, a linear or branched C₁-C₃ alkyl group, a C₁-C₃ haloalkyl group or a C₁-C₃ hydroxyalkyl group. Most preferably R₁ represents a hydrogen atom.

Typically, in the compound of formula (I) R₂ represents a linear or branched C₁-C₆ alkyl group, a C₁-C₄ haloalkyl group, a C₁-C₄ hydroxyalkyl group, a C₃-C₇ cycloalkyl group, a monocyclic or bicyclic C₆-C₁₄ aryl group, a 5- to 7- membered heteroaryl group containing one, two or three heteroatoms selected from O, S and N, a 5- to 7-membered heterocyclyl group containing one, two or three heteroatoms selected from O, S and N, or a bicyclyl group which is a monocyclic C₆-C₉ aryl or 5- to 9- membered heteroaryl group fused to a 5- to 9- membered cycloalkyl or heterocyclyl group, said heteroaryl or heterocyclyl group containing one, two or three heteroatoms selected from O, S and N,
wherein the cycloalkyl, cycloalkenyl, aryl, heteroaryl, and heterocyclyl groups and bicyclyl group which is a monocyclic C₆-C₉ aryl or 5- to 9- membered heteroaryl group fused to a 5- to 9- membered cycloalkyl or heterocyclyl group are unsubstituted or substituted by one or more substituents selected from a halogen atom, a cyano group, a linear or branched C₁-C₆ alkyl group, a C₁-C₄ haloalkyl group, a C₁-C₄ hydroxyalkyl group, a C₃-C₇ cycloalkyl group, a monocyclic or bicyclic C₆-C₁₄ aryl group, a 5- to 14- membered heteroaryl group containing at least one heteroatom selected from O, S and N, a 5- to 14-membered heterocyclyl group containing at least one heteroatom selected from O, S and N, a -(CH₂)₁₋₃CN group, a -(CH₂)ₙOR₁₁ group, a -NR₁₀R₁₁ group, a -NR₁₀C(O)-(CH₂)ₙ-R₁₁ group, a -NR₁₀C(O)-(CH₂)ₙ-NR₁₁R₁₂ group, a -C(O)-(CH₂)₁₋₃-CN group, a -C(O)-(CH₂)ₙ-R₁₁ group, a -C(O)-(CH₂)ₙ-NR₁₀R₁₁ group, a -S(O)₂(CH₂)ₙR₁₁ group, a -S(O)₂(CH₂)ₙNR₁₀R₁₁ group, or a -NR₁₀S(O)₂(CH₂)ₙNR₁₁R₁₂ group; wherein each n is 0, 1 or 2; and wherein R₁₀, R₁₁ and R₁₂ are as defined above.

Preferably R₂ represents a linear or branched C₁-C₆ alkyl group, a C₁-C₄ haloalkyl group, a C₁-C₄ hydroxyalkyl group, a C₃-C₇ cycloalkyl group, a phenyl group, a pyridyl group, a pyrimidinyl group, a pyrrolidinyl group, a piperidyl group, a tetrahydropyranyl group, a morpholinyl group, or a tetrahydronaphthalenyl group,
wherein the cycloalkyl, phenyl, pyridyl, pyrimidinyl, pyrrolidinyl, piperidyl, tetrahydropyranyl, morpholinyl or tetrahydronaphthalenyl groups are unsubstituted or substituted by one, two or three substituents selected from a halogen atom, a cyano group, a linear or branched C₁-C₆ alkyl group, a C₁-C₄ haloalkyl group, a C₁-C₄ hydroxyalkyl group, a C₃-C₇ cycloalkyl group, a phenyl group, a pyridyl group, a pyrimidinyl group, a piperidyl group, a -(CH₂)₁₋₃CN group, a -(CH₂)ₙOR₁₁ group, a -NR₁₀R₁₁ group, a -NR₁₀C(O)-(CH₂)ₙ-R₁₁ group, a -NR₁₀C(O)-(CH₂)ₙ-NR₁₁R₁₂ group, a -C(O)-(CH₂)₁₋₃-CN group, a -C(O)-(CH₂)ₙ-R₁₁ group, a -C(O)-(CH₂)ₙ-NR₁₀R₁₁ group, a -S(O)₂(CH₂)ₙR₁₁ group, a -S(O)₂(CH₂)ₙNR₁₀R₁₁ group, or a -NR₁₀S(O)₂(CH₂)ₙNR₁₁R₁₂ group; wherein each n is 0, 1 or 2; and wherein R₁₀, R₁₁ and R₁₂ each independently represent a hydrogen atom or a linear or branched C₁-C₃ alkyl group.

More preferably, in the compound of formula (I) R₂ represents a C₃-C₇ cycloalkyl group, a phenyl group, a pyridyl group, a pyrimidinyl group, a tetrahydropyranyl group, or a tetrahydronaphthalenyl group,
wherein the cycloalkyl, phenyl, pyridyl, pyrimidinyl or tetrahydropyranyl groups are unsubstituted or substituted by one, two or three substituents selected from a halogen atom, a linear or branched C₁-C₃ alkyl group, a C₁-C₃ haloalkyl group, a -(CH₂)₁₋₃CN group or a -(CH₂)ₙOR₁₁ group; wherein is 0, 1 or 2; and wherein R₁₁ represents a hydrogen atom or a methyl group.

Preferably, when R₂ is a C₃-C₇ cycloalkyl group, it is a cyclopropyl group, a cyclobutyl group, a cyclopentyl group, a cyclohexyl group or a cycloheptyl group, which group is unsubstituted or substituted by one, two or three substituents selected from a halogen atom (preferably a fluorine atom or a chlorine atom), a linear or branched C₁-C₃ alkyl group, a C₁-C₃ haloalkyl group, a -(CH₂)₁₋₃CN group or a hydroxy group. More preferably, when R₂ is a C₃-C₇ cycloalkyl group group, it is preferably a cyclohexyl group unsubstituted or substituted by one, two or three substituents selected from linear or branched C₁-C₃ alkyl group (preferably a methyl group), a -(CH₂)₁₋₃CN group or a hydroxy group.

Preferably, when R₂ is a C₃-C₇ cycloalkyl group m is 0. In other words, when R₂ is a C₃-C₇ cycloalkyl group it is directly bonded to nitrogen atom of the imidazolidin-2-one ring.

Preferably, when R₂ is a pyridyl or pyrimidinyl group, said groups are linked to the rest of the molecule via a ring carbon atom, in other words they are linked to the group -(R₃ C R₄)ₘ-, which is bonded to the imidazolidin-2-one ring, via a ring carbon atom. Pyridyl and pyrimidinyl groups are unsubstituted or substituted with one, two or three substituents selected from a halogen atom (preferably a fluorine atom or a chlorine atom), a cyano group, a linear or branched C₁-C₃ alkyl group, a C₁-C₄ haloalkyl group (preferably a -CHF₂ group or a -CF₃ group), a C₃-C₇ cycloalkyl group, a phenyl group, a pyridyl group, a pyrimidinyl group, a piperidyl group, a -(CH₂)₁₋₃CN group, a - (CH₂)ₙOR₁₁ group, a -NR₁₀R₁₁ group, a -NR₁₀C(O)-(CH₂)ₙ-R₁₁ group, a -NR₁₀C(O)-(CH₂)ₙ-NR₁₁R₁₂ group, a -C(O)-(CH₂)₁₋₃-CN group, a -C(O)-(CH₂)ₙ-R₁₁ group, a -C(O)-(CH₂)ₙ-NR₁₀R₁₁ group, a -S(O)₂(CH₂)ₙR₁₁ group, a -S(O)₂(CH₂)ₙNR₁₀R₁₁ group, or a -NR₁₀S(O)₂(CH₂)ₙNR₁₁R₁₂ group; wherein each n is 0, 1 or 2; and wherein R₁₀, R₁₁ and R₁₂ each independently represent a hydrogen atom or a linear or branched C₁-C₃ alkyl group. More preferably, pyridyl and pyrimidinyl groups are substituted by one or two halogen atoms.

Preferably, when R₂ is a tetrahydropyranyl group, it is linked to the rest of the molecule via a ring carbon atom. In this case, m is 0. In other words, when R₂ is a tetrahydropyranyl group it is directly bonded to the nitrogen atom of the imidazolidin-2-one ring via a carbon atom.

Preferably, when R₂ is a tetrahydropyranyl group, it is unsubstituted or substituted by one, two or three substituents selected from a halogen atom (preferably a fluorine atom or a chlorine atom) or a linear or branched C₁-C₃ alkyl. Preferably when R₂ is a tetrahydropyranyl group it is unsubstituted.

Preferably, when R₂ is a tetrahydronaphthalenyl group, it is linked to the rest of the molecule via a ring carbon atom. In this case, m is 0. In other words, when R₂ is a tetrahydronaphthalenyl group it is directly bonded to the nitrogen atom of the imidazolidin-2-one ring via a carbon atom.

Preferably, when R₂ is a tetrahydronaphthalenyl group, it is unsubstituted or substituted by one, two or three substituents selected from a halogen atom (preferably a fluorine atom or a chlorine atom), a linear or branched C₁-C₃ alky or a hydroxy group.

Typically, in the compound of formula (I) R₃ and R₄ each independently represent a hydrogen atom or a linear or branched C₁-C₆ alkyl group, which alkyl group is unsubstituted or substituted by a C₁-C₂ alkoxy group. Preferably, R₃ and R₄ each independently represent a hydrogen atom or a linear or branched C₁-C₃ alkyl group. More preferably, R₃ and R₄ each independently represent a hydrogen atom or a methyl group.

Typically, in the compound of formula (I) R₅ represents a hydrogen atom, a halogen atom, a cyano group, a linear or branched C₁-C₄ alkyl group, a C₁-C₄ haloalkyl group, a C₁-C₄ hydroxyalkyl group, a C₃-C₇ cycloalkyl group, a phenyl group, a pyridyl group, a pyrimidinyl group, a pyrrolidinyl group, a piperidyl group, a tetrahydropyranyl group or a morpholinyl group,
wherein the phenyl, pyridyl, pyrimidinyl, pyrrolidinyl, piperidyl, tetrahydropyranyl or morpholinyl groups are unsubstituted or substituted by one, two or three substituents selected from a halogen atom, a cyano group, a linear or branched C₁-C₆ alkyl group, a C₁-C₄ haloalkyl group, a C₁-C₄ hydroxyalkyl group, a C₃-C₇ cycloalkyl, a phenyl group, a pyridyl group, a pyrimidinyl group, a piperidyl group, or a -C(O)-(CH₂)ₙ-NR₁₀R₁₁ group; wherein n is 0, 1 or 2; and wherein R₁₀ and R₁₁ are as defined above.

Preferably, in the compound of formula (I) R₅ represents a hydrogen atom, a halogen atom, a cyano group, a linear or branched C₁-C₄ alkyl group, a C₁-C₄ haloalkyl group, a C₁-C₄ hydroxyalkyl group or a C₃-C₇ cycloalkyl group.

More preferably, in the compound of formula (I) R₅ represents a hydrogen atom, a halogen atom (preferably a fluorine atom or a chlorine atom), a linear or branched C₁-C₃ alkyl group or a C₁-C₃ haloalkyl group.

Typically, in the compound of formula (I), R₆ represents a hydrogen atom, a halogen atom, a cyano group, a linear or branched C₁-C₄ alkyl group, a C₁-C₄ haloalkyl group, a C₁-C₄ hydroxyalkyl group, a C₃-C₇ cycloalkyl group, a phenyl group, a pyridyl group, a pyrimidinyl group, a pyrrolidinyl group, a piperidyl group, a tetrahydropyranyl group, a morpholinyl group, or a tetrahydronaphthalenyl group, wherein the cycloalkyl, phenyl, pyridyl, pyrimidinyl, pyrrolidinyl, piperidyl, tetrahydropyranyl, morpholinyl or tetrahydronaphthalenyl groups are unsubstituted or substituted by one, two or three substituents selected from a halogen atom, a cyano group, a linear or branched C₁-C₆ alkyl group, a C₁-C₄ haloalkyl group, a C₁-C₄ hydroxyalkyl group, a C₃-C₇ cycloalkyl group, a phenyl group, a pyridyl group, a pyrimidinyl group, a piperidyl group, a -(CH₂)₁₋₃CN group, a -(CH₂)ₙOR₁₁ group, a -NR₁₀R₁₁ group, a -NR₁₀C(O)-(CH₂)ₙ-R₁₁ group, a -NR₁₀C(O)-(CH₂)ₙ-NR₁₁R₁₂ group, a -C(O)-(CH₂)₁₋₃-CN group, a -C(O)-(CH₂)ₙ-R₁₁ group, a -C(O)-(CH₂)ₙ-NR₁₀R₁₁ group, a -S(O)₂(CH₂)ₙR₁₁ group, a -S(O)₂(CH₂)ₙNR₁₀R₁₁ group, or a -NR₁₀S(O)₂(CH₂)ₙNR₁₁R₁₂ group; wherein each n is 0, 1 or 2; and wherein R₁₀, R₁₁ and R₁₂ each independently represent a hydrogen atom or a linear or branched C₁-C₃ alkyl group.

Preferably, in the compound of formula (I) R₆ represents a hydrogen atom, a halogen atom, a cyano group, a linear or branched C₁-C₃ alkyl group, a C₁-C₄ haloalkyl group, a C₁-C₄ hydroxyalkyl group, a C₃-C₇ cycloalkyl group, a phenyl group, a pyridyl group, a morpholinyl, or tetrahydronaphthalenyl groups,
wherein the cycloalkyl, phenyl, pyridyl, pyrimidinyl, pyrrolidinyl, piperidyl, tetrahydropyranyl, morpholinyl or tetrahydronaphthalenyl groups are unsubstituted or substituted by one, two or three substituents selected from a halogen atom, a cyano group, a linear or branched C₁-C₆ alkyl group, a C₁-C₄ haloalkyl group, a C₁-C₄ hydroxyalkyl group, a C₃-C₇ cycloalkyl group.

More preferably, in the compound of formula (I) R₆ represents a hydrogen atom, a halogen atom (preferably a fluorine atom or a chlorine atom), a cyano group, a linear or branched C₁-C₃ alkyl group, a C₁-C₄ haloalkyl group, a C₁-C₄ hydroxyalkyl group, a C₃-C₇ cycloalkyl group, a phenyl group or a morpholinyl group.

Preferably, when R₆ is a morpholinyl group it is linked to the rest of the molecule via the ring nitrogen atom. In other words, when R₆ is a morpholinyl group it is bonded to the pyridyl ring via the ring nitrogen atom of the morpholinyl group.

Typically, in the compound of formula (I), R₇ represents a hydrogen atom or a linear or branched C₁-C₆ alkyl group, which alkyl group is unsubstituted or substituted by a C₁-C₂ alkoxy group. Preferably, R₇ represents a hydrogen atom or a linear or branched C₁-C₃ alkyl group. More preferably, R₇ represents a hydrogen atom or a methyl group.

Typically, in the compound of formula (I), m is 0, 1 or 2; preferably 0 or 1.

Typically, in the compound of formula (I), W represents a linker selected from a -NR₇-group, a -(CR₈R₉)- group, -O- or -S-, wherein R₇, R₈ and R₉ are as defined above. Preferably, in the compound of formula (I), W represents a linker selected from a -NR₇-group or a -(CR₈R₉)- group, wherein R₇, R₈ and R₉ are as defined above. More preferably W represents a -NR₇- group wherein R₇ is as defined above. Even more preferably W represents a -NR₇- group wherein R₇ is a hydrogen atom or a C₁-C₃ alkyl group. Most preferably, W represents a -NR₇- group wherein R₇ is a hydrogen atom or a methyl group.

When the cycloalkyl, cycloalkenyl, aryl, heteroaryl, heterocyclyl and bicyclyl groups that R₂ and R₆ may represent are substituted by one or more -NR₁₀C(O)-(CH₂)ₙ-R₁₁ groups or one or more -C(O)-(CH₂)ₙ-R₁₁ groups, and n is 0, then it is preferred that R₁₁ does not represent a hydrogen atom.

In a particularly preferred embodiment, in the compound of formula (I)
m is 0 or 1;
X is a nitrogen atom and Y is a -CR₅ group; or Y is a nitrogen atom and X is a -CR₅ group; or both X and Y are a -CR₅ group;
A is a nitrogen atom and B is a -CR₆ group; or B is a nitrogen atom and A is a -CR₆ group; or both A and B are a -CR₆ group;
W represents a linker selected from a -NR₇- group, a -(CR₈R₉)- group, -O- or -S-;
R₁ represents a hydrogen atom, a linear or branched C₁-C₄ alkyl group, a C₁-C₄ haloalkyl group, a C₁-C₄ hydroxyalkyl group, a C₃-C₇ cycloalkyl group, a phenyl group or a pyridyl group;
R₂ represents a linear or branched C₁-C₆ alkyl group, a C₁-C₄ haloalkyl group, a C₁-C₄ hydroxyalkyl group, a C₃-C₇ cycloalkyl group, a monocyclic or bicyclic C₆-C₁₄ aryl group, a 5- to 9- membered heteroaryl group containing one, two or three heteroatoms selected from O, S and N, a 5- to 9- membered heterocyclyl group containing one, two or three heteroatoms selected from O, S and N, or a bicyclyl group which is a monocyclic C₆-C₉ aryl or 5- to 9- membered heteroaryl group fused to a 5- to 9-membered cycloalkyl or heterocyclyl group, said heteroaryl or heterocyclyl groups containing one, two or three heteroatoms selected from O, S and N,
wherein the cycloalkyl, aryl, heteroaryl, and heterocyclyl groups and bicyclyl group which is a monocyclic C₆-C₉ aryl or 5- to 9- membered heteroaryl group fused to a 5- to 9- membered cycloalkyl or heterocyclyl group are unsubstituted or substituted by one or more substituents selected from a halogen atom, a cyano group, a linear or branched C₁-C₆ alkyl group, a C₁-C₄ haloalkyl group, a C₁-C₄ hydroxyalkyl group, a C₃-C₇ cycloalkyl group, a monocyclic or bicyclic C₆-C₁₄ aryl group, a 5- to 14- membered heteroaryl group containing at least one heteroatom selected from O, S and N, a 5- to 14- membered heterocyclyl group containing at least one heteroatom selected from O, S and N, a -(CH₂)₁₋₃CN group, a -(CH₂)ₙOR₁₁ group, a -NR₁₀R₁₁ group, a -NR₁₀C(O)-(CH₂)ₙ-R₁₁ group, a -NR₁₀C(O)-(CH₂)ₙ-NR₁₁R₁₂ group, a -C(O)-(CH₂)₁₋₃-CN group, a -C(O)-(CH₂)ₙ-R₁₁ group, a -C(O)-(CH₂)ₙ-NR₁₀R₁₁ group, a -S(O)₂(CH₂)ₙR₁₁ group, a -S(O)₂(CH₂)ₙNR₁₀R₁₁ group, or a -NR₁₀S(O)₂(CH₂)ₙNR₁₁R₁₂ group; wherein each n is 0, 1 or 2;
R₃ and R₄ each independently represent a hydrogen atom or a linear or branched C₁-C₃ alkyl group;
R₅ represent a hydrogen atom, a halogen atom, a cyano group, a linear or branched C₁-C₄ alkyl group, a C₁-C₄ haloalkyl group, a C₁-C₄ hydroxyalkyl group, a C₃-C₇ cycloalkyl group, a monocyclic or bicyclic C₆-C₁₄ aryl group, a 5- to 9- membered heteroaryl group containing one, two or three heteroatoms selected from O, S and N, a 5- to 9- membered heterocyclyl group containing one, two or three heteroatoms selected from O, S and N,
wherein the cycloalkyl, aryl, heteroaryl, and heterocyclyl groups are unsubstituted or substituted by one, two or three substituents selected from a halogen atom, a cyano group, a linear or branched C₁-C₆ alkyl group, a C₁-C₄ haloalkyl group, a C₁-C₄ hydroxyalkyl group, a C₃-C₇ cycloalkyl, a phenyl group, a pyridyl group, a pyrimidinyl group, a piperidyl group, or a -C(O)-(CH₂)ₙ-NR₁₀R₁₁ group; wherein n is 0, 1 or 2;
R₆ represents a hydrogen atom, a halogen atom, a cyano group, a linear or branched C₁-C₄ alkyl group, a C₁-C₄ haloalkyl group, a C₁-C₄ hydroxyalkyl group, a C₃-C₇ cycloalkyl group, a monocyclic or bicyclic C₆-C₁₄ aryl group, a 5- to 9- membered heteroaryl group containing one, two or three heteroatoms selected from O, S and N, a 5- to 9- membered heterocyclyl group containing one, two or three heteroatoms selected from O, S and N,
wherein the cycloalkyl, aryl, heteroaryl, and heterocyclyl groups are unsubstituted or substituted by one, two or three substituents selected from a halogen atom, a cyano group, a linear or branched C₁-C₆ alkyl group, a C₁-C₄ haloalkyl group, a C₁-C₄ hydroxyalkyl group, a C₃-C₇ cycloalkyl group, a phenyl group, a pyridyl group, a pyrimidinyl group, a piperidyl group, a -(CH₂)₁₋₃CN group, a -(CH₂)ₙOR₁₁ group, a -NR₁₀R₁₁ group, a -NR₁₀C(O)-(CH₂)ₙ-R₁₁ group, a -NR₁₀C(O)-(CH₂)ₙ-NR₁₁ R₁₂ group, a -C(O)-(CH₂)₁₋₃-CN group, a -C(O)-(CH₂)ₙ-R₁₁ group, a -C(O)-(CH₂)ₙ-NR₁₀R₁₁ group, a -S(O)₂(CH₂)ₙR₁₁ group, a -S(O)₂(CH₂)ₙNR₁₀R₁₁ group, or a -NR₁₀S(O)₂(CH₂)ₙNR₁₁R₁₂ group; wherein each n is 0, 1 or 2;
R₇ represents a hydrogen atom or a linear or branched C₁-C₆ alkyl group, which alkyl group is unsubstituted or substituted by a C₁-C₂ alkoxy group; R₈ and R₉ each independently represent a hydrogen atom or a linear or branched C₁-C₃ alkyl group;
R₁₀, R₁₁ and R₁₂ each independently represent a hydrogen atom or a linear or branched C₁-C₃ alkyl group.

In a further particular preferred embodiment, in the compound of formula (I):
m is 0 or 1;
X is a nitrogen atom and Y is a -CR₅ group; or Y is a nitrogen atom and X is a -CR₅ group; or both X and Y are a -CR₅ group;
A is a nitrogen atom and B is a -CR₆ group; or B is a nitrogen atom and A is a -CR₆ group; or both A and B are a -CR₆ group;
W represents a linker selected from a -NR₇- group or a -(CR₈R₉)- group;
R₁ represents a hydrogen atom, a linear or branched C₁-C₃ alkyl group, a C₁-C₃ haloalkyl group or a C₁-C₃ hydroxyalkyl group;
R₂ represents a linear or branched C₁-C₄ alkyl group, a C₁-C₄ haloalkyl group, a C₁-C₄ hydroxyalkyl group, a C₃-C₇ cycloalkyl group, a phenyl group, a pyridyl group, a pyrimidinyl group, a pyrrolidinyl group, a piperidyl group, a tetrahydropyranyl group or a morpholinyl group,
   wherein the cycloalkyl, phenyl, pyridyl, pyrimidinyl, pyrrolidinyl, piperidinyl, tetrahydropyranyl or morpholinyl groups are unsubstituted or substituted by one, two or three substituents selected from a halogen atom, a cyano group, a linear or branched C₁-C₃ alkyl group, a C₁-C₄ haloalkyl group, a C₁-C₄ hydroxyalkyl group, a C₃-C₇ cycloalkyl, a -(CH₂)₁₋₃CN group, a -(CH₂)ₙOR₁₁ group, a -NR₁₀R₁₁ group, a -C(O)-(CH₂)₁₋₃-CN group, a -C(O)-(CH₂)ₙ-R₁₀ group, a -C(O)-(CH₂)ₙ-NR₁₀R₁₁ group, a -S(O)₂(CH₂)ₙR₁₁ group or a - S(O)₂(CH₂)ₙNR₁₀R₁₁ group; wherein each n is 0, 1 or 2; and wherein R₁₀ is a hydrogen atom, a cyano group, a linear or branched C₁-C₃ alkyl group, a C₁-C₄ haloalkyl group or a C₃-C₇ cycloalkyl group and wherein R₁₁ is a hydrogen atom or a linear or branched C₁-C₃ alkyl group;
R₃ and R₄ each independently represent a hydrogen atom or a linear or branched C₁-C₃ alkyl group;
R₅ represents a hydrogen atom, a halogen atom, a cyano group, a linear or branched C₁-C₃ alkyl group, a C₁-C₄ haloalkyl group, a C₁-C₄ hydroxyalkyl group, a C₃-C₇ cycloalkyl group, a phenyl group, a pyridyl group, a pyrimidinyl group, a pyrrolidinyl group, a piperidyl group, a tetrahydropyranyl group or a morpholinyl group;
R₆ represents a hydrogen atom, a halogen atom, a cyano group, a linear or branched C₁-C₃ alkyl group, a C₁-C₄ haloalkyl group, a C₁-C₄ hydroxyalkyl group, a C₃-C₇ cycloalkyl group, a phenyl group, a pyridyl group, a pyrimidinyl group, a pyrrolidinyl group, a piperidyl group, a tetrahydropyranyl group or a morpholinyl group;
R₇ represents a hydrogen atom or a linear or branched C₁-C₃ alkyl group;
R₈ and R₉ each independently represent a hydrogen atom or a linear or branched C₁-C₃ alkyl group;

In a further particular preferred embodiment, in the compound of formula (I):
m is 0 or 1;
X is a nitrogen atom and Y is a -CR₅ group; or Y is a nitrogen atom and X is a -CR₅ group; or both X and Y are a -CR₅ group;
A is a nitrogen atom and B is a -CR₆ group; or B is a nitrogen atom and A is a -CR₆ group; or both A and B are a -CR₆ group;
W represents a -NR₇- group;
R₁ represents a hydrogen atom, a C₁-C₃ haloalkyl group, a C₁-C₃ hydroxyalkyl group or a linear or branched C₁-C₃ alkyl group;
R₂ represents a C₃-C₇ cycloalkyl group, a phenyl group, a pyridyl group, a pyrimidinyl group, a tetrahydropyranyl group, or a tetrahydronaphthalenyl group,
   wherein the cycloalkyl, phenyl, pyridyl, pyrimidinyl. tetrahydropyranyl and tetrahydronaphthalenyl groups are unsubstituted or substituted by one, two or three substituents selected from a halogen atom, a linear or branched C₁-C₃ alkyl group, a C₁-C₃ haloalkyl group, a -(CH₂)₁₋₃CN group or a -(CH₂)ₙOR₁₁ group; wherein each n is 0, 1 or 2;
R₃ and R₄ each independently represent a hydrogen atom or a methyl group;
R₅ represents a hydrogen atom, a halogen atom, a linear or branched C₁-C₃ alkyl group, a C₁-C₃ haloalkyl group or a C₃-C₇ cycloalkyl group;
R₆ represents a hydrogen atom, a halogen atom, a linear or branched C₁-C₃ alkyl group, a C₁-C₃ haloalkyl group, a C₃-C₇ cycloalkyl group or a morpholinyl group;
R₇ represents a hydrogen atom or a methyl group;
R₁₁ represents a hydrogen atom or a methyl group.

In yet a further particular preferred embodiment, in the compound of formula (I):
m is 0 or 1;
X is a nitrogen atom and Y is a -CR₅ group; or Y is a nitrogen atom and X is a -CR₅ group; or both X and Y are a -CR₅ group;
A is a nitrogen atom and B is a -CR₆ group;; or both A and B are a -CR₆ group;
W represents a -NR₇- group;
R₁ represents a hydrogen atom, a C₁-C₃ hydroxyalkyl group or a linear or branched C₁-C₃ alkyl group;
R₂ represents a C₃-C₇ cycloalkyl group, a pyridyl group, a pyrimidinyl group, a tetrahydropyranyl group, or a tetrahydronaphthalenyl group,
   wherein the cycloalkyl, pyridyl, pyrimidinyl, tetrahydropyranyl and tetrahydronaphthalenyl groups are unsubstituted or substituted by one, two or three substituents selected from a halogen atom, a linear or branched C₁-C₃ alkyl group, a -(CH₂)₁₋₃CN group or a -(CH₂)ₙOR₁₁ group; wherein each n is 0, 1 or 2;
R₃ and R₄ each independently represent a hydrogen atom or a methyl group;
R₅ represents a hydrogen atom, a halogen atom, a linear or branched C₁-C₃ alkyl group, or a C₁-C₃ haloalkyl group;
R₆ represents a hydrogen atom, a halogen atom or a morpholinyl group;
R₇ represents a hydrogen atom or a methyl group; and
R₁₁ represents a hydrogen atom or a methyl group.

Particular individual compounds of the invention include:
2-(2-Oxo-1,2-dihydropyridin-3-ylamino)-9-(tetrahydro-2H-pyran-4-yl)-7H-purin-8(9H)-one;
2-(5-chloro-2-oxo-1,2-dihydropyridin-3-ylamino)-9-(tetrahydro-2H-pyran-4-yl)-7H-purin-8(9H)-one;
2-(5-fluoro-2-oxo-1,2-dihydropyridin-3-ylamino)-9-(tetrahydro-2H-pyran-4-yl)-7H-purin-8(9H)-one;
2-(5-methyl-2-oxo-1,2-dihydropyridin-3-ylamino)-9-(tetrahydro-2H-pyran-4-yl)-7H-purin-8(9H)-one;
2-(5-(difluoromethyl)-2-oxo-1,2-dihydropyridin-3-ylamino)-9-(tetrahydro-2H-pyran-4-yl)-7H-purin-8(9H)-one;
(R)-9-(1-(5-Fluoropyridin-2-yl)ethyl)-2-(2-oxo-1,2-dihydropyridin-3-ylamino)-7H-purin-8(9H)-one;
2-((1r,4r)-4-(8-Oxo-2-(2-oxo-1,2-dihydropyridin-3-ylamino)-7H-purin-9(8H)-yl) cyclohexyl)acetonitrile;
7-methyl-2-(2-oxo-1,2-dihydropyridin-3-ylamino)-9-(tetrahydro-2H-pyran-4-yl)-7H-purin-8(9H)-one;
7-Methyl-2-(methyl(2-oxo-1,2-dihydropyridin-3-yl)amino)-9-(tetrahydro-2H-pyran-4-yl)-7H-purin-8(9H)-one;
7-(2-hydroxyethyl)-2-(2-oxo-1,2-dihydropyridin-3-ylamino)-9-(tetrahydro-2H-pyran-4-yl)-7H-purin-8(9H)-one;
5-(2-Oxo-1,2-dihydropyridin-3-ylamino)-3-(tetrahydro-2H-pyran-4-yl)-1 H-imidazo [4,5-b]pyridin-2(3H)-one;
6-fluoro-5-(2-oxo-1,2-dihydropyridin-3-ylamino)-3-(tetrahydro-2H-pyran-4-yl)-1 H-imidazo[4,5-b]pyridin-2(3H)-one;
6-morpholino-2-(2-oxo-1,2-dihydropyridin-3-ylamino)-9-(tetrahydro-2H-pyran-4-yl)-7H-purin-8(9H)-one;
2-(6-Oxo-1,6-dihydropyrimidin-5-ylamino)-9-(tetrahydro-2H-pyran-4-yl)-7H-purin-8(9H)-one;
(R)-9-(1-(5-fluoropyrimidin-2-yl)ethyl)-2-(6-oxo-1,6-dihydropyrimidin-5-ylamino)-7H-purin-8(9H)-one;
2-(3-oxo-2,3-dihydropyridazin-4-ylamino)-9-(tetrahydro-2H-pyran-4-yl)-7H-purin-8(9H)-one;
2-(6-chloro-3-oxo-2,3-dihydropyridazin-4-ylamino)-9-(tetrahydro-2H-pyran-4-yl)-7H-purin-8(9H)-one;
(2-(1r,4r)-4-{2-[(5-Chloro-2-oxo-1,2-dihydropyridin-3-yl)amino]-8-oxo-7,8-dihydro-9H-purin-9-yl}cyclohexyl)acetonitrile;
(R)-2-(5-fluoro-2-oxo-1,2-dihydropyridin-3-ylamino)-9-(1-(5-fluoropyridin-2-yl)ethyl)-7H-purin-8(9H)-one;
(R)-9-(1-(5-fluoropyridin-2-yl)ethyl)-2-(5-methyl-2-oxo-1,2-dihydropyridin-3-ylamino)-7H-purin-8(9H)-one;
(R)-2-(5-chloro-2-oxo-1,2-dihydropyridin-3-ylamino)-9-(1-(5-fluoropyridin-2-yl)ethyl)-7H-purin-8(9H)-one;
2-(5-fluoro-2-oxo-1,2-dihydropyridin-3-ylamino)-9-((1r,4r)-4-hydroxycyclohexyl)-7H-purin-8(9H)-one;
9-((1 R,4R)-5,7-difluoro-4-hydroxy-1,2,3,4-tetrahydronaphthalen-1-yl)-2-(2-oxo-1,2-dihydropyridin-3-ylamino)-7H-purin-8(9H)-one;
9-((1S,2R)-2-methylcyclohexyl)-2-(2-oxo-1,2-dihydropyridin-3-ylamino)-7H-purin-8(9H)-one;
9-((1r,4r)-4-(hydroxymethyl)cyclohexyl)-2-(2-oxo-1,2-dihydropyridin-3-ylamino)-7H-purin-8(9H)-one;
   or a pharmaceutically acceptable salt, or solvate, or N-oxide, or stereoisomer or deuterated derivative thereof.

Examples of the preferred compounds are:
2-(5-chloro-2-oxo-1,2-dihydropyridin-3-ylamino)-9-(tetrahydro-2H-pyran-4-yl)-7H-purin-8(9H)-one;
(R)-9-(1-(5-Fluoropyridin-2-yl)ethyl)-2-(2-oxo-1,2-dihydropyridin-3-ylamino)-7H-purin-8(9H)-one;
2-((1r,4r)-4-(8-Oxo-2-(2-oxo-1,2-dihydropyridin-3-ylamino)-7H-purin-9(8H)-yl) cyclohexyl)acetonitrile;
(2-(1r,4r)-4-{2-[(5-Chloro-2-oxo-1,2-dihydropyridin-3-yl)amino]-8-oxo-7,8-dihydro-9H-purin-9-yl}cyclohexyl)acetonitrile;
or a pharmaceutically acceptable salt, or solvate, or N-oxide, or stereoisomer or deuterated derivative thereof.

According to one embodiment of the present invention, compounds of general formula (I) may be prepared from compounds of formula (II) as illustrated in Scheme 1.

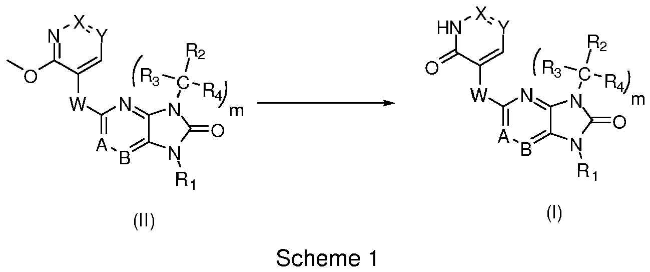

When the defined R groups are susceptible to chemical reaction under the conditions of the hereinbefore described processes or are incompatible with said processes, conventional protecting groups may be used in accordance with standard practice, for example see T. W. Greene and P. G. M. Wuts in 'Protective Groups in Organic Synthesis', 3rd Edition, John Wiley & Sons (1999). It may be that deprotection will form the last step in the synthesis of compounds of formula (I).

The term amino-protecting group refers to a protecting group suitable for preventing undesired reactions at amino nitrogen. Representative amino-protecting groups include, but are not limited to, formyl; acyl groups, for example alkanoyl groups such as acetyl; alkoxycarbonyl groups such as tert-butoxycarbonyl (Boc); arylmethoxycarbonyl groups such as benzyloxycarbonyl (Cbz) and 9-fluorenylmethoxycarbonyl (Fmoc); arylmethyl groups such as benzyl (Bn), trityl (Tr), and 1,1-di-(4'-methoxyphenyl)methyl; silyl groups, such as trimethylsilyl (TMS), tert-butyldimethylsilyl (TBS); trimethylsiloxyethoxymethyl (SEM) and the like.

The term hydroxy-protecting group refers to a protecting group suitable for preventing undesired reactions at a hydroxy group. Representative hydroxy-protecting groups include, but are not limited to, alkyl groups, such as methyl, ethyl, and tert-butyl; acyl groups, for example alkanoyl groups, such as acetyl; arylmethyl groups, such as benzyl (Bn), p-methoxybenzyl (PMB), 9-fluorenylmethyl (Fm), and diphenylmethyl (benzhydryl, DPM); Tetrahydropyranyl ethers (THP ethers) such as methoxy-THP or ethoxy-THP; silyl groups, such as trimethylsilyl (TMS), tert-butyldimethylsilyl (TBS); trimethylsiloxyethoxymethyl (SEM) and the like.

Treatment of compounds of formula (II) with a suitable reagent, such as a mixture of trimethylsilyl chloride and sodium iodide in a solvent such as acetonitrile at temperatures ranging from ambient temperature to reflux gives rise to compounds of formula (I).

In the particular case of formula (II) where W = NH, compounds of subformula (II-a) may be prepared by the synthetic approach as shown in Scheme 2.

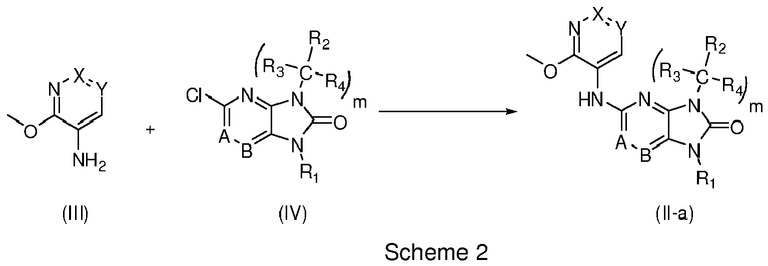

Treatment of amines of formula (III) with compounds of formula (IV) in the presence of a suitable catalyst, such as the catalytic species generated from (tris (dibenzylideneacetone)dipalladium (0) and 9,9-dimethyl-4,5-bis(diphenylphosphino) xanthene, and a base such as cesium carbonate in a solvent such as 1,4-dioxane at temperatures ranging from 80-120 °C gives rise to compounds of formula (II-a).

Compounds of general formula (II-a) in which the residue -(R₃-C-R₄)ₘ-R₂ or R₁ contains a "protected" heteroatom, such as nitrogen or oxygen, may be "deprotected" by removal of the protecting group to give compounds of formula (IIa) in which the residue -(R₃-C-R₄)ₘ-R₂ or R₁ contains the "deprotected" heteroatom. Typical examples of protecting groups for heteroatoms, such as nitrogen and oxygen, and their removal (deprotection) may be found in several textbooks, for example: Greene's Protective Groups in Organic Synthesis, ISBN: 0471697540. Furthermore said "deprotected" heteroatoms may be further functionalized by, for example, alkylation, amidation, sulfonamidation or arylation under standard reaction conditions.

In the particular cases of compounds of formula (II-a) in which R₁ represents in its entirety an appropriate nitrogen protecting group such as the trimethylsilylethoxymethyl (SEM) moiety, then this group may be subsequently removed under appropriate conditions with, for example, tetrabutylammonium fluoride in a solvent such as tetrahydrofuran at temperatures ranging from ambient temperature to reflux to give compounds of formula (II-a) in which R₁ now represents a hydrogen atom.

In another synthetic pathway compounds of formula (II-a) may also be prepared by the synthetic route as illustrated in Scheme 3.

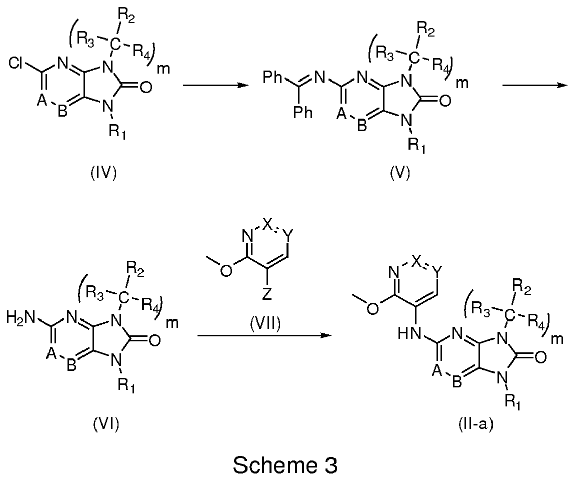

Compounds of formula (IV) may be reacted with benzophenone imine in the presence of a base such as cesium carbonate in the presence of a suitable catalyst such as the catalytically active species generated from palladium (II) acetate and 2,2'-bis (diphenylphosphino)-1,1'-binaphthyl in a solvent such as toluene at temperatures ranging from 80 °C to reflux to give imines of formula (V).

Compounds of formula (V) may be deprotected to give amines of formula (VI) under standard conditions, for example, by treatment with hydroxylamine hydrochloride in the presence of a base such as sodium acetate in a solvent such as methanol at ambient temperature.

Treatment of amines of formula (VI) with compounds of formula (VII), where Z represents an halogen atom, in the presence of a suitable catalyst, such as the catalytic species generated from (tris(dibenzylideneacetone)dipalladium (0) and 9,9-dimethyl-4,5-bis(diphenylphosphino)xanthene, and a base such as cesium carbonate in a solvent such as 1,4-dioxane at temperatures ranging from 80-120 °C gives rise to compounds of formula (II-a).

In yet another synthetic pathway compounds of formula (II-a) may also be prepared by the synthetic route as illustrated in Scheme 4.

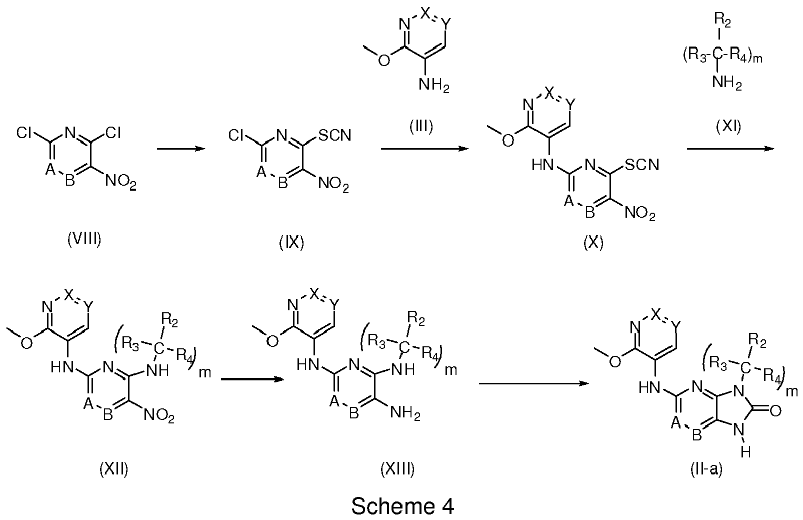

Thiocyanates of formula (IX) may be accessed from compounds of formula (VIII) by selective displacement of one of the halogen atoms with potassium thiocyanate in a solvent such as acetic acid at temperatures ranging from 0 ºC to ambient temperature.

Compounds of formula (IX) may be reacted with amines of formula (III) in the presence of a base, such as *N*,*N*-diisopropylethylamine or triethylamine, in a solvent such as ethanol at temperatures ranging from -78 °C to ambient temperature to furnish compounds of formula (X).

Compounds of formula (X) may be reacted with amines of formula (XI) in the presence of a base, such as *N*,*N*-diisopropylethylamine, in a solvent such as tetrahydrofuran at temperatures ranging from ambient temperature to reflux to furnish compounds of formula (XII).

Compounds of formula (XII) may in turn be converted to amines of formula (XIII) by reduction with hydrogen gas at atmospheric pressure using a suitable catalyst such as palladium on carbon in a solvent such as ethanol at ambient temperature.

Treatment of compounds of formula (XIII) with a suitable reagent such as 1,1'-carbonylbis-1*H*-imidazole in a solvent such as tetrahydrofuran or acetonitrile at temperatures ranging from ambient temperature to reflux furnishes compounds of formula (II-a).

Intermediate compounds of general formula (IV) may be prepared by the following synthetic route as illustrated in Scheme 5.

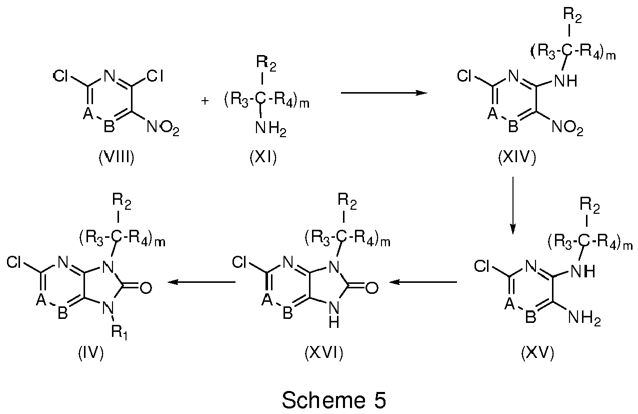

Compounds of formula (VIII) may be reacted with amines of formula (XI), in the presence of a base, such as *N*,*N*-diisopropylethylamine or triethylamine, in a solvent such as dichloromethane, chloroform or tetrahydrofuran at temperatures ranging from - 78 °C to reflux to furnish compounds of formula (XIV).

Compounds of formula (XIV) may in turn be converted to amines of formula (XV) by treatment with tin (II) chloride dihydrate in a solvent such as ethanol at temperatures ranging from 20-100 °C or by reduction with hydrogen gas at atmospheric pressure using a suitable catalyst such as platinum on carbon in the presence of an additive such as zinc bromide in a solvent such as ethyl acetate at ambient temperature. Compounds of formula (XV) may be converted into compounds of formula (XVI) by treatment with a suitable reagent such as 1,1'-carbonylbis-1*H*-imidazole in a solvent such as tetrahydrofuran or acetonitrile at temperatures ranging from ambient temperature to reflux.

Treatment of compounds of formula (XVI) with a suitable base such as sodium hydride or potassium carbonate in a solvent such as *N*,*N'*-dimethylformamide followed by addition of an electrophile, for example methyl iodide or (2-(chloromethoxy) ethyl)trimethylsilane at temperatures ranging from 0-100 °C furnishes compounds of formula (IV).

In the particular case of formula (XIV) where A = N and B represents a -CR₆ group (R₆ is as defined in the claims section), compounds of subformula (XIV-a) may be prepared by the synthetic approach as shown in Scheme 6.

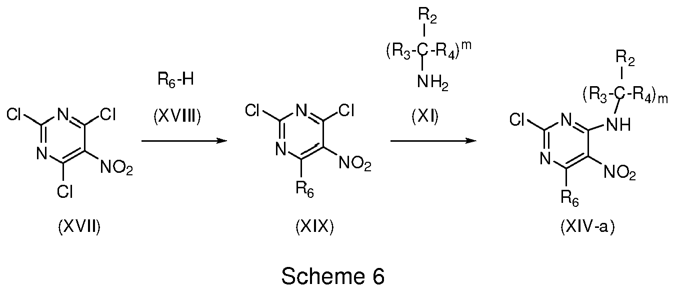

2,4,6-Trichloro-5-nitropyrimidine (XVII) when treated with an appropriate nucleophile of formula (XVIII), such as morpholine, in the presence of a base, such as triethylamine, in a solvent such as dichloromethane at temperatures ranging from 0-25 °C gives rise to compounds of formula (XIX).

Compounds of formula (XIX) may be reacted with amines of formula (XI), in the presence of a base, such as *N*,*N*-diisopropylethylamine or triethylamine, in a solvent such as dichloromethane, chloroform or tetrahydrofuran at temperatures ranging from - 78 °C to reflux to furnish compounds of formula (XIV-a).

Compounds of formula (XVI) may also be prepared by the following synthetic route as illustrated in Scheme 7.

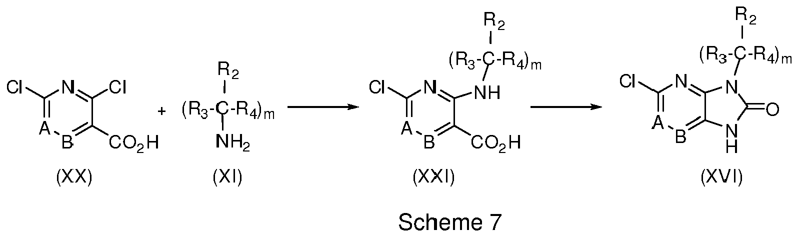

Compounds of formula (XXI) may be accessed from carboxylic acids of formula (XX) by selective displacement of one of the halogen atoms with an amine of formula (XI) such as tetrahydro-2*H*-pyran-4-amine in the presence of a base, such as *N*,*N-*diisopropylethylamine, in a solvent such as acetonitrile at temperatures ranging from 80-130 °C under microwave irradiation.

Compounds of formula (XXI) may be converted into compounds of formula (XVI) by treatment with a reagent such as diphenylphosphoryl azide in the presence of a base such as triethylamine in a suitable solvent such as 1,4-dioxane at temperatures ranging from ambient temperature to reflux.

### EXAMPLES

The syntheses of the compounds of the invention and of the intermediates for use therein are illustrated by the following Examples (1-25) (including Preparation Examples (Preparations 1-34)) are given in order to provide a person skilled in the art with a sufficiently clear and complete explanation of the present invention, but should not be considered as limiting of the essential aspects of its subject, as set out in the preceding portions of this description.

### PREPARATION 1

### 2-Chloro-9-(tetrahydro-2H-pyran-4-yl)-7-((2-(trimethylsilyl)ethoxy)methyl)-7H-purin-8(9H)one

### a) 2-Chloro-5-nitro-N-(tetrahydro-2H-pyran-4-yl)pyrimidin-4-amine

Diisopropylethylamine (19.80 mL, 110 mmol) was added dropwise over 15 minutes to a stirred suspension of 2,4-dichloro-5-nitropyrimidine (11.56 g, 60 mmol) and tetrahydro-2*H*-pyran-4-amine hydrochloride (prepared as described in WO200424728(A2), 7.81 g, 60 mmol) in dichloromethane (400 mL) at -78 ºC under a nitrogen atmosphere. The reaction mixture was stirred at -78 ºC for 2 hours and then was allowed to warm to ambient temperature. The solvent was evaporated, water was added and the resultant solid was filtered, washed with water and dried to yield the title compound (13.62 g, 93%) as a yellow solid.
LRMS (m/z): 259 (M+1)⁺.
¹H NMR δ (300 MHz, CDCl₃): 1.62-1.80 (m, 2H), 2.06 (d, 2H), 3.59 (t, 2H), 4.04 (d, 2H), 4.45 (td, 1 H), 8.33 (br s, 1 H), 9.07 (s, 1 H).

### b) 2-Chloro-N⁴-(tetrahydro-2H-pyran-4-yl)pyrimidine-4,5-diamine

Zinc bromide (2.37 g, 10.5 mmol) and 5% platinum on carbon (5.13 g, 25.7 mmol) were added to a solution of 2-chloro-5-nitro-*N*-(tetrahydro-2*H*-pyran-4-yl)pyrimidin-4-amine (Preparation 1a, 13.62 g, 51.0 mmol) in ethyl acetate (200 mL) and the reaction mixture was stirred at ambient temperature overnight under a hydrogen atmosphere. The mixture was then filtered through diatomaceous earth (Celite®) and the filter cake was washed with methanol. The combined filtrate and washings were concentrated to give the title compound (11.9 g, 100%) as a solid.
LRMS (m/z): 229 (M+1)⁺.
¹H NMR δ (300 MHz, DMSO-*d*₆): 1.35-1.62 (m, 2H), 1.85 (d, 2H), 3.40 (t, 2H), 3.87 (d, 2H), 4.03 (m, 1H), 4.96 (br s, 2H), 6.66 (d, 1H), 7.38 (s, 1H).

### c) 2-Chloro-9-(tetrahydro-2H-pyran-4-yl)-7,9-dihydro-8H-purin-8-one

A mixture of 2-chioro-*N*⁴-(tetrahydro-2*H*-pyran-4-yl)pyrimidine-4,5-diamine (Preparation 1b, 5.40 g, 20 mmol) and 1,1'-carbonylbis-1*H*-imidazole (5.74 g, 40 mmol) in acetonitrile (100 mL) was stirred and heated to 80 ºC in a sealed tube. After 2 hours, the solvent was evaporated under reduced pressure and the residue was dissolved in 2M aqueous hydrochloric acid solution. 2M Aqueous sodium hydroxide solution was added until the pH reached approximately 7 and the resultant precipitate was filtered and dried to give the title compound (4.80 g, 80%) as a white solid.
LRMS (m/z): 255 (M+1)⁺.
¹H NMR δ (300 MHz, CDCl₃): 1.75 (m, 2H), 2.68-2.86 (m, 2H), 3.55 (m, 2H), 4.16 (m, 2H), 4.53-4.68 (m, 1 H), 8.16 (s, 1 H).

### d) 2-Chloro-9-(tetrahydro-2H-pyran-4-yl)-7-{[2-(trimethylsilyl)ethoxy]methyl}-7,9-dihydro-8H purin-8-one

Sodium hydride (60% dispersion in mineral oil, 0.40 g, 10.0 mmol) was added portion wise to a stirred solution of 2-chloro-9-(tetrahydro-2*H*-pyran-4-yl)-7,9-dihydro-8*H*-purin-8-one (Preparation 1c, 1.98 g, 7.8 mmol) in *N,N'*-dimethylformamide (30 mL) at 0 ºC under an argon atmosphere. After 15 minutes, (2-(chloromethoxy)ethyl)trimethylsilane (1.53 mL, 8.6 mmol) was added and the mixture was warmed to ambient temperature and stirred for 4 hours. The mixture was then partitioned between water and ethyl acetate and the organic layer was washed with water and brine, dried (MgSO₄) and the solvent was evaporated under reduced pressure. The residue was purified by flash chromatography (99:1 dichloromethane/methanol) to give the title compound (2.94 g, 98%) as a pale yellow oil.
LRMS (m/z): 385 (M+1)⁺.
¹H NMR δ (300 MHz, CDCl₃): -0.20-0.08 (m, 9H), 0.92 (m, 2H), 1.73 (m, 2H), 2.62-2.84 (m, 2H), 3.39-3.71 (m, 4H), 4.15 (m, 2H), 4.48-4.76 (m, 1 H), 5.31 (s, 2H), 8.18 (s, 1 H).

### PREPARATION 2

### 2-(2-Methoxypyridin-3-ylamino)-9-(tetrahydro-2H-pyran-4-yl)-7H-purin-8(9H)-one

### a) 2-(2-Methoxypyridin-3-ylamino)-9-(tetrahydro-2H-pyran-4-yl)-7-((2-(trimethyl silyl)ethoxy)methyl)-7H-purin-8(9H)-one

An oven-dried resealable Schlenk tube was charged with 2-chloro-9-(tetrahydro-2H-pyran-4-yl)-7-{[2-(trimethylsilyl)ethoxy]methyl}-7,9-dihydro-8*H-*purin-8-one (Preparation 1d, 0.400 g, 1.04 mmol), 2-methoxypyridin-3-amine (0.142 g, 1.14 mmol), cesium carbonate (0.677 g, 2.08 mmol) and 1,4-dioxane (9 mL). The Schlenk tube was subjected to three cycles of evacuation-backfilling with argon then (tris(dibenzylideneacetone)dipalladium (0) (0.095 g, 0.1 mmol) and 9,9-dimethyl-4,5-bis(diphenylphosphino)xanthene (0.060 g, 0.1 mmol) were added. After three further cycles of evacuation-backfilling with argon, the Schlenk tube was capped and then stirred and heated to 100 ºC. After 2 hours the mixture was cooled, diluted with ethyl acetate and filtered through Celite®. The filtrate was evaporated and the residue was purified by flash chromatography (3:1 hexanes/ethyl acetate) to give the title compound (0.375 g, 76%) as a pale yellow oil.
LRMS (m/z): 473 (M+1)⁺.
¹H NMR δ (300 MHz, CDCl₃): 0.01 (s, 9H), 0.95 (d, 2H), 1.74 (d, 2H), 2.84 (m, 2H), 3.59 (m, 4H), 4.10 (s, 3H), 4.16 (m, 2H), 4.57 (m, 1 H), 5.29 (s, 2H), 6.95 (m, 1 H), 7.48 (m, 1 H), 7.69 (m, 1 H), 8.11 (m, 1 H), 8.76 (s, 1 H).

### b) 2-(2-Methoxypyridin-3-ylamino)-9-(tetrahydro-2H-pyran-4-yl)-7H-purin-8(9H)-one

Tetrabutylammonium fluoride (1M solution in tetrahydrofuran, 2.35 mL, 2.35 mmol) was added to a solution of 2-(2-methoxypyridin-3-ylamino)-9-(tetrahydro-2*H*-pyran-4-yl)-7-((2-(trimethylsilyl)ethoxy)methyl)-7*H*-purin-8(9*H*)-one (Preparation 2a, 0.370 g, 0.78 mmol) in tetrahydrofuran (1 mL) and the mixture was stirred and heated to 80 ºC in a sealed tube. After 6 hours, the mixture was concentrated and water was added. The precipitate was filtered and washed with water to give the title compound (0.230 g, 86%) as a beige solid.
LRMS (m/z): 343 (M+1)⁺.
¹H NMR δ (300 MHz, DMSO-*d*₆): 1.72 (d, 2H), 2.63 (m, 2H), 3.54 (m, 2H), 4.02-4.08 (m, 5H), 4.47 (m, 1 H), 7.04 (dd, 1 H), 7.81 (m, 1 H), 7.87 (s, 1 H), 8.05 (s, 1H), 8.60 (dd, 1 H), 11.17 (s, 1H).

### PREPARATION 3

### 5-Chloro-2-methoxypyridin-3-amine

### a) 5-Chloro-2-methoxy-3-nitropyridine

A solution of sodium methoxide (0.84 g, 16.6 mmol) in methanol (4 mL) was added dropwise to a solution of 2,5-dichloro-3-nitropyridine (1.00 g, 5.2 mmol) in methanol (10 mL) and the mixture was stirred and heated to reflux. After 7 hours, the mixture was cooled and diluted with water and the precipitate was filtered and washed with water to give the title compound (0.95 g, 97%) as a white solid.
¹H NMR δ (300 MHz, CDCl₃): 4.11 (s, 3H), 8.23 (s, 1H), 8.32 (s, 1H).

### b) 5-Chloro-2-methoxypyridin-3-amine

Obtained as a white solid in quantitative yield from 5-chloro-2-methoxy-3-nitropyridine (Preparation 3a) following the experimental procedure as described in Preparation 1b.
LRMS (m/z): 159 (M+1)⁺.
¹H NMR δ (300 MHz, CDCl₃): 3.97 (s, 3H), 6.98 (s, 1H), 7.50 (s, 1H).

### PREPARATION 4

### 2-(5-Chloro-2-methoxypyridin-3-ylamino)-9-(tetrahydro-2H-pyran-4-yl)-7H-purin-8(9H)-one

### a) 2-(5-Chloro-2-methoxypyridin-3-ylamino)-9-(tetrahydro-2H-pyran-4-yl)-7-((2-(trimethylsilyl)ethoxy)methyl)-7H-purin-8(9H)-one

Obtained as a pale brown solid (68%) from 2-chloro-9-(tetrahydro-2*H*-pyran-4-yl)-7-{[2-(trimethylsilyl)ethoxy]methyl}-7,9-dihydro-8*H*-purin-8-one (Preparation 1 d) and 5-Chloro-2-methoxypyridin-3-amine (Preparation 3b) following the experimental procedure as described in Preparation 2a.
LRMS (m/z): 507 (M+1)⁺.
¹H NMR δ (300 MHz, CDCl₃): 0.01 (s, 9H), 0.95 (m, 2H), 1.77 (d, 2H), 2.81 (m, 2H), 3.60 (m, 4H), 4.07 (s, 3H), 4.15 (m, 2H), 4.54 (m, 1H), 5.30 (s, 2H), 7.56 (s, 1H), 7.71 (s, 1H), 8.16 (s, 1H), 8.87 (s, 1H).

### b) 2-(5-Chloro-2-methoxypyridin-3-ylamino)-9-(tetrahydro-2H-pyran-4-yl)-7H-purin-8(9H)-one

Obtained as a beige solid (79%) from 2-(5-chloro-2-methoxypyridin-3-ylamino)-9-(tetrahydro-2*H*-pyran-4-yl)-7-((2-(trimethylsilyl)ethoxy)methyl)-7*H*-purin-8(9*H*)-one (Preparation 4a) following the experimental procedure as described in Preparation 2b.
LRMS (m/z): 377 (M+1)⁺.
¹H NMR δ (300 MHz, DMSO-*d*₆): 1.73 (d, 2H), 2.58 (m, 2H), 3.42 (m, 2H), 4.03-4.10 (m, 5H), 4.48 (m, 1H), 7.82 (s, 1H), 8.01 (s, 1H), 8.11 (s, 1H), 8.76 (s, 1H).

### PREPARATION 5

### 5-Fluoro-2-methoxypyridin-3-amine

### a) 5-Fluoro-3-nitropyridin-2-ol

A mixture of concentrated sulphuric acid (1 mL) and fuming nitric acid (1 mL) was added dropwise to a stirred, cooled (ice-bath) mixture of 5-fluoropyridin-2-ol (1.20 g, 10.6 mmol) and concentrated sulphuric acid (2.7 mL). The mixture was warmed to ambient temperature and then to 85 °C. After 2 hours, the mixture was cooled and poured onto ice-water. The precipitate was filtered and dried to give the title compound (0.72 g, 43%) as a yellow solid.
LRMS (m/z): 157 (M-1)⁺.
¹H NMR δ (300 MHz, DMSO-*d*₆): 8.28 (s, 1H), 8.67 (s, 1H).

### b) 5-Fluoro-2-methoxy-3-nitropyridine

Iodomethane (1.97 mL, 31.7 mmol) was added to a suspension of 5-fluoro-3-nitropyridin-2-ol (Preparation 5a, 0.500 g, 3.2 mmol) and silver(I) carbonate (1.04 g, 3.8 mmol) in chloroform (15 mL) and the mixture was stirred overnight at ambient temperature. The mixture was filtered through Celite®, the filtrate was evaporated and the residue was purified by flash chromatography (2:1 hexanes/ethyl acetate) to give the title compound (0.300 g, 55%) as a white solid.
LRMS (m/z): 173 (M+1)⁺.
¹H NMR δ (300 MHz, CDCl₃): 4.17 (s, 3H), 8.15 (dd, 1H), 8.37 (d, 1H).

### c) 5-Fluoro-2-methoxypyridin-3-amine

10% Palladium on carbon (0.300 g) was added to a solution of 5-fluoro-2-methoxy-3-nitropyridine (Preparation 5b, 0.300 g, 1.74 mmol) in ethanol (15 mL) and the reaction mixture was stirred at ambient temperature under a hydrogen atmosphere. After 5 hours, the mixture was then filtered through Celite® and the filter cake was washed with ethanol. The combined filtrate and washings were concentrated to give the title compound (0.220 g, 89%) as a brown solid.
LRMS (m/z): 143 (M+1)⁺.
¹H NMR δ (300 MHz, CDCl₃): 3.96 (s, 3H), 6.67 (dd, 1H), 7.39 (d, 1H).

### PREPARATION 6

### 2-(5-Fluoro-2-methoxypyridin-3-ylamino)-9-(tetrahydro-2H-pyran-4-yl)-7H-purin-8(9H)-one

### a) 2-(5-Fluoro-2-methoxypyridin-3-ylamino)-9-(tetrahydro-2H-pyran-4-yl)-7-((2-(trimethylsilyl)ethoxy)methyl)-7H-purin-8(9H)-one

Obtained as a yellow solid (64%) from 2-chloro-9-(tetrahydro-2*H*-pyran-4-yl)-7-{[2-(trimethylsilyl)ethoxy]methyl}-7,9-dihydro-8*H*-purin-8-one (Preparation 1d) and 5-fluoro-2-methoxypyridin-3-amine (Preparation 5c) following the experimental procedure as described in Preparation 2a.
LRMS (m/z): 491 (M+1)⁺.
¹H NMR δ (300 MHz, CDCl₃): 0.01 (s, 9H), 0.95 (m, 2H), 1.76 (d, 2H), 2.83 (m, 2H), 3.59 (m, 4H), 4.08 (s, 3H), 4.17 (m, 2H), 4.57 (m, 1H), 5.29 (s, 2H), 7.60 (s, 1H), 8.12 (s, 1H), 8.73 (d, 1H).

### b) 2-(5-Fluoro-2-methoxypyridin-3-ylamino)-9-(tetrahydro-2H-pyran-4-yl)-7H-purin-8(9H)-one

Obtained as a yellow solid (94%) from 2-(5-fluoro-2-methoxypyridin-3-ylamino)-9-(tetrahydro-2*H*-pyran-4-yl)-7-((2-(trimethylsilyl)ethoxy)methyl)-7*H*-purin-8(9*H*)-one (Preparation 6a) following the experimental procedure as described in Preparation 2b.
LRMS (m/z): 361 (M+1)⁺.
¹H NMR δ (300 MHz, CDCl₃): 1.72 (m, 2H), 2.79 (m, 2H), 3.55 (m, 2H), 4.06 (s, 3H), 4.14 (m, 2H), 4.54 (m, 1H), 7.56 (s, 1H), 8.12 (s, 1H), 8.69 (d, 1H), 9.44 (s, 1H).

### PREPARATION 7

### 2-Methoxy-5-methylpyridin-3-amine

### a) 2-Methoxy-5-methyl-3-nitropyridine

Obtained as a yellow solid (78%) from 2-chloro-5-methyl-3-nitropyridine and sodium methoxide following the experimental procedure as described in Preparation 3a.
LRMS (m/z): 169 (M+1)⁺.
¹H NMR δ (300 MHz, CDCl₃): 2.46 (s, 3H), 4.01 (s, 3H), 8.10 (s, 1H), 8.22 (s, 1H).

### b) 2-Methoxy-5-methylpyridin-3-amine

Obtained as a white solid (98%) from 2-methoxy-5-methyl-3-nitropyridine (Preparation 7a) following the experimental procedure as described in Preparation 5c.
LRMS (m/z): 139 (M+1)⁺.
¹H NMR δ (300 MHz, CDCl₃): 2.17 (s, 3H), 3.94 (s, 3H), 6.73 (s, 1H), 7.37 (s, 1H).

### PREPARATION 8

### 2-(2-Methoxy-5-methylpyridin-3-ylamino)-9-(tetrahydro-2H-pyran-4-yl)-7H-purin-8(9H)-one

### a) 2-(2-Methoxy-5-methylpyridin-3-ylamino)-9-(tetrahydro-2H-pyran-4-yl)-7-((2-(trimethylsilyl)ethoxy)methyl)-7H-purin-8(9H)-one

Obtained as a yellow oil (65%) from 2-chloro-9-(tetrahydro-2*H*-pyran-4-yl)-7-{[2-(trimethylsilyl)ethoxy]methyl}-7,9-dihydro-8*H*-purin-8-one (Preparation 1 d) and 2-methoxy-5-methylpyridin-3-amine (Preparation 7b) following the experimental procedure as described in Preparation 2a.
LRMS (m/z): 487 (M+1)⁺.
¹H NMR δ (300 MHz, CDCl₃): 0.01 (s, 9H), 0.93 (m, 2H), 1.79 (d, 2H), 2.33 (s, 3H), 2.82 (m, 2H), 3.58 (m, 4H), 4.04 (s, 3H), 4.16 (m, 2H), 4.56 (m, 1H), 5.29 (s, 2H), 7.57 (br s, 2H), 8.11 (s, 1H), 8.63 (s, 1H).

### b) 2-(2-Methoxy-5-methylpyridin-3-ylamino)-9-(tetrahydro-2H-pyran-4-yl)-7H-purin-8(9H)-one

Obtained as a beige solid (51%) from 2-(2-methoxy-5-methylpyridin-3-ylamino)-9-(tetrahydro-2*H*-pyran-4-yl)-7-((2-(trimethylsilyl)ethoxy)methyl)-7*H*-purin-8(9*H*)-one (Preparation 8a) following the experimental procedure as described in Preparation 2b.
LRMS (m/z): 357 (M+1)⁺.
¹H NMR δ (300 MHz, DMSO-*d*₆): 1.68 (d, 2H), 2.25 (s, 3H), 2.58 (m, 2H), 3.45 (m, 2H), 3.88 (m, 3H), 3.98 (m, 2H), 4.43 (m, 1H), 7.55 (s, 1H), 7.71 (s, 1H), 7.97 (s, 1H), 8.47 (s, 1H).

### PREPARATION 9

### 5-(Difluoromethyl)-2-methoxypyridin-3-amine

### a) Methyl 6-chloro-5-nitronicotinate

*N,N*'-Dimethylformamide (0.4 mL) was added to a suspension of 6-hydroxy-5-nitronicotinic acid (10.0 g, 50 mmol) in thionyl chloride (50 mL) and the mixture was stirred and heated to 60 °C. After gas evolution had ceased, the mixture was heated to 80 °C and stirred overnight. The mixture was concentrated *in vacuo* and then co-evaporated with toluene three times. The residue was taken up in dichloromethane (20 mL), cooled to 0 °C and methanol (12 mL) was added dropwise with stirring. The mixture was stirred for 1 hour then evaporated. The residue was partitioned between ethyl acetate and water and the organic layer was separated, dried (MgSO₄ and evaporated to give the title compound (8.83 g, 75%) as a pale yellow solid.
LRMS (m/z): 217 (M+1)⁺.
¹H NMR δ (300 MHz, CDCl₃): 4.03 (s, 3H), 8.77 (d, 1H), 9.18 (d, 1H).

### b) Methyl 6-methoxy-5-nitronicotinate

Sodium methoxide (2.1 g, 38.9 mmol) was added portionwise to a stirred suspension of methyl 6-chloro-5-nitronicotinate (Preparation 9a, 2.86 g, 13.21 mmol) in anhydrous methanol (45 mL) and the mixture was stirred overnight. The mixture was concentrated and the residue was partitioned between ethyl acetate and water and the organic layer was washed with brine, dried (MgSO₄ and evaporated to give the title compound (2.66 g, 95%) as a cream-coloured solid.
LRMS (m/z): 217 (M+1)⁺.
¹H NMR δ (300 MHz, CDCl₃): 3.98 (s, 3H), 4.20 (s, 3H), 8.83 (d, 1H), 9.01 (d, 1H).

### c) 6-Methoxy-5-nitronicotinic acid

2M Aqueous sodium hydroxide solution (7.70 mL, 15.4 mmol) was added to a stirred suspension of methyl 6-methoxy-5-nitronicotinate (Preparation 9b, 2.66 g, 12.5 mmol) in methanol (60 mL). After 2 hours the mixture was concentrated *in vacuo,* diluted with water (35 mL) and then the pH was adjusted to 1 with concentrated hydrochloric acid solution. The precipitate was filtered, washed with ice-cold water and dried to give the title compound (2.13 g, 86%) as a white solid.
LRMS (m/z): 197 (M-1)⁺.
¹H NMR δ (300 MHz, DMSO-*d*₆): 4.11 (s, 3H), 8.72 (d, 1H), 8.97 (d, 1H).

### d) (6-Methoxy-5-nitropyridin-3-yl)methanol

Diborane (1M solution in tetrahydrofuran, 26 mL) was added dropwise to a cooled (ice-bath), stirred solution of 6-methoxy-5-nitronicotinic acid (Preparation 9c, 2.00 g, 10.1 mmol) in tetrahydrofuran (30 mL) under an atmosphere of argon. After the addition, the ice-bath was removed and the mixture was stirred and heated to 60 °C. After 4 hours, the mixture was cooled using an ice-bath and further diborane (1M solution in tetrahydrofuran, 10 mL) was added and the mixture was stirred and heated to 60 °C. After a further 2 hours, the mixture was cooled and treated with methanol (30 mL) and then evaporated to dryness. Saturated aqueous ammonium chloride solution (50 mL) was added and the mixture was extracted with ethyl acetate. The organic layer was dried (MgSO₄) and evaporated and the residue was washed with hexanes to give the title compound (1.70 g, 92%) as a white solid.
LRMS (m/z): 185 (M+1)⁺.
¹H NMR δ (300 MHz, CDCl₃): 2.03 (br s, 1H), 4.12 (s, 3H), 4.75 (s, 2H), 8.32 (d, 1H), 8.38 (d, 1H).

### e) 6-Methoxy-5-nitronicotinaldehyde

Dess-martin periodinane (4.4 g, 10.4 mmol) was added to a stirred solution of (6-methoxy-5-nitropyridin-3-yl)methanol (Preparation 9d, 1.70 g, 9.2 mmol) in dichloromethane (75 mL). After 2 hours, the mixture was diluted with diethyl ether (150 mL) and 4% aqueous sodium hydrogencarbonate solution (95 mL). Sodium thiosulphate (18.4 g) was then added and the mixture was vigorously stirred for 10 minutes. The organic layer was separated, dried (MgSO₄) and evaporated to give the title compound (1.58 g, 94%) as a white solid.
LRMS (m/z): 183 (M+1)⁺.
¹H NMR δ (300 MHz, CDCl₃): 4.24 (s, 3H), 8.70 (d, 1H), 8.88 (d, 1H), 10.07 (s, 1H).

### f) 5-(Difluoromethyl)-2-methoxy-3-nitropyridine

*N,N*-Diethylamino sulphur triflouride (2.30 mL, 17.6 mmol) was added dropwise to a cooled (-78 °C), stirred solution of 6-methoxy-5-nitronicotinaldehyde (Preparation 9e, 1.58 g, 8.7 mmol) in dichloromethane (40 mL) under a nitrogen atmosphere. After the addition the mixture was warmed to 0 °C, stirred for 1 hour, then was warmed to ambient temperature and stirred overnight. 4% Aqueous sodium hydrogencarbonate solution (160 mL) was added and the mixture was stirred vigorously for 20 minutes and then extracted with ethyl acetate. The organic extract was washed with brine, dried (MgSO₄) and evaporated to give the title compound (1.77 g, 100%) as an orange oil.
LRMS (m/z): 205 (M+1)⁺.
¹H NMR δ (300 MHz, CDCl₃): 4.17 (s, 3H), 6.74 (t, 1H), 8.42 (d, 1H), 8.54 (d, 1H).

### g) 5-(Difluoromethyl)-2-methoxypyridin-3-amine

10% Palladium on carbon (0.21 g) was added to a solution of 5-(difluoromethyl)-2-methoxy-3-nitropyridine (Preparation 9f, 0.400 g, 2.0 mmol) in methanol (15 mL) and the reaction mixture was stirred at ambient temperature under a hydrogen atmosphere. Futher palladium catalyst (0.21 g) was added after 1 hour and again after 6 hours and the mixture was stirred overnight. The mixture was then filtered through Celite® and the filter cake was washed with ethyl acetate. The combined filtrate and washings were concentrated and the residue was purified by flash chromatography to give the title compound (0.190 g, 56%) as a beige solid.
LRMS (m/z): 175 (M+1)⁺.
¹H NMR δ (300 MHz, CDCl₃): 4.00 (br s, 2H), 4.03 (s, 3H), 6.57 (t, 1H), 7.01 (d, 1H), 7.67 (d, 1H).

### PREPARATION 10

### 2-(5-(Difluoromethyl)-2-methoxypyridin-3-ylamino)-9-(tetrahydro-2H-pyran-4-yl)-7H-purin-8(9H)-one

### a) 2-(5-(Difluoromethyl)-2-methoxypyridin-3-ylamino)-9-(tetrahydro-2H-pyran-4-yl)-7-((2-(trimethylsilyl)ethoxy)methyl)-7H-purin-8(9H)-one

Obtained as a pale yellow solid (47%) from 2-chloro-9-(tetrahydro-2*H*-pyran-4-yl)-7-{[2-(trimethylsilyl)ethoxy]methyl}-7,9-dihydro-8*H*-purin-8-one (Preparation 1 d) and 5-(difluoromethyl)-2-methoxypyridin-3-amine (Preparation 9g) following the experimental procedure as described in Preparation 2a.
LRMS (m/z): 523 (M+1)⁺.
¹H NMR δ (300 MHz, CDCl₃): 0.01 (s, 9H), 0.95 (m, 2H), 1.76 (d, 2H), 2.80 (m, 2H), 3.59 (m, 4H), 4.29 (m, 5H), 4.59 (m, 1H), 5.29 (s, 2H), 6.74 (t, 1H), 7.65 (s, 1H), 7.91 (s, 1H), 8.13 (s, 1H), 9.00 (s, 1H).

### b) 2-(5-(Difluoromethyl)-2-methoxypyridin-3-ylamino)-9-(tetrahydro-2H-pyran-4-yl)-7H-purin-8(9H)-one

Obtained as a beige solid (73%) from 2-(5-(difluoromethyl)-2-methoxypyridin-3-ylamino)-9-(tetrahydro-2*H*-pyran-4-yl)-7-((2-(trimethylsilyl)ethoxy)methyl)-7*H*-purin-8(9*H*)-one (Preparation 10a) following the experimental procedure as described in Preparation 2b.
LRMS (m/z): 393 (M+1)⁺.
¹H NMR δ (300 MHz, DMSO-d₆): 1.73 (d, 2H), 2.60 (m, 2H), 3.64 (m, 2H), 4.01-4.06 (m, 5H), 4.45 (m, 1H), 7.12 (t, 1H), 8.06 (br s, 2H), 8.16 (s, 1H), 8.89 (s, 1 H).

### PREPARATION 11

### (R)-1-(5-Fluoropyridin-2-yl)ethanamine hydrochloride

### a) N-(1-(5-Fluoropyridin-2-yl)vinyl)acetamide

A solution of 5-fluoropicolinonitrile (9.30 g, 76.2 mmol) in tetrahydrofuran (40 mL) was added dropwise to a cold (0 °C), stirred solution of methylmagnesium bromide (3M solution in diethylether, 30.47 mL, 91.4 mmol) in tetrahydrofuran (40 mL). The mixture was stirred for 30 minutes at 0 °C then diluted with dichloromethane (30 mL) and then acetic anhydride (8.64 mL, 91.4 mmol) in dichloromethane (2 mL) was added dropwise at 0 °C. The mixture was warmed to ambient temperature and stirred overnight. 4% Aqueous sodium hydrogencarbonate solution was added and the mixture was extracted with ethyl acetate. The organic layer was separated, dried (MgSO₄) evaporated and the residue was purified by flash chromatography (3:1 hexanes/ethyl acetate) to give the title compound (4.30 g, 31 %) as a yellow solid.
LRMS (m/z): 181 (M+1)⁺.
¹H NMR δ (300 MHz, CDCl₃): 2.21 (s, 3H), 5.47 (s, 1H), 6.47 (s, 1H), 7.48 (m, 1H), 7.78 (m, 1H), 8.37 (m, 1H), 9.07 (br s, 1H).

### b) (R)-N-(1-(5-Fluoropyridin-2-yl)ethyl)acetamide

A solution of *N*-(1-(5-fluoropyridin-2-yl)vinyl)acetamide (Preparation 11a, 2.00 g, 11.1 mmol) and 1,2-bis[(2*R*, 5*R*)-2,5-diethylphospholano]benzene(1,5-cyclooctadiene) rhodium (I) trifluoromethanesulphonate (0.08 g, 0.11 mmol) in methanol (15 mL) was hydrogenated at 130 psi for 4 hours. The mixture was then concentrated *in vacuo* and the residue was purified by flash chromatography (3:1 to 1:1 hexanes/ethyl acetate) to give the title compound (1.91 g, 92%) as a pale yellow oil. The enantiomeric excess of the product was determined to be 96% (Chiralpak IA, 4:1 heptane/isopropyl alcohol).
LRMS (m/z): 183 (M+1)⁺.
¹H NMR δ (300 MHz, DMSO-d₆): 1.35 (d, 3H), 1.82 (s, 3H), 4.98 (m, 1H), 7.40 (m, 1 H), 7.72 (m, 1H), 8.38 (d, 1H), 8.45 (d, 1H).

### c) (R)-Tert-butyl 1-(5-fluoropyridin-2-yl)ethylcarbamate

A solution of *(R)*-*N*-(1-(5-fluoropyridin-2-yl)ethyl)acetamide (Preparation 11b, 1.85 g, 10.15 mmol), *N*,*N*-dimethylpyridin-4-amine (0.24 g, 1.96 mmol) and di-*tert*-butyl dicarbonate (4.46 g, 20.44 mmol) in tetrahydrofuran (15 mL) was stirred and heated to 50 °C. After 20 hours, the mixture was cooled and a solution of lithium hydroxide monohydrate (0.89 g, 21.21 mmol) in water (18 mL) was added and stirring was continued for 5 hours at ambient temperature. Diethyl ether (100 mL) was then added and the organic layer was separated, washed with brine, dried (MgSO₄) and concentrated. The resulting residue was purified by flash chromatography (4:1 hexanes/ethyl acetate) to give the title compound (1.65 g, 68%) as a yellow solid.
LRMS (m/z): 241 (M+1)⁺.
¹H NMR δ (300 MHz, DMSO-d₆): 1.35 (d, 3H), 1.40 (s, 9H), 4.87 (m, 1H), 7.41 (m, 2H), 7.78 (m, 1H), 8.57 (d, 1H).

### d) (R)-1-(5-Fluoropyridin-2-yl)ethanamine hydrochloride

A 4M solution of hydrogen chloride in dioxane (13 mL) was added to a solution of (R)-*tert*-butyl 1-(5-fluoropyridin-2-yl)ethylcarbamate (Preparation 11c, 1.65 g, 6.87 mmol) in dichloromethane (12 mL). After stirring at ambient temperature for 2.5 hours the mixture was concentrated *in vacuo* to give the title compound (1.30 g, 100%) as a white solid.
LRMS (m/z): 141 (M+1)⁺.
¹H NMR δ (300 MHz, DMSO-d₆): 1.45 (d, 3H), 4.62 (m, 1H), 7.62 (m, 1H), 7.92 (m, 1H), 8.60 (br s, 4H).

### PREPARATION 12

### (R)-2-Chloro-9-(1-(5-fluoropyridin-2-yl)ethyl)-7-((2-(trimethylsilyl)ethoxy)methyl)-7H-purin-8(9H)-one

### a) (R)-2-Chloro-N-(1-(5-fluoropyridin-2-yl)ethyl)-5-nitropyrimidin-4-amine

Obtained as a pale yellow solid (56%) from 2,4-dichloro-5-nitropyrimidine and (*R*)-1-(5-fluoropyridin-2-yl)ethanamine (Preparation 11d) following the experimental procedure as described in Preparation 1 a followed by purification of the crude product by flash chromatography (3:1 hexanes/ethyl acetate).
LRMS (m/z): 298 (M+1)⁺.
¹H NMR δ (300 MHz, CDCl₃): 1.62 (d, 3H), 4.12 (m, 1H), 7.33 (dd, 1H), 7.45 (m, 1H), 8.49 (s, 1H), 9.05 (s, 1 H), 9.65 (br s, 1H).

### b) (R)-2-Chloro-N⁴-(1-(5-fluoropyridin-2-yl)ethyl)pyrimidine-4,5-diamine

Obtained as a yellow solid in quantitative yield from (R)-2-chloro-*N*-(1-(5-fluoropyridin-2-yl)ethyl)-5-nitropyrimidin-4-amine (Preparation 12a) following the experimental procedure as described in Preparation 1 b.
LRMS (m/z): 268 (M+1)⁺.
¹H NMR δ (300 MHz, DMSO-*d*₆): 1.53 (d, 3H), 5.13 (s, 2H), 5.29 (m, 1H), 7.25 (d, 1H), 7.44-7.45 (m, 2H), 7.72 (td, 1H), 8.56 (d, 1H).

### c) (R)-2-Chloro-9-(1-(5-fluoropyridin-2-yl)ethyl)-7H-purin-8(9H)-one

Obtained as a white solid (63%) from (*R*)-2-chloro-*N⁴*-(1-(5-fluoropyridin-2-yl)ethyl) pyrimidine-4,5-diamine (Preparation 12b) following the experimental procedure as described in Preparation 1c.
LRMS (m/z): 294 (M+1)⁺.
¹H NMR δ (300 MHz, DMSO-d₆): 1.94 (d, 3H), 5.68 (m, 1H), 7.57 (m, 1H), 7.75 (td, 1H), 8.18 (s, 1H), 8.47 (d, 1H).

### d) (R)-2-Chloro-9-(1-(5-fluoropyridin-2-yl)ethyl)-7-((2-(trimethylsilyl)ethoxy) methyl)-7H-purin-8(9H)-one

Obtained as a yellow oil (72%) from (*R*)-2-chloro-9-(1-(5-fluoropyridin-2-yl)ethyl)-7*H-*purin-8(9*H*)-one (Preparation 12c) and (2-(chloromethoxy)ethyl)trimethylsilane following the experimental procedure as described in Preparation 1d.
LRMS (m/z): 424 (M+1)⁺.
¹H NMR δ (300 MHz, CDCl₃): 0.01 (s, 9H), 0.95 (m, 2H), 2.08 (d, 3H), 3.59 (m, 2H), 5.33 (s, 2H), 5.90 (m, 1H), 7.47 (m, 2H), 8.22 (s, 1H), 8.35 (d, 1H).

### PREPARATION 13

### (R)-9-(1-(5-Fluoropyridin-2-yl)ethyl)-2-(2-methoxypyridin-3-ylamino)-7H-purin-8(9H)-one

### a) (R)-9-(1-(5-Fluoropyridin-2-yl)ethyl)-2-(2-methoxypyridin-3-ylamino)-7-((2-(trimethylsilyl)ethoxy)methyl)-7H-purin-8(9H)-one

Obtained as a pale brown solid (76%) from (*R*)-2-chloro-9-(1-(5-fluoropyridin-2-yl)ethyl)-7-((2-(trimethylsilyl)ethoxy)methyl)-7*H*-purin-8(9*H*)-one (Preparation 12d) and 2-methoxypyridin-3-amine following the experimental procedure as described in Preparation 2a.
LRMS (m/z): 512 (M+1)⁺.
¹H NMR δ (300 MHz, CDCl₃): 0.01 (s, 9H), 0.95 (t, 2H), 2.07 (d, 3H), 3.61 (t, 2H), 4.11 (s, 3H), 5.31 (s, 2H), 5.87 (m, 1H), 6.87 (m, 1H), 7.37-7.44 (m, 2H), 7.54 (s, 1H), 7.75 (d, 1H), 8.11 (s, 1H), 8.42 (s, 1H), 8.51 (d, 1H).

### b) (R)-9-(1-(5-Fluoropyridin-2-yl)ethyl)-2-(2-methoxypyridin-3-ylamino)-7H-purin-8(9H)-one

Obtained as a pale brown solid (97%) from (*R*)-9-(1-(5-fluoropyridin-2-yl)ethyl)-2-(2-methoxypyridin-3-ylamino)-7-((2-(trimethylsilyl)ethoxy)methyl)-7*H*-purin-8(9*H*)-one (Preparation 13a) following the experimental procedure as described in Preparation 2b.
LRMS (m/z): 382 (M+1)⁺.
¹H NMR δ (300 MHz, CDCl₃): 2.08 (d, 3H), 4.04 (s, 3H), 5.86 (m, 2H), 6.87 (m, 1H), 7.35-7.52 (m, 3H), 7.74 (m, 1H), 8.03 (m, 1H), 8.13 (s, 1H), 8.42 (m, 1H).

### PREPARATION 14

### 2-((1r,4r)-4-Aminocyclohexyl)acetonitrile

### a) Tert-Butyl (1r,4r)-4-(hydroxymethyl)cyclohexylcarbamate

Di-*tert*-butyl dicarbonate (3.04 g, 13.9 mmol) was added to a stirred solution of ((1r,4r)-4-aminocyclohexyl)methanol (1.50 g, 11.6 mmol) in tetrahydrofuran (20 mL). After stirring overnight at ambient temperature, the mixture was evaporated and partitioned between ethyl acetate and water. The organic layer was separated, washed with water and brine, dried (MgSO₄) and evaporated. The residue was treated with hexanes and the suspension was filtered to give the title compound (2.11 g, 79%) as a white solid.
LRMS (m/z): 228 (M-H)⁺.
¹H NMR δ (300 MHz, DMSO-d₆): 0.84-0.95 (m, 2H), 1.05-1.18 (m, 2H), 1.20-1.29 (m, 2H), 1.40 (s, 9H), 1.71-1.80 (m, 3H), 3.14 (m, 1H), 3.21 (t, 2H), 4.41 (t, 1H), 6.73 (d, 1H).

### b) ((1r,4r)-4-(Tert-Butoxycarbonylamino)cyclohexyl)methyl 4-methylbenzene-sulfonate

A solution of 4-methylbenzene-1-sulfonyl chloride (2.28 g, 11.96 mmol) in dichloromethane was added to a solution of *tert*-butyl (1r,4r)-4-(hydroxymethyl) cyclohexylcarbamate (Preparation 14a, 2.11 g, 9.2 mmol) and triethylamine (1.59 mL, 11.4 mmol) in dichloromethane (50 mL) and the resulting mixture was stirred overnight at ambient temperature. The mixture was washed with 1M aqueous sodium hydroxide solution and the organic layer was dried (MgSO₄), evaporated and the residue was purified by flash chromatography (diethyl ether/hexanes) to give the title compound (2.91 g, 83%) as a white solid.
LRMS (m/z): 382 (M-H)⁺.
¹H NMR δ (300 MHz, CDCl₃): 0.90-1.12 (m, 4H), 1.43 (s, 3H), 1.78 (dd, 2H), 1.99 (d, 2H), 3.34 (m, 1H), 3.46 (t, 1H), 3.81 (d, 2H), 4.37 (m, 1H), 7.34 (d, 2H), 7.77 (d, 2H).

### c) Tert-Butyl (1 r,4r)-4-(cyanomethyl)cyclohexylcarbamate

Sodium cyanide (0.38 g, 7.8 mmol) was added to a solution of ((1r,4r)-4-(*tert-*butoxycarbonylamino)cyclohexyl)methyl 4-methylbenzene-sulfonate (Preparation 14b, 1.00 g, 2.6 mmol) in dimethylsulphoxide (10 mL) and the mixture was stirred and heated to 55 °C. After stirring for 20 hours, the mixture was diluted with ethyl acetate and washed with saturated aqueous potassium carbonate solution, water and brine, dried (MgSO₄) and evaporated. The residue was purified by flash chromatography (100% dichloromethane to 95:5 dichloromethane/methanol) to give the title compound (0.450 g, 72%) as a white solid.
LRMS (m/z): 239 (M+H)⁺.
¹H NMR δ (300 MHz, CDCl₃): 1.06-1.25 (m, 4H), 1.44 (s, 9H), 1.65 (m, 1H), 1.90 (d, 2H), 2.06 (d, 2H), 2.25 (d, 2H), 3.39 (m, 1H), 4.38 (m, 1H).

### d) 2-((1r,4r)-4-Aminocyclohexyl)acetonitrile hydrochloride

A mixture of *tert*-butyl (1r,4r)-4-(cyanomethyl)cyclohexylcarbamate (Preparation 14c,

0.348 g, 1.46 mmol) and 4M hydrogen chloride solution in dioxane (3.65 mL) was stirred overnight at ambient temperature. The mixture was evaporated *in vacuo* and treated with diethyl ether and the resultant suspension was filtered to give the hydrochloride salt of the title compound (0.226 g, 89%) as a white solid.
LRMS (m/z): 139 (M+H)⁺.
¹H NMR δ (300 MHz, DMSO-*d*₆): 1.14 (ddd, 2H), 1.37 (ddd, 2H), 1.60 (m, 1H), 1.83 (d, 2H), 1.99 (d, 2H), 2.50 (d, 2H), 2.94 (m, 1H), 8.08 (br s, 2H).

PREPARATION 15

### 2-((1r,4r)-4-(2-(2-Methoxypyridin-3-ylamino)-8-oxo-7H-purin-9(8H)-yl)cyclohexyl) acetonitrile

### a) 5-Nitro-4-thiocyanatopyrimidine

Potassium thiocyanate (2.10 g, 21.6 mmol) was added in portions over 2 hours to a stirred solution of 2,4-dichloro-5-nitropyrimidine (4.00 g, 20.6 mmol) in glacial acetic acid (25 mL) cooled to 10-15 °C using an ice-water bath. The mixture was then stirred at ambient temperature for 1 hour then diluted with water and the precipitate was filtered, washed with water, ice-cold diethyl ether and dried to give the title compound (2.82 g, 63%) as a white solid.
¹H NMR δ (300 MHz, CDCl₃): 9.40 (s, 1H).

### b) N-(2-Methoxypyridin-3-yl)-5-nitro-4-thiocyanatopyrimidin-2-amine

2-Methoxypyridin-3-amine (0.100 g, 0.8 mmol) was added portionwise to a stirred, cooled (ice-bath) suspension of 5-nitro-4-thiocyanatopyrimidine (Preparation 15a, 0.174 g, 0.8 mmol) in ethanol (3 mL). Triethylamine (0.170 mL, 1.2 mmol) was then added dropwise and the mixture was stirred for 30 minutes at 0 °C. The precipitate was filtered and dried to give the title compound (0.135 g, 49%) as a yellow solid.
LRMS (m/z): 305 (M+H)⁺.
¹H NMR δ (300 MHz, DMSO-*d*₆): 3.95 (s, 3H), 7.03 (br s, 1H), 8.07 (br s, 1H), 9.24 (br s, 1H), 10.62 (s, 1H).

### c) 2-((1r,4r)-4-(2-(2-Methoxypyridin-3-ylamino)-5-nitropyrimidin-4-ylamino) cyclohexyl)acetonitrile

A mixture of *N*-(2-methoxypyridin-3-yl)-5-nitro-4-thiocyanatopyrimidin-2-amine (Preparation 15b, 0.178 g, 0.58 mmol), 2-((1r,4r)-4-aminocyclohexyl)acetonitrile hydrochloride (Preparation 14d, 0.123 g, 0.70 mmol) and *N,N*-diisopropylethylamine (0.62 mL, 3.5 mmol) in tetrahydrofuran (10 mL) was stirred and heated to 50 °C. After stirring overnight, the mixture was partitioned between water and ethyl acetate and the organic extract was washed with brine, dried (MgSO₄) and evaporated. The residue was purified by flash chromatography (0-50% ethyl acetate in hexanes) to give the title compound (0.109 g, 49%) as a white solid.
LRMS (m/z): 384 (M+H)⁺.
¹H NMR δ (300 MHz, CDCl₃): 1.27-1.48 (m, 5H), 1.82 (m, 1H), 2.05 (d, 2H), 2.28 (m, 2H), 2.41 (d, 2H), 4.08 (s, 3H), 6.96 (dd, 1H), 7.91 (d, 1H), 7.96 (m, 1H), 8.43 (m, 1H), 8.66 (d, 1H), 9.08 (s, 1H).

### d) 2-((1r,4r)-4-(5-Amino-2-(2-methoxypyridin-3-ylamino)pyrimidin-4-ylamino) cyclohexyl)acetonitrile

Obtained as an off-white solid (96%) from 2-((1r,4r)-4-(2-(2-methoxypyridin-3-ylamino)-5-nitropyrimidin-4-ylamino)cyclohexyl)acetonitrile (Preparation 15c) following the experimental procedure as described in Preparation 5c.
LRMS (m/z): 354 (M+1)⁺.
¹H NMR δ (300 MHz, CDCl₃): 1.19-1.43 (m, 4H), 1.76 (m, 1H), 2.00 (d, 2H), 2.26 (d, 2H), 2.35 (d, 2H), 2.62 (s, 2H), 3.89-3.99 (m, 1H), 4.03 (s, 3H), 5.06 (d, 1H), 6.87 (dd, 1H), 7.63 (s, 1H), 7.70 (dd, 1H), 8.70 (dd, 1H).

### e) 2-((1r,4r)-4-(2-(2-Methoxypyridin-3-ylamino)-8-oxo-7H-purin-9(8H)-yl) cyclohexyl)acetonitrile

Obtained as an off-white solid (74%) from 2-((1r,4r)-4-(5-amino-2-(2-methoxypyridin-3-ylamino)pyrimidin-4-ylamino)cyclohexyl)acetonitrile (Preparation 15d) following the experimental procedure as described in Preparation 1c.
LRMS (m/z): 380 (M+1)⁺.
¹H NMR δ (300 MHz, DMSO-d₆): 1.23 (m, 6H), 1.83 (m, 2H), 2.04 (m, 2H), 2.42 (m, 1H), 4.01 (s, 3H), 4.08 (m, 1H), 7.05 (m, 1H), 7.83 (m, 1H), 7.88 (m, 1H), 8.03 (m, 1H), 8.58(m, 1H), 11.13 (br s, 1H).

PREPARATION 16

### 2-(2-Methoxypyridin-3-ylamino)-7-methyl-9-(tetrahydro-2H-pyran-4-yl)-7H-purin-8(9H)-one

### a) 2-Chloro-7-methyl-9-(tetrahydro-2H-pyran-4-yl)-7H-purin-8(9H)-one

Obtained as a brown solid (89%) from 2-chloro-9-(tetrahydro-2*H*-pyran-4-yl)-7*H*-purin-8(9*H*)-one (Preparation 1c) and iodomethane following the experimental procedure as described in Preparation 1d.
LRMS (m/z): 269 (M+1)⁺.
¹H NMR δ (300 MHz, CDCl₃): 1.71 (dd, 2H), 2.74 (qd, 2H), 3.45 (s, 3H), 3.52 (dd, 2H), 4.13 (dd, 2H), 4.59 (m, 1H), 8.02 (s, 1H).

### b) 2-(2-Methoxypyridin-3-ylamino)-7-methyl-9-(tetrahydro-2H-pyran-4-yl)-7H-purin-8(9H)-one

Obtained as a yellow solid (78%) from 2-chloro-7-methyl-9-(tetrahydro-2*H*-pyran-4-yl)-7*H*-purin-8(9*H*)-one (Preparation 16a) and 2-methoxypyridin-3-amine following the experimental procedure as described in Preparation 2a.
LRMS (m/z): 357 (M+1)⁺.
¹H NMR δ (300 MHz, CDCl₃): 1.73 (d, 2H), 2.80 (m, 2H), 3.41 (s, 3H), 3.53 (dd, 2H), 4.06 (s, 3H), 4.15 (dd, 2H), 4.56 (m, 1H), 6.95 (m, 1H), 7.54 (s, 1H), 7.75 (br s, 1H), 7.92 (s, 1H), 8.79 (m, 1H).

PREPARATION 17

### 2-((2-Methoxypyridin-3-yl)(methyl)amino)-7-methyl-9-(tetrahydro-2H-pyran-4-yl)-7H-purin-8(9H)-one

Obtained as a yellow solid (62%) from 2-(2-methoxypyridin-3-ylamino)-9-(tetrahydro-2*H*-pyran-4-yl)-7*H*-purin-8(9*H*)-one (Preparation 2b) and iodomethane (2 equivalents) following the experimental procedure as described in Preparation 1d followed by purification of the crude product by flash chromatography (95:5 dichloromethane/methanol).
LRMS (m/z): 371 (M+1)⁺.
¹H NMR δ (300 MHz, CDCl₃): 1.69 (m, 2H), 2.64 (m, 2H), 3.38 (s, 3H), 3.44 (s, 3H), 3.54 (m, 2H), 3.89 (s, 3H), 4.05 (m, 2H), 4.46 (m, 1H), 6.99 (dd, 1H), 7.58(dd, 1H), 7.81 (s, 1H), 8.13 (m, 1H).

PREPARATION 18

### 7-(2-Hydroxyethyl)-2-(2-methoxypyridin-3-ylamino)-9-(tetrahydro-2H-pyran-4-yl)-7H-purin-8(9H)-one

### a) 2-Chloro-9-(tetrahydro-2H-pyran-4-yl)-7-[2-(tetrahydro-2H-pyran-2-yloxy)ethyl]-7,9-dihydro-8H-purin-8-one

Obtained as an orange solid (90%) from 2-chloro-9-(tetrahydro-2*H*-pyran-4-yl)-7,9-dihydro-8*H*-purin-8-one (Preparation 1c) and 2-(2-bromoethoxy)tetrahydro-2*H*-pyran following the experimental procedure as described in Preparation 1d.
LRMS (m/z): 383 (M+1)⁺.
¹H NMR δ (300 MHz, DMSO-*d*₆): 1.25-1.56 (m, 6H), 1.68 (d, 2H), 2.35-2.48 (m, 2H), 3.45 (t, 4H), 3.58-3.68 (m, 1H), 3.78-3.89 (m, 1H), 3.97 (d, 2H), 4.01-4.18 (m, 2H), 4.41-4.58 (m, 2H), 8.40 (s, 1H).

### b) 2-(2-Methoxypyridin-3-ylamino)-7-(2-(tetrahydro-2H-pyran-2-yloxy)ethyl)-9-(tetrahydro-2H-pyran-4-yl)-7H-purin-8(9H)-one

Obtained as a yellow solid (56%) from 2-chloro-9-(tetrahydro-2*H*-pyran-4-yl)-7-[2-(tetrahydro-2*H*-pyran-2-yloxy)ethyl]-7,9-dihydro-8*H*-purin-8-one (Preparation 18a) and 2-methoxypyridin-3-amine following the experimental procedure as described in Preparation 2a.
LRMS (m/z): 471 (M+1)⁺.
¹H NMR δ (300 MHz, CDCl₃): 1.58-1.77 (m, 8H), 2.83 (m, 2H), 3.47-3.60 (m, 4H), 3.69 (m, 2H), 3.92-4.18 (m, 7H), 4.58 (m, 2H), 6.95 (m, 1H), 7.54 (s, 1H), 7.75 (d, 1H), 8.14 (s, 1H), 8.75 (d, 1H).

### c) 7-(2-Hydroxyethyl)-2-(2-methoxypyridin-3-ylamino)-9-(tetrahydro-2H-pyran-4-yl)-7H-purin-8(9H)-one

A mixture of 2-(2-methoxypyridin-3-ylamino)-7-(2-(tetrahydro-2*H*-pyran-2-yloxy)ethyl)-9-(tetrahydro-2*H*-pyran-4-yl)-7*H*-purin-8(9*H*)-one (Preparation 18b, 0.210 g, 0.45 mmol) and 2M aqueous hydrochloric acid solution (3 mL) was stirred at ambient temperature. After 30 minutes, the mixture was washed with diethyl ether and the resultant aqueous solution was taken to pH 8 with 8M aqueous sodium hydroxide solution. The precipitate that formed was filtered and dried to give the title compound (0.159 g, 92%) as a white solid.
LRMS (m/z): 387 (M+1)⁺.
¹H NMR δ (300 MHz, CDCl₃): 1.73 (d, 2H), 2.81 (d, 2H), 3.55 (t, 2H), 3.99 (m, 4H), 4.08-4.17 (m, 5H), 4.56 (m, 1H), 6.93 (m, 1H), 7.75 (d, 1H), 8.06 (s, 1H), 8.73 (d, 1H).

### PREPARATION 19

### 5-Chloro-3-(tetrahydro-2H-pyran-4-yl)-1-{[2-(trimethylsilyl)ethoxy]methyl}-1,3-dihydro-2H imidazo[4,5-b]pyridin-2-one

### a) 6-Chloro-3-nitro-N-(tetrahydro-2H-pyran-4-yl)pyridin-2-amine

Tetrahydro-2*H*-pyran-4-amine (prepared as described in WO200424728(A2), 3.00 g,

21.8 mmol) and triethylamine (5.50 mL, 39.9 mmol) were added to a suspension of 2,6-dichloro-3-nitropyridine (3.50 g, 18.1 mmol) in chloroform (60 mL) and the reaction mixture was stirred at ambient temperature overnight and then at 50 °C for 24 hours. The mixture was then cooled to ambient temperature, washed with water, dried (MgSO₄) and evaporated. The residue was purified by flash chromatography (98:2 dichloromethane/methanol) to give the title compound (4.20 g, 90%) as a yellow solid.
LRMS (m/z): 258 (M+1)⁺.
¹H NMR δ (300 MHz, CDCl₃): 1.60-1.75 (m, 2H), 2.08 (dd, 2H), 3.60 (td, 2H), 4.03 (ddd, 2H), 4.31-4.50 (m, 1H), 6.64 (d, 1H), 8.30 (br s, 1H), 8.37 (d, 1H).

### b) 6-Chloro-N²-(tetrahydro-2H-pyran-4-yl)pyridine-2,3-diamine

Obtained as a brown solid (100%) from 6-chloro-3-nitro-*N*-(tetrahydro-2*H*-pyran-4-yl)pyridin-2-amine (Preparation 19a) following the experimental procedure as described in Preparation 1b.
LRMS (m/z): 228 (M+1)⁺.
¹H NMR δ (300 MHz, DMSO-*d*₆):1.43 (qd, 2H), 1.82-1.94 (m, 2H), 3.38-3.49 (m, 2H), 3.79-4.11 (m, 3H), 4.87 (s, 2H), 5.69 (d, 1H), 6.33 (d, 1H), 6.67 (d, 1H).

### c) 5-Chloro-3-(tetrahydro-2H-pyran-4-yl)-1,3-dihydro-2H-imidazo[4,5-b]pyridin-2-one

Obtained as a pale purple solid (75%) from 6-chloro-*N*²-(tetrahydro-2*H*-pyran-4-yl)pyridine-2,3-diamine (Preparation 19b) following the experimental procedure as described in Preparation 1c.
LRMS (m/z): 254 (M+1)⁺.
¹H NMR δ (300 MHz, CDCl₃): 1.74 (d, 2H), 2.72-2.94 (m, 2H), 3.56 (t, 2H), 4.15 (dd, 2H), 4.54-4.71 (m, 1H), 7.03 (d, 1H), 7.25 (d, 1H), 9.23 (br s, 1H).

### d) 5-Chloro-3-(tetrahydro-2H-pyran-4-yl)-1-{[2-(trimethylsilyl)ethoxy]methyl}-1,3-dihydro-2H-imidazo[4,5-b]pyridin-2-one

Obtained as a yellow solid (89%) from 5-chloro-3-(tetrahydro-2*H*-pyran-4-yl)-1,3-dihydro-2*H*-imidazo[4,5-*b*]pyridin-2-one (Preparation 19c) following the experimental procedure as described in Preparation 1 d.
LRMS (m/z): 384 (M+1)⁺.
¹H NMR δ (300 MHz, CDCl₃): 0.00 (s, 9H), 0.94 (m, 2H), 1.75 (m, 2H), 2.84 (m, 2H), 3.59 (m, 4H), 4.15 (m, 2H), 4.65 (m, 1H), 5.32 (m, 2H), 7.08 (d, 1H), 7.30 (d, 1H).

### PREPARATION 20

### 5-(2-Methoxypyridin-3-ylamino)-3-(tetrahydro-2H-pyran-4-yl)-1H-imidazo[4,5-b] pyridin-2(3H-one

### a) 5-(2-Methoxypyridin-3-ylamino)-3-(tetrahydro-2H-pyran-4-yl)-1-((2-(trimethyl silyl)ethoxy)methyl)-1H-imidazo[4,5-b]pyridin-2(3H)-one

Obtained as a yellow solid (70%) from 5-chloro-3-(tetrahydro-2*H*-pyran-4-yl)-1-{[2-(trimethylsilyl)ethoxy]methyl}-1,3-dihydro-2*H*-imidazo[4,5-b]pyridin-2-one (Preparation 19d) and 2-methoxypyridin-3-amine following the experimental procedure as described in Preparation 2a.
LRMS (m/z): 472 (M+1)⁺.
¹H NMR δ (300 MHz, DMSO-d₆): 0.01 (s, 9H), 0.92 (t, 2H), 1.75 (d, 2H), 2.68 (ddd, 2H), 3.54 (t, 2H), 3.63 (t, 2H), 4.05 (s, 3H), 4.09 (dm, 2H), 4.55 (m, 1H), 5.31 (s, 2H), 6.97 (d, 1H), 7.00 (dd, 1H), 7.58 (d, 1H), 7.77 (dd, 1H), 8.38 (s, 1H), 8.61 (dd, 1H).

### b) 5-(2-Methoxypyridin-3-ylamino)-3-(tetrahydro-2H-pyran-4-yl)-1H-imidazo[4,5-b] pyridin-2(3H)-one

Obtained as an off-white solid (65%) from 5-(2-methoxypyridin-3-ylamino)-3-(tetrahydro-2*H*-pyran-4-yl)-1-((2-(trimethylsilyl)ethoxy)methyl)-1*H*-imidazo[4,5-b]pyridin-2(3*H*)-one (Preparation 20a) following the experimental procedure as described in Preparation 2b.
LRMS (m/z): 342(M+1)⁺.
¹H NMR δ (300 MHz, DMSO-d₆): 1.67 (dd, 2H), 2.63 (ddd, 2H), 3.47 (t, 2H), 3.99 (s, 3H), 4.03 (dd, 2H), 4.41 (m, 1H), 6.84 (d, 1H), 6.93 (dd, 1H), 7.27 (d, 1H), 7.69 (d, 1H), 8.21 (s, 1H), 8.55 (d, 1H), 10.82 (s, 1H).

### PREPARATION 21

### 5-Chloro-6-fluoro-3-(tetrahydro-2H-pyran-4-yl)-1-{[2-(trimethylsilyl)ethoxy] methyl}-1,3-dihydro-2H-imidazo[4,5-b]pyridin-2-one

### a) 6-Chloro-5-fluoro-2-(tetrahydro-2H-pyran-4-ylamino)nicotinic acid

A mixture of 2,6-dichloro-5-fluoronicotinic acid (1.01 g, 4.8 mmol), diisopropylethylamine (11 mL, 62.5 mmol) and tetrahydro-2*H*-pyran-4-amine hydrochloride (prepared as described in WO200424728(A2), 3.30 g, 24 mmol) in acetonitrile (5 mL) was stirred and heated under microwave irradiation at 130 °C for 21 hours. The mixture was then cooled, dichloromethane was added and the organic layer was washed with 5% aqueous citric acid solution, water and brine, dried (MgSO₄) and the solvent was evaporated. The residue was purified by reverse phase chromatography (C-18 silica from Waters©, water/acetonitrile/methanol as eluents [0.1% v/v formic acid buffered] 0% to 100%) to give the title compound (0.51 g, 39%) as a white solid.
LRMS (m/z): 273 (M-1)⁺.
¹H NMR δ (300 MHz, CDCl₃): 1.48-1.69 (m, 2H), 1.97-2.14 (m, 2H), 3.58 (t, 2H), 4.03 (dd, 2H), 4.29 (ddd, 1H), 5.04 (d, 1H), 7.84 (d, 1H).

### b) 5-Chloro-6-fluoro-3-(tetrahydro-2H-pyran-4-yl)-1,3-dihydro-2H-imidazo[4,5-b] pyridin-2-one

Triethylamine (0.20 mL, 1.43 mmol) and diphenylphosphoryl azide (0.19 mL, 0.88 mmol) were added to a solution of 6-chloro-5-fluoro-2-(tetrahydro-2*H*-pyran-4-ylamino)nicotinic acid (Preparation 21 a, 0.200 g, 0.74 mmol) in 1,4-dioxane (5 mL) and the mixture was stirred and heated to 110 °C. After 2 hours, the solvent was evaporated and the residue was partitioned between water and ethyl acetate and the organic layer was washed with 4% aqueous sodium hydrogencarbonate solution and dried (MgSO₄). The solvent was evaporated and the residue was triturated with diethyl ether to give a solid which was filtered and dried to give the title compound (0.092 g, 46%) as a white solid.
LRMS (m/z): 270 (M-1)⁺.
¹H NMR δ (300 MHz, DMSO-d₆): 1.63 (d, 2H), 2.49 (ddd, 2H), 3.55-3.48 (m, 3H), 3.97 (dd, 2H), 4.41 (tt, 1H), 7.57 (d, 1H).

### c) 5-Chloro-6-fluoro-3-(tetrahydro-2H-pyran-4-yl)-1-{[2-(trimethylsilyl)ethoxy] methyl}-1,3-dihydro-2H-imidazo[4,5-b]pyridin-2-one

Obtained as a white solid (70%) from 5-chloro-6-fluoro-3-(tetrahydro-2*H*-pyran-4-yl)-1,3-dihydro-2*H*-imidazo[4,5-*b*]pyridin-2-one (Preparation 21 b) and (2-(chloromethoxy) ethyl)trimethylsilane following the experimental procedure as described in Preparation 1 d.
LRMS (m/z): 402 (M+1)⁺.
¹H NMR δ (300 MHz, CDCl₃): -0.01 (s, 9H), 0.80-1.05 (m, 2H), 1.64-1.77 (m, 2H), 2.78 (m, 2H), 3.47-3.65 (m, 4H), 4.14 (dd, 2H), 4.51-4.67 (m, 1H), 5.28 (s, 2H), 7.27 (s, 1H).

### PREPARATION 22

### 6-Fluoro-5-(2-methoxypyridin-3-ylamino)-3-(tetrahydro-2H-pyran-4-yl)-1H-imidazo [4,5-b]pyridin-2(3H)-one

### a) 6-Fluoro-5-(2-methoxypyridin-3-ylamino)-3-(tetrahydro-2H-pyran-4-yl)-1-((2-(trimethylsilyl)ethoxy)methyl)-1H-imidazo[4,5-b]pyridin-2(3H)-one

Obtained as a yellow solid (80%) from 5-chloro-6-fluoro-3-(tetrahydro-2*H*-pyran-4-yl)-1-{[2-(trimethylsilyl)ethoxy]methyl}-1,3-dihydro-2*H*-imidazo[4,5-*b*]pyridin-2-one (Preparation 21 c) and 2-methoxypyridin-3-amine following the experimental procedure as described in Preparation 2a.
LRMS (m/z): 490 (M+1)⁺.
¹H NMR δ (300 MHz, CDCl₃): 0.01 (s, 9H), 0.96 (m, 2H), 1.77 (m, 2H), 2.82 (br s, 2H), 3.60 (m, 4H), 4.13 (m, 5H), 4.61 (m, 1H), 5.29 (s, 2H), 6.81 (m, 1H), 7.20 (s, 1H), 7.78(d, 1H), 8.73 (d, 1H).

### b) 6-Fluoro-5-(2-methoxypyridin-3-ylamino)-3-(tetrahydro-2H-pyran-4-yl)-1H-imidazo[4,5-b]pyridin-2(3H)-one

Obtained as a white solid (98%) from 6-fluoro-5-(2-methoxypyridin-3-ylamino)-3-(tetrahydro-2*H*-pyran-4-yl)-1-((2-(trimethylsilyl)ethoxy)methyl)-1*H*-imidazo[4,5-b]pyridin-2(3*H*)-one (Preparation 22a) following the experimental procedure as described in Preparation 2b.
LRMS (m/z): 360 (M+1)⁺.
¹H NMR δ (300 MHz, DMSO-*d*₆): 1.68 (dd, 2H), 2.45 (m, 2H), 3.40-4.60 (m, 5H), 7.00-8.00 (4, 4H), 8.42 (s, 1H), 11.20 (s, 1H).

### PREPARATION 23

### 2-Chloro-6-morpholino-9-(tetrahydro-2H-pyran-4-yl)-7-((2-(trimethylsilyl)ethoxy) methyl)-7H-purin-8(9H)-one

### a) 2,4,6-Trichloro-5-nitropyrimidine

5-Nitropyrimidine-2,4,6-triol was dried in a vaccuum oven at 90 °C for 48 hours. Anhydrous material (5.0 g, 29 mmol) was added to phosphorous oxychloride (29.0 mL, 316 mmol) under an argon atmosphere. Then *N,N*-diethylaniline (24.0 mL, 150 mmol) was added dropwise to the stirred reaction mixture at such a rate that the internal reaction temperature did not exceed 45 °C (periodic cooling with an ice-water bath was necessary). The mixture was stirred at ambient temperature for 3 hours then added slowly onto ice-water at such a rate that the temperature did not exceed 5 °C. The mixture was then warmed to ambient temperature and extracted with diethyl ether (3 x 170 mL). The combined organic extract was washed with water, dried (MgSO₄) and concentrated and the resultant oil was extracted with hot hexane. The hexane extract was concentrated to give the title compound (2.20 g, 33%) as a brown solid which was used without further purification.

### b) 4-(2,6-Dichloro-5-nitropyrimidin-4-yl)morpholine

A solution of morpholine (0.362 g, 4.2 mmol) and triethylamine (0.580 mL, 4.2 mmol) in dichloromethane (11 mL) was added dropwise to a cooled (ice-bath), stirred solution of 2,4,6-trichloro-5-nitropyrimidine (Preparation 23a, 0.950 g, 4.2 mmol) in dichloromethane (25 mL). The mixture was warmed to ambient temperature and stirred overnight. After this period the mixture was concentrated and the residue was purified by flash chromatography (3:1 hexanes/ethyl acetate) to give the title compound (0.780 g, 67%) as a yellow solid.
LRMS (m/z): 279 (M+1)⁺.
¹H NMR δ (300 MHz, CDCl₃): 3.62 (m, 4H), 3.77 (m, 4H).

### c) 2-Chloro-6-morpholino-5-nitro-N-(tetrahydro-2H-pyran-4-yl)pyrimidin-4-amine

A solution of tetrahydro-2*H*-pyran-4-amine hydrochloride (prepared as described in WO200424728(A2), 0.280 g, 2.0 mmol) and triethylamine (0.57 mL, 4.1 mmol) in dichloromethane (4 mL) was added dropwise to a cooled (ice-bath), stirred solution of 4-(2,6-dichloro-5-nitropyrimidin-4-yl)morpholine (Preparation 23b, 0.379 g, 1.4 mmol) in dichloromethane (4 mL). The mixture was warmed to ambient temperature and stirred for 30 minutes. After this period the mixture was diluted with saturated aqueous sodium hydrogencarbonate solution and extracted with dichloromethane. The organic extract was dried (MgSO₄) and concentrated and the residue was purified by flash chromatography (4:1 hexanes/ethyl acetate) to give the title compound (0.414 g, 89%) as a yellow solid.
LRMS (m/z): 344(M+1)⁺.
¹H NMR δ (300 MHz, CDCl₃): 1.57-1.63 (m, 2H), 2.00 (m, 2H), 3.56 (m, 6H), 3.77 (m, 4H), 3.99 (m, 2H), 4.34 (m, 1H), 8.40 (br s, 1H).

### d) 2-Chloro-6-morpholino-N⁴-(tetrahydro-2H-pyran-4-yl)pyrimidine-4,5-diamine

Obtained as a beige solid in quantitative yield from 2-chloro-6-morpholino-5-nitro-*N-*(tetrahydro-2*H*-pyran-4-yl)pyrimidin-4-amine (Preparation 23c) following the experimental procedure as described in Preparation 1 b.
LRMS (m/z): 314 (M+1)⁺.
¹H NMR δ (300 MHz, CDCl₃): 1.61 (m, 2H), 2.05 (m, 2H), 3.22 (m, 4H), 3.57 (td, 2H), 3.91 (m, 4H), 4.02 (m, 2H), 4.18-4.30 (m, 1 H).

### e) 2-Chloro-6-morpholino-9-(tetrahydro-2H-pyran-4-yl)-7H-purin-8(9H)-one

Obtained as a white solid (78%) from 2-chloro-6-morpholino-*N⁴*-(tetrahydro-2*H*-pyran-4-yl)pyrimidine-4,5-diamine (Preparation 23d) following the experimental procedure as described in Preparation 1 c.
LRMS (m/z): 340 (M+1)⁺.
¹H NMR δ (300 MHz, DMSO-*d*₆): 1.67 (m, 2H), 2.56 (m, 2H), 3.60 (m, 6H), 3.73 (m, 4H), 4.01 (m, 2H), 4.44 (m, 1H), 11.30 (br s, 1H).

### f) 2-Chloro-6-morpholino-9-(tetrahydro-2H-pyran-4-yl)-7-((2-(trimethylsilyl)ethoxy) methyl)-7H-purin-8(9H)-one

Obtained as a white solid (75%) from 2-chloro-6-morpholino-9-(tetrahydro-2*H*-pyran-4-yl)-7*H*-purin-8(9*H*)-one (Preparation 23e) and (2-(chloromethoxy)ethyl)trimethylsilane following the experimental procedure as described in Preparation 1d.
LRMS (m/z): 470 (M+1)⁺.
¹H NMR δ (300 MHz, CDCl₃): 0.00 (s, 9H), 0.92 (m, 2H), 1.69 (m, 2H), 2.67-2.79 (m, 2H), 3.44 (m, 4H), 3.53 (t, 2H), 3.72-3.78 (m, 2H), 3.82 (m, 4H), 4.13 (m, 2H), 4.56 (m, 1H), 5.28 (s, 2H).

### PREPARATION 24

### 2-(2-Methoxypyridin-3-ylamino)-6-morpholino-9-(tetrahydro-2H-pyran-4-yl)-7H-purin-8(9H)-one

### a) 2-(2-Methoxypyridin-3-ylamino)-6-morpholino-9-(tetrahydro-2H-pyran-4-yl)-7-((2-(trimethylsilyl)ethoxy)methyl)-7H-purin-8(9H)-one

Obtained as a white solid (46%) from 2-chloro-6-morpholino-9-(tetrahydro-2*H*-pyran-4-yl)-7-((2-(trimethylsilyl)ethoxy)methyl)-7*H*-purin-8(9*H*)-one(Preparation 23f) and 2-methoxypyridin-3-amine following the experimental procedure as described in Preparation 2a.
LRMS (m/z): 558 (M+1)⁺.
¹H NMR δ (300 MHz, CDCl₃): 0.01 (s, 9H), 0.93 (m, 2H), 1.68 (d, 2H), 2.81 (m, 2H), 3.41 (m, 4H), 3.54 (m, 2H), 3.74 (m, 2H), 3.84 (m, 4H), 4.09 (s, 3H), 4.13 (m, 2H), 4.52 (m, 1H), 5.28 (s, 2H), 6.93 (m, 1H), 7.44 (s, 1H), 7.75 (d, 1H), 8.70 (d, 1H).

### b) 2-(2-Methoxypyridin-3-ylamino)-6-morpholino-9-(tetrahydro-2H-pyran-4-yl)-7H-purin-8(9H)-one

Obtained as a beige solid (57%) from 2-(2-methoxypyridin-3-ylamino)-6-morpholino-9-(tetrahydro-2*H*-pyran-4-yl)-7-((2-(trimethylsilyl)ethoxy)methyl)-7*H*-purin-8(9*H*)-one (Preparation 24a) following the experimental procedure as described in Preparation 2b.
LRMS (m/z): 428 (M+1)⁺.
¹H NMR δ (300 MHz, DMSO-*d*₆): 1.62 (d, 2H), 2.57 (m, 2H), 3.42 (m, 2H), 3.53 (m, 4H), 3.68 (m, 4H), 3.98 (s, 3H), 4.00 (m, 2H), 4.37 (m, 1H), 6.96 (m, 1H), 7.47 (br s, 1H), 7.71 (d, 1H), 8.51 (d, 1H).

### PREPARATION 25

### 4-Methoxypyrimidin-5-amine

### a) 4-Chloro-6-methoxy-5-nitropyrimidine

Sodium methoxide (2.94 g, 54.4 mmol) was added portionwise to a cooled (ice-bath) stirred suspension of 4,6-dichloro-5-nitropyrimidine (5.00 g, 25.8 mmol) in anhydrous methanol (90 mL). After 4 hours stirring at 0 °C the mixture was filtered and the filtrate was concentrated *in vacuo.* The mixture was suspended in hexane, refiltered and the filtrate was concentrate to give an oil which was purified by by flash chromatography (5:1 hexanes/ethyl acetate) to give the title compound (1.97 g, 40%) as a yellow solid.
LRMS (m/z): 190 (M+1)⁺.
¹H NMR δ (300 MHz, CDCl₃): 4.19 (s, 3H), 8.57 (s, 1H).

### b) 4-Methoxypyrimidin-5-amine

10% Palladium on carbon (1.10 g) was added to a solution of 4-chloro-6-methoxy-5-nitropyrimidine (Preparation 25a, 1.97 g, 10.4 mmol) in ethanol (80 mL) and the reaction mixture was stirred at ambient temperature overnight under a hydrogen atmosphere at a pressure of 2 bars. The mixture was then filtered through Celite® and the filter cake was washed with ethanol. The combined filtrate and washings were concentrated to give the title compound (1.30 g, 100%) as a solid.
LRMS (m/z): 126 (M+1)⁺.
¹H NMR δ (300 MHz, DMSO-*d*₆): 4.11 (s, 3H), 7.91 (s, 1 H), 8.59 (s, 1 H).

### PREPARATION 26

### 2-(4-Methoxypyrimidin-5-ylamino)-9-(tetrahydro-2H-pyran-4-yl)-7H-purin-8(9H)-one

### a) 2-(4-Methoxypyrimidin-5-ylamino)-9-(tetrahydro-2H-pyran-4-yl)-7-((2-(trimethyl silyl)ethoxy)methyl)-7H-purin-8(9H)-one

Obtained as a white solid (68%) from 2-chloro-9-(tetrahydro-2*H*-pyran-4-yl)-7-((2-(trimethylsilyl)ethoxy)methyl)-7*H*-purin-8(9*H*)-one (Preparation 1d) and 4-methoxy pyrimidin-5-amine (Preparation 25b) following the experimental procedure as described in Preparation 2a.
LRMS (m/z): 558 (M+1)⁺.
¹H NMR δ (300 MHz, CDCl₃): 0.01 (s, 9H), 0.97 (m, 2H), 1.77 (d, 2H), 2.83 (dt, 2H), 3.61 (m, 4H), 4.18-4.21 (m, 5H), 4.65 (m, 1H), 5.31 (s, 2H), 7.31 (s, 1H), 8.15 (s, 1H), 8.49 (s, 1H), 9.70 (s, 1H).

### b) 2-(4-Methoxypyrimidin-5-ylamino)-9-(tetrahydro-2H-pyran-4-yl)-7H-purin-8(9H)-one

Obtained as a pale grey solid (64%) from 2-(4-methoxypyrimidin-5-ylamino)-9-(tetrahydro-2*H*-pyran-4-yl)-7-((2-(trimethylsilyl)ethoxy)methyl)-7*H*-purin-8(9*H*)-one (Preparation 26a) following the experimental procedure as described in Preparation 2b.
LRMS (m/z): 344 (M+1)⁺.
¹H NMR δ (300 MHz, DMSO-*d*₆): 1.71 (d, 2H), 2.58 (m, 2H), 3.34-3.68 (m, 4H), 4.03 (s, 3H), 4.45 (m, 1H), 8.02 (s, 1H), 8.20 (s, 1 H), 8.52 (s, 1H), 9.26 (s, 1H).

### PREPARATION 27

### (R)-1-(5-Fluoropyrimidin-2-yl)ethanamine hydrochloride

### a) N-(1-(5-Fluoropyrimidin-2-yl)vinyl)acetamide

Obtained as a yellow solid (23%) from 5-fluoropyrimidine-2-carbonitrile following the experimental procedure as described in Preparation 11a.
LRMS (m/z): 182 (M+1)⁺.
¹H NMR δ (300 MHz, CDCl₃): 2.20 (s, 3H), 6.32 (s, 1H), 6.60 (s, 1H), 8.58 (s, 2H), 8.8 (br s, 1H).

### b) (R)-N-(1-(5-Fluoropyrimidin-2-yl)ethyl)acetamide

Obtained as a yellow solid (80%) from *N*-(1-(5-fluoropyrimidin-2-yl)vinyl)acetamide (Preparation 27a) following the experimental procedure as described in Preparation 11 b. The enantiomeric excess of the product was determined to be 99% (Chiralpak IA, 9:1 heptane/ethanol).
LRMS (m/z): 184 (M+1)⁺.
¹H NMR δ (300 MHz, CDCl₃): 1.45 (d, 3H), 2.10 (s, 3H), 5.32 (m, 1H), 6.68 (br s, 1H), 8.59 (s, 2H).

### c) (R)-Tert-butyl 1-(5-fluoropyrimidin-2-yl)ethylcarbamate

Obtained as a colourless oil (74%) from *(R)-N*-(1-(5-fluoropyrimidin-2-yl)ethyl)acetamide (Preparation 27b) following the experimental procedure as described in Preparation 11c.
LRMS (m/z): 242 (M+1)⁺.
¹H NMR δ (300 MHz, CDCl₃): 1.32-1.49 (m, 12H), 5.02 (m, 1H), 5.60 (br s, 1H), 8.58 (s, 2H).

### d) (R)-1-(5-Fluoropyrimidin-2-yl)ethanamine hydrochloride

Obtained as a white solid (88%) from (*R*)*-*tert-butyl 1-(5-fluoropyrimidin-2-yl) ethylcarbamate (Preparation 27c) following the experimental procedure as described in Preparation 11 d.
LRMS (m/z): 142 (M+1)⁺.
¹H NMR δ (300 MHz, DMSO-d₆): 1.49 (d, 3H), 4.59 (m, 1H), 8.60 (br s, 3H), 9.02 (m, 1H).

### PREPARATION 28

### (R)-2-Chloro-9-(1-(5-fluoropyrimidin-2-yl)ethyl)-7-((2-(trimethylsilyl)ethoxy)methyl) -7H-purin-8(9H)-one

### a) (R)-2-Chloro-N-(1-(5-fluoropyrimidin-2-yl)ethyl)-5-nitropyrimidin-4-amine

Obtained as a pale yellow solid (81%) from 2,4-dichloro-5-nitropyrimidine and (*R*)-1-(5-fluoropyrimidin-2-yl)ethanamine hydrochloride (Preparation 27d) following the experimental procedure as described in Preparation 1 a followed by purification of the crude product by flash chromatography (3:1 hexanes/ethyl acetate).
LRMS (m/z): 299 (M+1)⁺.
¹H NMR δ (300 MHz, CDCl₃): 1.72 (d, 3H), 5.69 (m, 1H), 8.64 (s, 2H), 9.08 (s, 1H), 9.62 (br s, 1H).

### b) (R)-2-Chloro-N⁴-(1-(5-fluoropyrimidin-2-yl)ethyl)pyrimidine-4,5-diamine

Obtained as a white solid (61 %) from (*R*)-2-chloro-*N*-(1-(5-fluoropyrimidin-2-yl)ethyl)-5-nitropyrimidin-4-amine (Preparation 28a) following the experimental procedure as described in Preparation 1b followed by purification of the crude product by flash chromatography (98:2 to 95:5 dichloromethane/methanol).
LRMS (m/z): 269 (M+1)⁺.
¹H NMR δ (300 MHz, CDCl₃): 1.65 (d, 3H), 3.13 (br s, 2H), 5.31 (m, 1H), 6.35 (d, 1H), 7.65 (s, 2H), 8.60 (s, 1H).

### c) (R)-2-Chloro-9-(1-(5-fluoropyrimidin-2-yl)ethyl)-7H-purin-8(9H)-one

Obtained as a white solid (76%) from (*R*)-2-chloro-*N⁴*-(1-(5-fluoropyrimidin-2-yl)ethyl)pyrimidine-4,5-diamine (Preparation 28b) following the experimental procedure as described in Preparation 1 c.
LRMS (m/z): 295 (M+1)⁺.
¹H NMR δ (300 MHz, DMSO-d₆): 2.04 (d, 3H), 5.79 (q, 1H), 7.07 (s, 1H), 8.27 (s, 1H), 8.93 (s,2H).

### d) (R)-2-Chloro-9-(1-(5-fluoropyrimidin-2-yl)ethyl)-7-((2-(trimethylsilyl)ethoxy) methyl)-7H-purin-8(9H)-one

Obtained as a colourless oil (57%) from (R)-2-chloro-9-(1-(5-fluoropyrimidin-2-yl)ethyl)-7H-purin-8(9*H*)-one (Preparation 28c) and (2-(chloromethoxy)ethyl)trimethylsilane following the experimental procedure as described in Preparation 1d followed by purification of the crude product by flash chromatography (2:1 hexanes/ethyl acetate).
LRMS (m/z): 425 (M+1)⁺.
¹H NMR δ (300 MHz, CDCl₃): 0.01 (s, 9H), 0.97 (dd, 2H), 2.12 (d, 3H), 3.63 (dd, 2H), 5.35 (s, 2H), 5.93 (m, 1H), 8.24 (s, 1H), 8.53 (s, 2H).

### PREPARATION 29

### (R)-9-(1-(5-Fluoropyrimidin-2-yl)ethyl)-2-(4-methoxypyrimidin-5-ylamino)-7H-purin-8(9H)-one

### a) (R)-9-(1-(5-Fluoropyrimidin-2-yl)ethyl)-2-(4-methoxypyrimidin-5-ylamino)-7-((2-(trimethylsilyl)ethoxy)methyl)-7H-purin-8(9H)-one

Obtained as a pale yellow solid (50%) from (*R*)-2-chloro-9-(1-(5-fluoropyrimidin-2-yl)ethyl)-7-((2-(trimethylsilyl)ethoxy)methyl)-7*H*-purin-8(9H)-one (Preparation 28d) and 4-methoxypyrimidin-5-amine (Preparation 25b) following the experimental procedure as described in Preparation 2a.
LRMS (m/z): 514 (M+1)⁺.
¹H NMR δ (300 MHz, CDCl₃): 0.01 (s, 9H), 0.96 (dd, 2H), 2.10 (d, 3H), 3.62 (dd, 2H), 4.06 (s, 3H), 5.33 (s, 2H), 5.91 (m, 1H), 7.26 (d, 1H), 8.13 (s, 1H), 8.56 (s, 2H), 9.37 (s, 1H).

### b) (R)-9-(1-(5-Fluoropyrimidin-2-yl)ethyl)-2-(4-methoxypyrimidin-5-ylamino)-7H-purin-8(9H)-one

Obtained as an off-white solid (57%) from (*R*)-9-(1-(5-fluoropyrimidin-2-yl)ethyl)-2-(4-methoxypyrimidin-5-ylamino)-7-((2-(trimethylsilyl)ethoxy)methyl)-7*H*-purin-8(9*H*)-one (Preparation 29a) following the experimental procedure as described in Preparation 2b.

LRMS (m/z): 384 (M+1)⁺.

### PREPARATION 30

### 6-Chloro-4-iodo-3-methoxypyridazine

Butyllithium (2.5M solution in hexanes, 19.90 mL, 49.8 mmol) was added dropwise to a stirred, cooled (-30 °C) solution of 2,2,6,6-tetramethylpiperidine (8.44 mL, 50.0 mmol) in tetrahydrofuran (230 mL). After the addition the mixture was warmed to 0 °C over 1 hour then cooled to -78 °C and a solution of 3-chloro-6-methoxypyridazine (3.27 g, 22.6 mmol) in tetrahydrofuran (35 mL) was added dropwise at such a rate that the internal temperature did not exceed -75 °C. After stirring for an additional 30 minutes, iodine (6.08 g, 23.95 mmol) was added in three portions and stirring was continued at -78 °C for 2 hours. After this period, saturated aqueous sodium thiosulphate solution was added and the mixture was warmed to ambient temperature and then partitioned between water and dichloromethane. The aqueous layer was extracted with further dichloromethane and the combined organic extract was washed with brine, dried (MgSO₄) and evaporated to give a residue which was partially purified by flash chromatography (20:1 to 10:1 hexanes/ethyl acetate) to give the title compound (1.62 g) in ca. 90% purity which was used as is.
LRMS (m/z): 271 (M+1)⁺.
¹H NMR δ (300 MHz, CDCl₃): 4.17 (s, 3H), 7.92 (s, 1H).

### PREPARATION 31

### 2-(3-Methoxypyridazin-4-ylamino)-9-(tetrahydro-2H-pyran-4-yl)-7H-purin-8(9H)-one

### a) 2-Amino-9-(tetrahydro-2H-pyran-4-yl)-7-((2-(trimethylsilyl)ethoxy)methyl)-7H-purin-8(9H)-one

An oven-dried resealable Schlenk tube was charged with 2-chloro-9-(tetrahydro-2*H-*pyran-4-yl)-7-{[2-(trimethylsilyl)ethoxy]methyl}-7,9-dihydro-8*H*-purin-8-one (Preparation 1d, 0.500 g, 1.30 mmol), diphenylmethanimine (0.282 g, 1.56 mmol), cesium carbonate (0.593 g, 1.82 mmol) and dry toluene (5 mL). The Schlenk tube was subjected to three cycles of evacuation-backfilling with argon then palladium(II) acetate (0.012 g, 0.05 mmol) and 2,2'-bis(diphenylphosphino)-1,1'-binaphthyl (0.049 g, 0.08 mmol) were added. After three further cycles of evacuation-backfilling with argon, the Schlenk tube was capped and then stirred and heated to 100 °C. After stirring overnight, the mixture was cooled and then partitioned between ethyl acetate and water. The organic extract was washed with brine, dried (MgSO₄) and evaporated to give an oil which was taken up in methanol (18 mL) and sodium acetate (0.64 g, 7.87 mmol) and hydroxylamine hydrochloride (0.218 g, 3.14 mmol) were then added. After stirring 1 hour at ambient temperature the mixture was concentrated *in vacuo* and then partitioned between ethyl acetate and water. The organic extract was washed with brine, dried (MgSO₄) and evaporated to give a solid which was purified by flash chromatography (dichloromethane to 98:2 dichloromethane/methanol) to give the title compound (0.370 g, 78%) as a pale yellow solid.
LRMS (m/z): 366 (M+1)⁺.
¹H NMR δ (300 MHz, CDCl₃): 0.01 (s, 9H), 0.93 (t, 2H), 1.70 (d, 2H), 2.77 (dq, 2H), 3.49-3.61 (m, 4H), 4.12 (dd, 2H), 4.46-4.56 (m, 1H), 4.82 (br s, 2H), 5.23 (s, 2H), 7.93 (s, 1H).

### b) 2-(6-Chloro-3-methoxypyridazin-4-ylamino)-9-(tetrahydro-2H-pyran-4-yl)-7-((2-(trimethylsilyl)ethoxy)methyl)-7H-purin-8(9H)-one

An oven-dried resealable Schlenk tube was charged with 2-amino-9-(tetrahydro-2*H-*pyran-4-yl)-7-((2-(trimethylsilyl)ethoxy)methyl)-7*H*-purin-8(9*H*)-one (Preparation 31 a, 0.118 g, 0.32 mmol), 6-chloro-4-iodo-3-methoxypyridazine (Preparation 30, 0.088 g, 0.33 mmol), cesium carbonate (0.210 g, 0.64 mmol) and 1,4-dioxane (3 mL). The Schlenk tube was subjected to three cycles of evacuation-backfilling with argon then (tris(dibenzylideneacetone)dipalladium (0) (0.018 g, 0.02 mmol) and 9,9-dimethyl-4,5-bis(diphenylphosphino)xanthene (0.015 g, 0.03 mmol) were added. After three further cycles of evacuation-backfilling with argon, the Schlenk tube was capped and then stirred and heated to 100 °C. After stirring 19 hours the mixture was partitioned between ethyl acetate and water. The organic extract was washed with brine, dried (MgSO₄) and evaporated to give a solid which was purified by flash chromatography (0-50% ethyl acetate in hexanes) to give the title compound (0.078 g, 48%) as a white solid.
LRMS (m/z): 509 (M+1)⁺.
¹H NMR δ (300 MHz, CDCl₃): 0.00 (s, 9H), 0.95 (t, 2H), 1.76 (dd, 2H), 2.78 (dq, 2H), 3.53-3.64 (m, 4H), 4.18 (dd, 2H), 4.26 (s, 3H), 4.53-4.62 (m, 1H), 5.31 (s, 2H), 7.78 (br s, 1H), 8.19 (s, 1H), 8.60 (s, 1H).

### c) 2-(3-Methoxypyridazin-4-ylamino)-9-(tetrahydro-2H-pyran-4-yl)-7-((2-(trimethyl silyl)ethoxy)methyl)-7H-purin-8(9H)-one

10% Palladium on carbon (0.033 g) was added to a solution of 2-(6-chloro-3-methoxypyridazin-4-ylamino)-9-(tetrahydro-2*H*-pyran-4-yl)-7-((2-(trimethylsilyl)ethoxy) methyl)-7H-purin-8(9*H*)-one (Preparation 31 b, 0.078 g, 0.15 mmol) in methanol (3 mL) and the reaction mixture was stirred at ambient temperature overnight under an atmosphere of hydrogen. The mixture was then filtered through Celite® and the filter cake was washed with methanol. The combined filtrate and washings were concentrated to give the title compound (0.067 g, 92%) as an off-white solid.
LRMS (m/z): 474 (M+1)⁺.
¹H NMR δ (300 MHz, CDCl₃): 0.00 (s, 9H), 0.95 (t, 2H), 1.76 (dd, 2H), 2.68-2.84 (m, 2H), 3.50-3.65 (m, 4H), 4.17 (dd, 2H), 4.35 (s, 3H), 4.54-4.66 (m, 1H), 5.33 (s, 2H), 8.24 (d, 1H), 8.35 (d, 1H), 8.88 (br s, 1H), 9.09 (br s, 1H).

### d) 2-(3-Methoxypyridazin-4-ylamino)-9-(tetrahydro-2H-pyran-4-yl)-7H-purin-8(9H)-one

Obtained as an off-white solid (80%) from 2-(3-methoxypyridazin-4-ylamino)-9-(tetrahydro-2*H*-pyran-4-yl)-7-((2-(trimethylsilyl)ethoxy)methyl)-7*H*-purin-8(9*H*)-one (Preparation 31 c) following the experimental procedure as described in Preparation 2b.
LRMS (m/z): 344 (M+1)⁺.

### PREPARATION 32

### 2-(6-Chloro-3-oxo-2,3-dihydropyridazin-4-ylamino)-9-(tetrahydro-2H-pyran-4-yl)-7-((2-(trimethylsilyl)ethoxy)methyl)-7H-purin-8(9H)-one

An oven-dried resealable Schlenk tube was charged with 2-amino-9-(tetrahydro-2*H-*pyran-4-yl)-7-((2-(trimethylsilyl)ethoxy)methyl)-7*H*-purin-8(9H)-one (Preparation 31 a, 0.250 g, 0.68 mmol), 4-bromo-6-chloropyridazin-3(2*H*)-one (0.144 g, 0.69 mmol), cesium carbonate (0.446 g, 1.37 mmol) and 1,4-dioxane (5 mL). The Schlenk tube was subjected to three cycles of evacuation-backfilling with argon then (tris (dibenzylideneacetone)dipalladium (0) (0.038 g, 0.04 mmol) and 9,9-dimethyl-4,5-bis(diphenylphosphino)xanthene (0.032 g, 0.06 mmol) were added. After three further cycles of evacuation-backfilling with argon, the Schlenk tube was capped and then stirred and heated to 110 ºC. After stirring overnight the mixture was evaporated *in vacuo* and diluted with water. The resultant precipitate was filtered and washed with several portions of diethyl ether to give the crude title compound (0.250 g, 74%) as an off-white solid which was used in the next step with further purification.
LRMS (m/z): 495 (M+1)⁺.
¹H NMR δ (300 MHz, DMSO-*d*₆): -0.10 (s, 9H), 0.82 (t, 2H), 1.66 (d, 2H), 2.40-2.60 (m, 2H), 3.40-3.57 (m, 4H), 3.98 (d, 2H), 4.38-4.51 (m, 1 H), 5.21 (s, 2H), 7.75 (s, 1 H), 8.32 (s, 1 H), 8.43 (s, 1 H).

### PREPARATION 33

### [2-(1r,4r)-4-(2-chloro-8-oxo-7-{[2-(trimethylsilyl)ethoxy]methyl}-7,8-dihydro-9H-purin-9-yl)cyclohexyl]acetonitrile

### a) {2-(1r,4r)-4-[(2-chloro-5-nitropyrimidin-4-yl)amino]cyclohexyl}acetonitrile

Obtained as an orange solid in quantitative yield from 2,4-dichloro-5-nitropyrimidine and 2-((1r,4r)-4-Aminocyclohexyl)acetonitrile hydrochloride (Preparation 14d) following the experimental procedure as described in Preparation 1a.
¹H NMR δ (250 MHz, DMSO-*d*₆): 1.26 (m, 4H), 1.61 (m, 3H), 1.87 (m, 4H), 2.46 (m, 2H), 4.08 (m, 1 H), 8.59 (d, 1 H), 9.02 (s, 1 H).

### b) {2-(1r,4r)-4-[(5-amino-2-chloropyrimidin-4-yl)amino]cyclohexyl}acetonitrile

Tin (II) chloride dihydrate (8.71 g, 38.64 mmol) was added to a stirred solution of {2-(1r,4r)-4-[(2-chloro-5-nitropyrimidin-4-yl)amino]cyclohexyl}acetonitrile (Preparation 33a, 3.81 g, 12.88 mmol) in ethanol (100 mL) and the resulting mixture was heated to reflux for 2h. After cooling to ambient temperature, solvent was evaporated and the residue was then added slowly onto ice-water. 6N Aqueous sodium hydroxide solution was added until the pH reached approximately 9 and the reaction mixture was extracted with ethyl acetate (2 x 150 mL). The combined organic extracts were dried (MgSO₄) and concentrated *in vacuo.* Purification of the residue by flash chromatography (3-5% methanol in dichloromethane) gave the title compound (2.38 g, 70%) as a red solid.
¹H NMR δ (250 MHz, DMSO-d₆): 0.90-2.06 (m, 9H), 2.47 (m, 2H), 3.78 (br s, 1 H), 4.92 (br s, 2H), 6.62 (d, 1 H), 7.36 (s, 1 H).

### c) [2-(1r,4r)-4-(2-chloro-8-oxo-7,8-dihydro-9H-purin-9-yl)cyclohexyl]acetonitrile

Obtained as a pink solid in quantitative yield from {2-(1r,4r)-4-[(5-amino-2-chloropyrimidin-4-yl)amino]cyclohexyl}acetonitrile (Preparation 33b) following the experimental procedure as described in Preparation 1c.
¹H NMR δ (250 MHz, DMSO-*d*₆): 1.25 (m, 2H), 1.84 (m, 5H), 2.25 (m, 2H), 2.49 (m, 2H), 4.13 (m, 1 H), 6.90 (s, 1 H), 8.13 (s, 1 H).

### d) [2-(1r,4r)-4-(2-chloro-8-oxo-7-{[2-(trimethylsilyl)ethoxy]methyl}-7,8-dihydro-9H-purin-9-yl)cyclohexyl]acetonitrile

Obtained as an orange oil (83%) from [2-(1r,4r)-4-(2-chloro-8-oxo-7,8-dihydro-9*H-*purin-9-yl)cyclohexyl]acetonitrile (Preparation 33c) and (2-(chloromethoxy)ethyl) trimethylsilane following the experimental procedure as described in Preparation 1 d followed by purification of the crude product by flash chromatography (1-3% methanol in dichloromethane).
¹H NMR δ (250 MHz, CDCl₃): 0.03 (s, 9H), 0.91 (m, 2H), 1.34 (m, 1 H), 1.80-2.11 (m, 6H), 2.33 (d, 2H), 2.47 (m, 2H), 3.58 (dd, 2H), 4.35 (m, 1 H), 5.28 (s, 2H), 8.14 (s, 1 H).

### PREPARATION 34

### (2-(1r,4r)-4-{2-[(5-chloro-2-methoxypyridin-3-yl)amino]-8-oxo-7,8-dihydro-9H-purin-9-yl}cyclohexyl)acetonitrile

### a) [2-(1r,4r)-4-(2-[(5-chloro-2-methoxypyridin-3-yl)amino]-8-oxo-7-{[2-(trimethyl silyl)ethoxy]methyl}-7,8-dihydro-9H-purin-9-yl)cyclohexyl]acetonitrile

Obtained as a beige solid (63%) from [2-(1r,4r)-4-(2-chloro-8-oxo-7-{[2-(trimethyl silyl)ethoxy]methyl}-7,8-dihydro-9*H*-purin-9-yl)cyclohexyl]acetonitrile (Preparation 33d) and 5-chloro-2-methoxypyridin-3-amine (Preparation 3b) following the experimental procedure as described in Preparation 2a.
¹H NMR δ (250 MHz, CDCl₃): 0.02 (s, 9H), 0.93 (m, 2H), 1.37 (m, 1 H), 1.87-2.16 (m, 6H), 2.35 (d, 2H), 2.52 (m, 2H), 3.59 (m, 2H), 4.04 (s, 3H), 4.36 (m, 1 H), 5.27 (s, 2H), 7.56 (br s, 1H), 7.69 (d, 1H), 8.10 (s, 1H), 8.77 (d, 1 H).

### b) (2-(1r,4r)-4-{2-[(5-chloro-2-methoxypyridin-3-yl)amino]-8-oxo-7,8-dihydro-9H-purin-9-yl}cyclohexyl)acetonitrile

Obtained as a beige solid (96%) [2-(1r,4r)-4-(2-[(5-chloro-2-methoxypyridin-3-yl)amino]-8-oxo-7-{[2-(trimethylsilyl)ethoxy]methyl}-7,8-dihydro-9*H*-purin-9-yl)cyclohexyl] acetonitrile (Preparation 34a) following the experimental procedure as described in Preparation 2b.

### EXAMPLE 1

### 2-(2-Oxo-1,2-dihydropyridin-3-ylamino)-9-(tetrahydro-2H-pyran-4-yl)-7H-purin-8(9H)-one

Sodium iodide (0.131 g, 0.88 mmol) and trimethylsilyl chloride (0.111 mL, 0.88 mmol) were added to a solution of 2-(2-methoxypyridin-3-ylamino)-9-(tetrahydro-2*H*-pyran-4-yl)-7*H*-purin-8(9*H*)-one (Preparation 2b, 0.100 g, 0.29 mmol) in acetonitrile (2 mL) and the mixture was stirred and heated to 85 °C in a sealed tube. After 2 hours the mixture was concentrated and treated with saturated aqueous sodium thiosulphate solution. After stirring for 30 minutes, the precipitate was filtered, washed with water and diethyl ether and dried to give the title compound (0.080 g, 83%) as a beige solid.
LRMS (m/z): 329 (M+1)⁺.
¹H NMR δ (300 MHz, DMSO-*d*₆): 1.70 (d, 2H), 2.55 (m, 2H), 3.99 (m, 2H), 4.02 (d, 2H), 4.45 (m, 1 H), 6.28 (m, 1 H), 7.00 (s, 1 H), 8.04 (d, 2H), 8.35 (d, 1 H), 11.15 (s, 1 H), 11.94 (s, 1 H).

### EXAMPLE 2

### 2-(5-Chloro-2-oxo-1,2-dihydropyridin-3-ylamino)-9-(tetrahydro-2H-pyran-4-yl)-7H-purin-8(9H)-one

Obtained as a pale brown solid (68%) from 2-(5-chloro-2-methoxypyridin-3-ylamino)-9-(tetrahydro-2*H*-pyran-4-yl)-7*H*-purin-8(9*H*)-one (Preparation 4b) following the experimental procedure as described in Example 1.
LRMS (m/z): 363 (M+1)⁺.
¹H NMR δ (300 MHz, DMSO-*d*₆): 1.74 (d, 2H), 2.58 (m, 2H), 3.49 (m, 2H), 4.03 (d, 2H), 4.46 (m, 1H), 7.21 (d, 1H), 8.07 (s, 1H), 8.13 (s, 1H), 8.40 (s, 1 H), 11.26 (s, 1 H), 12.25 (br s, 1 H).

### EXAMPLE 3

### 2-(5-Fluoro-2-oxo-1,2-dihydropyridin-3-ylamino)-9-(tetrahydro-2H-pyran-4-yl)-7H-purin-8(9H)-one

Obtained as a white solid (48%) from 2-(5-fluoro-2-methoxypyridin-3-ylamino)-9-(tetrahydro-2*H*-pyran-4-yl)-7*H*-purin-8(9*H*)-one (Preparation 6b) following the experimental procedure as described in Example 1.
LRMS (m/z): 347 (M+1)⁺.
¹H NMR δ (300 MHz, DMSO-*d*₆): 1.69 (d, 2H), 2.54 (m, 2H), 3.47 (m, 2H), 4.00 (d, 2H), 4.43 (m, 1 H), 7.11 (s, 1 H), 8.08 (d, 1 H), 8.36 (d, 1 H).

### EXAMPLE 4

### 2-(5-Methyl-2-oxo-1,2-dihydropyridin-3-ylamino)-9-(tetrahydro-2H-pyran-4-yl)-7H-purin-8(9H)-one

Obtained as a white solid (85%) from 2-(2-methoxy-5-methylpyridin-3-ylamino)-9-(tetrahydro-2*H*-pyran-4-yl)-7*H*-purin-8(9*H*)-one (Preparation 8b) following the experimental procedure as described in Example 1.
LRMS (m/z): 343 (M+1)⁺.
¹H NMR δ (300 MHz, DMSO-*d*₆): 1.75 (d, 2H), 2.13 (s, 3H), 2.64 (m, 2H), 3.47 (m, 2H), 4.05 (d, 2H), 4.47 (m, 1 H), 6.84 (s, 1 H), 8.10 (s, 1 H), 8.22 (br s, 1 H), 8.26 (s, 1 H), 11.26 (s, 1 H), 11.78 (br s, 1 H).

### EXAMPLE 5

### 2-(5-(Difluoromethyl)-2-oxo-1,2-dihydropyridin-3-ylamino)-9-(tetrahydro-2H-pyran -4-yl)-7H-purin-8(9H)-one

Obtained as a white solid (91%) from 2-(5-(difluoromethyl)-2-methoxypyridin-3-ylamino)-9-(tetrahydro-2*H*-pyran-4-yl)-7*H*-purin-8(9*H*)-one (Preparation 10b) following the experimental procedure as described in Example 1.
LRMS (m/z): 379 (M+1)⁺.
¹H NMR δ (300 MHz, DMSO-*d*₆): 1.74 (d, 2H), 2.58 (m, 2H), 3.47 (dd, 2H), 4.04 (d, 2H), 4.47 (m, 1 H), 6.88 (t, 1 H), 7.41 (s, 1 H), 8.07 (s, 1 H), 8.13 (s, 1 H), 8.55 (s, 1 H), 11.22 (s, 1 H), 12.25 (br s, 1 H).

### EXAMPLE 6

### (R)-9-(1-(5-Fluoropyridin-2-yl)ethyl)-2-(2-oxo-1,2-dihydropyridin-3-ylamino)-7H-purin-8(9H)-one

Obtained as a white solid (65%) from (*R*)-9-(1-(5-fluoropyridin-2-yl)ethyl)-2-(2-methoxypyridin-3-ylamino)-7*H*-purin-8(9*H*)-one (Preparation 13b) following the experimental procedure as described in Example 1.
LRMS (m/z): 368 (M+1)⁺.
¹H NMR δ (300 MHz, DMSO-*d*₆): 1.94 (d, 3H), 5.70 (m, 1 H), 6.23 (t, 1 H), 6.95 (d, 1 H), 7.57 (dd, 1 H), 7.75 (dd, 1 H), 7.88 (s, 1 H), 7.95 (d, 1 H), 8.05 (s, 1 H), 8.53 (d, 1H).

### EXAMPLE 7

### 2-((1r,4r)-4-(8-Oxo-2-(2-oxo-1,2-dihydropyridin-3-ylamino)-7H-purin-9(8H)-yl) cyclohexyl)acetonitrile

Obtained as a white solid (43%) from 2-((1r,4r)-4-(2-(2-methoxypyridin-3-ylamino)-8-oxo-7*H*-purin-9(8*H*)-yl)cyclohexyl)acetonitrile (Preparation 15e) following the experimental procedure as described in Example 1.
LRMS (m/z): 366 (M+1)⁺.
¹H NMR δ (300 MHz, DMSO-*d*₆): 1.30 (m, 2H), 1.73-2.06 (m, 5H), 2.42 (m, 2H), 3.42 (m, 2H), 4.21 (m, 1 H), 6.35 (m, 1 H), 7.05 (m, 1 H), 8.08 (m, 1 H), 8.38 (m, 1 H), 11.19 (br s, 1 H), 12.01 (br s, 1 H).

### EXAMPLE 8

### 7-Methy)-2-(2-oxo-1,2-dihydropyridin-3-ylamino)-9-(tetrahydro-2H-pyran-4-yl)-7H-purin-8(9H)-one

Obtained as a beige solid (75%) from 2-(2-methoxypyridin-3-ylamino)-7-methyl-9-(tetrahydro-2*H*-pyran-4-yl)-7*H*-purin-8(9*H*)-one (Preparation 16b) following the experimental procedure as described in Example 1.
LRMS (m/z): 343 (M+1)⁺.
¹H NMR δ (300 MHz, DMSO-*d*₆): 1.73 (d, 2H), 2.56 (m, 2H), 3.37 (s, 3H), 3.49 (m, 2H), 4.04 (d, 2H), 4.50 (m, 1 H), 6.32 (m, 1 H), 7.06 (br s, 1 H), 8.24 (s, 1 H), 8.34 (d, 1 H), 12.01 (br s, 1 H).

### EXAMPLE 9

### 7-Methyl-2-(methyl(2-oxo-1,2-dihydropyridin-3-yl)amino)-9-(tetrahydro-2H-pyran-4-yl)-7H-purin-8(9H)-one

Obtained as a white solid (41%) from 2-((2-methoxypyridin-3-yl)(methyl)amino)-7-methyl-9-(tetrahydro-2*H*-pyran-4-yl)-7*H*-purin-8(9*H*)-one (Preparation 17) following the experimental procedure as described in Example 1.
LRMS (m/z): 357 (M+1)⁺.
¹H NMR δ (300 MHz, DMSO-*d*₆): 1.64 (d, 2H), 2.55 (m, 2H), 3.32 (s, 3H), 3.35-3.62 (m, 5H), 3.96 (d, 2H), 4.37 (m, 1 H), 6.25 (m, 1 H), 7.35 (d, 1 H), 7.48 (d, 1H), 8.04 (s, 1 H), 11.80 (s, 1 H).

### EXAMPLE 10

### 7-(2-Hydroxyethyl)-2-(2-oxo-1,2-dihydropyridin-3-ylamino)-9-(tetrahydro-2H-pyran-4-yl)-7H-purin-8(9H)-one

Obtained as a white solid (63%) from 7-(2-hydroxyethyl)-2-(2-methoxypyridin-3-ylamino)-9-(tetrahydro-2*H*-pyran-4-yl)-7*H*-purin-8(9*H*)-one (Preparation 18c) following the experimental procedure as described in Example 1.
LRMS (m/z): 373 (M+1)⁺.
¹H NMR δ (300 MHz, DMSO-*d*₆): 1.68 (d, 2H), 2.50-2.60 (m, 2H), 3.45-3.70 (m, 4H), 3.65 (m, 2H), 3.86 (m, 2H), 3.99 (d, 2H), 4.40-4.53 (m, 1 H), 6.26 (t, 1 H), 6.99 (s, 1H), 8.14 (s, 1H), 8.22 (s, 1H), 8.30 (d, 1 H), 11.94 (br s, 1 H).

### EXAMPLE 11

### 5-(2-Oxo-1,2-dihydropyridin-3-ylamino)-3-(tetrahydro-2H-pyran-4-yl)-1H-imidazo [4,5-b]pyridin-2(3H)-one

Obtained as a white solid (65%) from 5-(2-methoxypyridin-3-ylamino)-3-(tetrahydro-2*H-*pyran-4-yl)-1*H*-imidazo[4,5-b]pyridin-2(3*H*)-one (Preparation 20b) following the experimental procedure as described in Example 1.
LRMS (m/z): 328 (M+1)⁺.
¹H NMR δ (300 MHz, DMSO-*d*₆): 1.69 (d, 2H), 2.61-2.72 (m, 2H), 3.50 (t, 2H), 4.05 (m, 2H), 4.47 (s, 1 H), 6.21 (t, 1 H), 6.92 (s, 1 H), 6.93 (d, 1 H), 7.27 (d, 1 H), 8.37 (m, 2H), 10.83 (s, 1 H), 11.79 (br s, 1 H).

### EXAMPLE 12

### 6-Fluoro-5-(2-oxo-1,2-dihydropyridin-3-ylamino)-3-(tetrahydro-2H-pyran-4-yl)-1H-imidazo[4,5-b]pyridin-2(3H)-one

Obtained as a white solid (72%) from 6-fluoro-5-(2-methoxypyridin-3-ylamino)-3-(tetrahydro-2*H*-pyran-4-yl)-1*H*-imidazo[4,5-b]pyridin-2(3*H*)-one (Preparation 22b) following the experimental procedure as described in Example 1.
LRMS (m/z): 346 (M+1)⁺.
¹H NMR δ (300 MHz, DMSO-*d*₆): 1.67 (d, 2H), 2.50-2.70 (m, 2H), 3.47 (t, 2H), 4.01 (dd, 2H), 4.37-4.51 (m, 1 H), 6.23 (t, 1 H), 6.96 (s, 1 H), 7.48 (d, 1 H), 7.81 (s, 1 H), 8.27 (d, 1 H), 11.03 (br s, 1 H), 11.97 (br s, 1 H).

### EXAMPLE 13

### 6-Morpholino-2-(2-oxo-1,2-dihydropyridin-3-ylamino)-9-(tetrahydro-2H-pyran-4-yl)-7H-purin-8(9H)-one

Obtained as a white solid (52%) from 2-(2-methoxypyridin-3-ylamino)-6-morpholino-9-(tetrahydro-2*H*-pyran-4-yl)-7*H*-purin-8(9*H*)-one (Preparation 24b) following the experimental procedure as described in Example 1.
LRMS (m/z): 414 (M+1)⁺.
¹H NMR δ (300 MHz, DMSO-*d*₆): 1.70 (d, 2H), 2.63 (m, 2H), 3.48 (m, 2H), 3.60 (m, 4H), 3.78 (m, 4H), 4.06 (d, 2H), 4.50 (m, 1 H), 6.33 (t, 1 H), 7.02 (t, 1 H), 7.88 (s, 1 H), 8.29 (dd, 1 H), 10.90 (s, 1 H), 11.96 (br s, 1 H).

### EXAMPLE 14

### 2-(6-Oxo-1,6-dihydropyrimidin-5-ylamino)-9-(tetrahydro-2H-pyran-4-yl)-7H-purin-8(9H)-one

Obtained as a white solid (80%) from 2-(4-methoxypyrimidin-5-ylamino)-9-(tetrahydro-2*H*-pyran-4-yl)-7*H*-purin-8(9*H*)-one (Preparation 26b) following the experimental procedure as described in Example 1.
LRMS (m/z): 330 (M+1)⁺.
¹H NMR δ (300 MHz, DMSO-*d*₆): 1.68 (d, 2H), 2.55 (m, 2H), 3.43 (m, 2H), 3.99 (d, 2H), 4.42 (m, 1 H), 7.74 (s, 1 H), 7.90 (s, 1 H), 8.05 (s, 1 H), 8.86 (s, 1 H).

### EXAMPLE 15

### (R)-9-(1-(5-Fluoropyrimidin-2-yl)ethyl)-2-(6-oxo-1,6-dihydropyrimidin-5-ylamino)-7H-purin-8(9H)-one

Obtained as a white solid (9%) from (*R*)-9-(1-(5-fluoropyrimidin-2-yl)ethyl)-2-(4-methoxypyrimidin-5-ylamino)-7*H*-purin-8(9*H*)-one (Preparation 29b) following the experimental procedure as described in Example 1 followed by purification of the crude product by flash chromatography (98:2 to 95:5 dichloromethane/methanol).
LRMS (m/z): 370 (M+1)⁺.
¹H NMR δ (300 MHz, DMSO-*d*₆): 1.94 (d, 3H), 5.76 (m, 1 H), 7.59 (s, 1 H), 7.85 (s, 1 H), 8.05 (s, 1 H), 8.45 (s, 1 H), 8.87 (s, 2H).

### EXAMPLE 16

### 2-(3-Oxo-2,3-dihydropyridazin-4-ylamino)-9-(tetrahydro-2H-pyran-4-yl)-7H-purin-8(9H)-one

Obtained as a white solid (76%) from 2-(3-methoxypyridazin-4-ylamino)-9-(tetrahydro-2*H*-pyran-4-yl)-7*H*-purin-8(9*H*)-one (Preparation 31 d) following the experimental procedure as described in Example 1.
LRMS (m/z): 330 (M+1)⁺.
¹H NMR δ (300 MHz, DMSO-*d*₆): 1.68 (dd, 2H), 2.45-2.60 (m, 2H), 3.46 (t, 2H), 4.00 (dd, 2H), 4.39-4.49 (m, 1 H), 7.84 (d, 1 H), 8.04 (d, 1 H), 8.11 (s, 1 H), 8.35 (br s, 1 H), 11.30 (br s, 1 H).

### EXAMPLE 17

### 2-(6-Chloro-3-oxo-2,3-dihydropyridazin-4-ylamino)-9-(tetrahydro-2H-pyran-4-yl)-7H-purin-8(9H)-one

Obtained as a white solid (19%) from 2-(6-chloro-3-oxo-2,3-dihydropyridazin-4-ylamino)-9-(tetrahydro-2*H*-pyran-4-yl)-7-((2-(trimethylsilyl)ethoxy)methyl)-7*H*-purin-8(9*H*)-one (Preparation 32) following the experimental procedure as described in Preparation 2b.
LRMS (m/z): 364 (M+1)⁺.
¹H NMR δ (300 MHz, DMSO-*d*₆): 1.69 (dd, 2H), 2.46-2.60 (m, 2H), 3.40-3.49 (m, 2H), 4.00 (dd, 2H), 4.38-4.47 (m, 1 H), 8.09 (s, 1 H), 8.16 (s, 1 H), 8.50 (br s, 1 H).

### EXAMPLE 18

### (2-(1r,4r)-4-{2-[(5-Chloro-2-oxo-1,2-dihydropyridin-3-yl)amino]-8-oxo-7,8-dihydro-9H-purin-9-yl}cyclohexyl)acetonitrile

Obtained as a green solid (18%) from (2-(1r,4r)-4-{2-[(5-chloro-2-methoxypyridin-3-yl)amino]-8-oxo-7,8-dihydro-9*H*-purin-9-yl}cyclohexyl)acetonitrile (Preparation 34b) following the experimental procedure as described in Example 1.
LRMS (m/z): 400 (M+1)⁺.
¹H NMR δ (300 MHz, DMSO-*d*₆, registered at 60 ºC): 1.27 (m, 2H), 1.88 (m, 5H), 2.46 (m, 5H), 4.16 (m, 1 H), 7.08 (s, 1 H), 7.83 (d, 1 H), 8.04 (br s, 1 H), 8.22 (d, 1 H).

### EXAMPLE 19

### (R)-2-(5-fluoro-2-oxo-1,2-dihydropyridin-3-ylamino)-9-(1-(5-fluoropyridin-2-yl)ethyl)-7H-purin-8(9H)-one

### EXAMPLE 20

### (R)-9-(1-(5-fluoropyridin-2-yl)ethyl)-2-(5-methyl-2-oxo-1,2-dihydropyridin-3-ylamino)-7H-purin-8(9H)-one

### EXAMPLE 21

### (R)-2-(5-chloro-2-oxo-1,2-dihydropyridin-3-ylamino)-9-(1-(5-fluoropyridin-2-yl)ethyl)-7H-purin-8(9H)-one

### EXAMPLE 22

### 2-(5-fluoro-2-oxo-1,2-dihydropyridin-3-ylamino)-9-((1r,4r)-4-hydroxycyclohexyl)-7H-purin-8(9H)-one

### EXAMPLE 23

### 9-((1R,4R)-5,7-difluoro-4-hydroxy-1,2,3,4-tetrahydronaphthalen-1-yl)-2-(2-oxo-1,2-dihydropyridin-3-ylamino)-7H-purin-8(9H)-one

### EXAMPLE 24

### 9-((1S,2R)-2-methylcyclohexyl)-2-(2-oxo-1,2-dihydropyridin-3-ylamino)-7H-purin-8(9H)-one

### EXAMPLE 25

### 9-((1r,4r)-4-(hydroxymethyl)cyclohexyl)-2-(2-oxo-1,2-dihydropyridin-3-ylamino)-7H-purin-8(9H)-one

### PHARMACOLOGICAL ACTIVITY

### In vitro JAK kinase Assays

Compounds were screened for their ability to inhibit JAK1, JAK2 and JAK3 using the assays as indicated below.

The catalytic domains of human JAK1 (aa 850-1154), JAK2 (aa 826-1132), JAK3 (aa 795-1124) and Tyk2 (aa 871-1187) were expressed as N-terminal GST-fusion proteins using a baculovirus expression system and were purchased from Carna Biosciences. The enzymatic activity was assayed using as substrate a biotinylated peptide, poly (GT)-Biotin (CisBio). The peptide concentration in the reactions was 60 nM for JAK1, 20 nM for JAK2, 140 nM for JAK3 and 50 nM for Tyk2. The degree of phosphorylation was detected by TR-FRET (time-resolved fluorescence energy transfer).

IC₅₀s of compounds were measured for each kinase in a reaction mixture containing the enzyme, ATP and the peptide in 8 mM MOPS (pH 7.0), 10 mM MgCl₂, 0.05% β-mercaptoethanol, 0.45 mg/ml BSA. The ATP concentration in the reactions was 3 µM for JAK1, 0.2 µM for JAK2, 0.6 µM for JAK3 and 1.8 µM for Tyk2. The enzymatic reactions took place for 30 minutes at room temperature. Then, the reactions were stopped with 20 µL of quench detection buffer (50 mM HEPES, 0.5 M KF, EDTA 0.25 M, 0.1% (w/v) BSA, pH 7.5) containing 0.115 µg/mL of anti-phosphoTyr (PT66)-Cryptate (CisBio) and a variable concentration of SA-XL665 (CisBio) to keep the SA-B ratio constant. Incubate for 3 h and read on Victor 2V spectrofluorometer (PerkinElmer) set to read fluorescence resonance energy transfer.

Some of the acronyms used above have the following meaning:
AA: aminoacids
GST: glutathione-S-transferase
MOPS: 3-(N-morpholino)propane sulfonic acid
BSA: bovine serum albumin
ATP: adenosine tri-phosphate
EDTA: ethylenediaminetetraacetic acid
HEPES: 4-(2-hydroxyethyl)-1-piperazineethanesulfonic acid

Table 1 depicts IC₅₀ values for certain exemplary compounds described in the invention. In Table 1, "A" represents an IC₅₀ value of less than 0.1 µM (100 nM), "B" represents an IC₅₀ value in the range of 0.1 µM (100 nM) to 1 µM (1000 nM), and C represents an IC₅₀ value higher than 1 µM (1000 nM).

**Table 1**

| Example No. | IC₅₀ JAK3 (µM) | IC₅₀ JAK2 (µM) | IC₅₀ JAK1 (µM) |
|---|---|---|---|
| **5** | A | A | A |
| **10** | A | A | B |
| **12** | A | A | B |
| **13** | A | A | C |
| **14** | B | B | B |
| **15** | B | A | C |
| **17** | A | B | B |
| **18** | A | A | A |

It can be seen from Table 1 that the compounds of formula (I) are potent inhibitors of JAK1, JAK2 and JAK3 kinases. Preferred pyridin-2(1 H)-one derivatives of the invention possess an IC₅₀ value for the inhibition of JAK1, JAK2 and JAK3 kinases (determined as defined above) of less than 1 µM (1000 nM), preferably of less than 0.5 µM (500 nM), more preferably of less than 0.2 µM (200 nM), even more preferably of less than 0.1 µM (100 nM) for each Janus Kinase.

The invention is also directed to a compound of the invention as described herein for use in the treatment of the human or animal body by therapy. Compounds of the invention intended for pharmaceutical use may be administered as crystalline or amorphous products, or mixtures thereof. They may be obtained, for example, as solid plugs, powders, or films by methods such as precipitation, crystallization, freeze drying, spray drying, or evaporative drying. Microwave or radio frequency drying may be used for this purpose.

### Combinations

The pyridin-2(1 H)-one derivatives defined herein may also be combined with other active compounds in the treatment of a pathological condition or disease susceptible to amelioration by inhibition of Janus Kinases.

The combinations of the invention can optionally comprise one or more additional active substances which are known to be useful in the treatment of myeloproliferative disorders (such as polycythemia vera, essential thrombocythemia or mielofibrosis), leukemia, lymphoid malignancies and solid tumors; bone marrow and organ transplant rejection; immune-mediated diseases and inflammatory diseases, more in particular wherein the pathological condition or disease is selected from rheumatoid arthritis, multiple sclerosis, inflammatory bowel disease, dry eye, uveitis, allergic conjunctivitis, allergic rhinitis, asthma, chronic obstructive pulmonary disease (COPD), atopic dermatitis and psoriasis, such as (a) Dyhydrofolate reductase inhibitors, such as Methotrexate or CH-1504; (b) Dihydroorotate dehydrogenase (DHODH) inhibitors such as leflunomide, teriflunomide, or the compounds described in the International Patent Application Nos. WO2008/077639 and WO2009/021696; (c) Immunomodulators such as Glatiramer acetate (Copaxone), Laquinimod or Imiquimod; (d) Inhibitors of DNA synthesis and repair, such as Mitoxantrone or Cladribine; (e) Immunosuppressants, such as Imuran (azathioprine) or Purinethol (6-mercaptopurine or 6-MP); (f) Anti-alpha 4 integrin antibodies, such as Natalizumab (Tysabri); (g) Alpha 4 integrin antagonists such as R-1295 , TBC-4746, CDP-323, ELND-002, Firategrast or TMC-2003; (h) Corticoids and glucocorticoids such as prednisone or methylprednisolone, fluticasone, mometasone, budesonide, ciclesonide or beta-metasone; (i) Fumaric acid esters, such as BG-12; (j) Anti-tumor necrosis factor-alpha (Anti-TNF-alpha), such as Infliximab, Adalimumab, or Certolizumab pegol; (k) Soluble Tumor necrosis factor-alpha (TNF-alpha) receptors such as Ethanercept; (I) Anti-CD20 (lymphocyte protein) monoclonal antibodies such as Rituximab, Ocrelizumab Ofatumumab or TRU-015; (m) Anti-CD52 (lymphocyte protein) monoclonal antibodies such as alemtuzumab; (n) Anti-CD25 (lymphocyte protein) such as daclizumab; (o) Anti-CD88 (lymphocyte protein), such as eculizumab or pexilizumab; (p) Anti-Interleukin 6 Receptor (IL-6R), such as tocilizumab; (q) Anti-Interleukin 12 Receptor (IL-12R) / Interleukin 23 Receptor (IL-23R), such as ustekinumab; (r) Calcineurin inhibitors such as cyclosporine A or tacrolimus; (s) Inosine-monophosphate dehydrogenase (IMPDH) inhibitors, such as mycophenolate mophetyl, ribavirin, mizoribine or mycophenolic acid; (t) Cannabinoid receptor agonists such as Sativex; (u) Chemokine CCR1 antagonists such as MLN-3897 or PS-031291; (v) Chemokine CCR2 antagonists such as INCB-8696; (w) Necrosis factor-kappaB (NF-kappaB or NFKB) Activation Inhibitors such as Sulfasalazine, Iguratimod or MLN-0415; (x) Adenosine A_{2A} agonists, such as ATL-313, ATL-146e, CGS-21680, Regadenoson or UK-432,097; (y) Sphingosine-1 (S1 P) phosphate receptor agonists such as fingolimod, BAF-312, or ACT128800; (z) Sphingosine-1 (S1P) liase inhibitors such as LX2931; (aa) Spleen tyrosine kinase (Syk) inhibitors, such as R-112; (bb) Protein Kinase Inhibitors (PKC) inhibitors, such as NVP-AEB071; (cc) Anti-cholinergic agents such as tiotropium or aclidinium; (dd) Beta adrenergic agonists such as formoterol, indacaterol or LAS100977; (ee) Compounds having bifunctional Muscarinic Antagonist-Beta2 Agonist activity (MABAs); (ff) Histamine 1 (H1) receptor antagonists, such as azelastine or ebastine; (gg) Chemoattractant receptor homologous molecule expressed on TH₂ cells (CRTH2) inhibitors, such as OC-459, AZD-1981, ACT-129968, QAV-680; (hh) Vitamin D derivatives like calcipotriol (Daivonex); (ii) Anti-inflammatory agents, such as non-steroidal anti-inflammatory drugs (NSAIDs) or selective cyclooxygenase-2 (COX-2) inhibitors such as aceclofenac, diclofenac, ibuprofen, naproxen, apricoxib, celecoxib, cimicoxib, deracoxib, etoricoxib, lumiracoxib, parecoxib sodium, rofecoxib, selenocoxib-1 or valdecoxib; (jj) Anti-allergic agents; (kk) Anti-viral agents; (II) Phosphodiestearase (PDE) III inhibitors; (mm) Phosphosdiesterase (PDE) IV inhibitors such as roflumilast or GRC-4039; (nn) Dual Phosphodiestearase (PDE) III/IV inhibitors; (oo) Xanthine derivatives, such as theophylline or theobromine; (pp) p38 Mitogen-Activated Protein Kinase (p38 MAPK) Inhibitors such as ARRY-797; (qq) Mitogen-activated extracellular signal regulated kinase kinase (MEK) inhibitor, such as ARRY-142886 or ARRY-438162; (rr) Phosphoinositide 3-Kinases (PI3Ks) inhibitors; (ss) Interferons comprising Interferon beta 1 a such as Avonex from Biogen Idec, CinnoVex from CinnaGen and Rebif from EMD Serono, and Interferon beta 1 b such as Betaferon from Schering and Betaseron from Berlex; and (tt) Interferon alpha such as Sumiferon MP.

Specific examples of suitable corticoids and glucocorticoids that can be combined with the JAK inhibitors of the present invention are prednisolone, methylprednisolone, dexamethasone, dexamethasone cipecilate, naflocort, deflazacort, halopredone acetate, budesonide, beclomethasone dipropionate, hydrocortisone, triamcinolone acetonide, fluocinolone acetonide, fluocinonide, clocortolone pivalate, methylprednisolone aceponate, dexamethasone palmitoate, tipredane, hydrocortisone aceponate, prednicarbate, alclometasone dipropionate, halometasone, methylprednisolone suleptanate, mometasone furoate, rimexolone, prednisolone farnesylate, ciclesonide, butixocort propionate, RPR-106541, deprodone propionate, fluticasone propionate, fluticasone furoate, halobetasol propionate, loteprednol etabonate, betamethasone butyrate propionate, flunisolide, prednisone, dexamethasone sodium phosphate, triamcinolone, betamethasone 17-valerate, betamethasone, betamethasone dipropionate, hydrocortisone acetate, hydrocortisone sodium succinate, prednisolone sodium phosphate and hydrocortisone probutate.

Specific examples of suitable Syk kinase inhibitors that can be combined with the JAK inhibitors of the present invention are fosfamatinib (from Rigel), R-348 (from Rigel), R-343 (from Rigel), R-112 (from Rigel), piceatannol, 2-(2-Aminoethylamino)-4-[3-(trifluoromethyl)phenylamino] pyrimidine-5-carboxamide, R-091 (from Rigel), 6-[5-Fluoro-2-(3,4,5-trimethoxyphenylamino)pyrimidin-4-ylamino]-2,2-dimethyl-3,4-dihydro-2H-pyrido[3,2-b][1,4]oxazin-3-one benzenesulfonate (R-406 from Rigel), 1-(2,4,6-Trihydroxyphenyl)-2-(4-methoxyphenyl)ethan-1-one, N-[4-[6-(Cyclobutylamino)-9H-purin-2-ylamino]phenyl]-N-methylacetamide (QAB-205 from Novartis), 2-[7-(3,4-Dimethoxyphenyl)imidazo[1,2-c]pyrimidin-5-ylamino]pyridine-3-carboxamide dihydrochloride (BAY-61-3606 from Bayer) and AVE-0950 (from Sanofi-Aventis).

Specific examples of suitable M3 antagonists (anticholinergics) that can be combined with the JAK inhibitors of the present invention are tiotropium salts, oxitropium salts, flutropium salts, ipratropium salts, glycopyrronium salts, trospium salts, zamifenacin, revatropate, espatropate, darotropium bromide, CI-923, NPC-14695, BEA-2108, 3-[2-Hydroxy-2,2-bis(2-thienyl)acetoxy]-1-(3-phenoxypropyl)-1-azoniabicyclo[2.2.2]octane salts (in particular aclidinium salts, more preferably aclidinium bromide), 1-(2-Phenylethyl)-3-(9H-xanthen-9-ylcarbonyloxy)-1-azoniabicyclo[2.2.2]octane salts, 2-oxo-1,2,3,4-tetrahydroquinazoline-3-carboxylic acid endo-8-methyl-8-azabicyclo[3.2.1]oct-3-yl ester salts (DAU-5884), 3-(4-Benzylpiperazin-1-yl)-1-cyclobutyl-1-hydroxy-1-phenylpropan-2-one (NPC-14695), N-[1-(6-Aminopyridin-2-ylmethyl)piperidin-4-yl]-2(R)-[3,3-difluoro-1(R)-cyclopentyl]-2-hydroxy-2-phenylacetamide (J-104135), 2(R)-Cyclopentyl-2-hydroxy-N-[1-[4(S)-methylhexyl]piperidin-4-yl]-2-phenylacetamide (J-106366), 2(R)-Cyclopentyl-2-hydroxy-N-[1-(4-methyl-3-pentenyl)-4-piperidinyl]-2-phenylacetamide (J-104129), 1-[4-(2-Aminoethyl)piperidin-1-yl]-2(R)-[3,3-difluorocyclopent-1(R)-yl]-2-hydroxy-2-phenylethan-1-one (Banyu-280634), N-[N-[2-[N-[1-(Cyclohexylmethyl)piperidin-3(R)-ylmethyl]carbamoyl]ethyl]carbamoylmethyl]-3,3,3-triphenylpropionamide (Banyu CPTP), 2(R)-Cyclopentyl-2-hydroxy-2-phenylacetic acid 4-(3-azabicyclo[3.1.0]hex-3-yl)-2-butynyl ester (Ranbaxy 364057), 3(R)-[4,4-Bis(4-fluorophenyl)-2-oxoimidazolidin-1-yl]-1-methyl-1-[2-oxo-2-(3-thienyl)ethyl]pyrrolidinium iodide, N-[1-(3-Hydroxybenzyl)-1-methylpiperidinium-3(S)-yl]-N-[N-[4-(isopropoxycarbonyl)phenyl]carbamoyl]-L-tyrosinamide trifluoroacetate, UCB-101333, Merck's OrM3, 7-endo-(2-hydroxy-2,2-diphenylacetoxy)-9,9-dimethyl-3-oxa-9-azoniatricyclo[3.3.1.0(2,4)]nonane salts, 3(R)-[4,4-Bis(4-fluorophenyl)-2-oxoimidazolidin-1-yl]-1-methyl-1-(2-phenylethyl)pyrrolidinium iodide, trans-4-[2-[Hydroxy-2,2-(dithien-2-yl)acetoxy]-1-methyl-1-(2-phenoxyethyl)piperidinium bromide from Novartis (412682), 7-(2,2-diphenylpropionyloxy)-7,9,9-trimethyl-3-oxa-9-azoniatricyclo[3.3.1.0*2,4*]nonane salts, 7-hydroxy-7,9,9-trimethyl-3-oxa-9-azoniatricyclo[3.3.1.0*2,4*]nonane 9-methyl-9H-fluorene-9-carboxylic acid ester salts, all of them optionally in the form of their racemates, their enantiomers, their diastereomers and mixtures thereof, and optionally in the form of their pharmacologically-compatible acid addition salts. Among the salts chlorides, bromides, iodides and methanesulphonates are preferred.

Specific examples of suitable beta adrenergic agonists (β2-agonists) that can be combined with the JAK inhibitors of the present invention are are terbutaline sulphate, eformoterol fumarate, formoterol fumarate, bambuterol, ibuterol, isoprenaline hydrochloride, dopexamine, metaprotenerol, tulobuterol, procaterol hydrochloride, sibenadet hydrochloride, mabuterol hydrochloride, albuterol sulphate, salbutamol sulphate, salmefamol, salmeterol xinafoate, carmoterol hydrochloride, (R)-albuterol hydrochloride, Levalbuterol hydrochloride; Levosalbutamol hydrochloride; (-)-Salbutamol hydrochloride, formoterol, (R,R)-Formoterol tartrate; Arformoterol tartrate, sulfonterol, Bedoradrine sulphate, Indacaterol, Trantinterol hydrochloride, Milveterol hydrochloride, Olodaterol, fenoterol hydrobromide, rimoterol hydrobromide, riproterol hydrochloride, Vilanterol broxaterol, pirbuterol hydrochloride, bitolterol mesylate, clenbuterol hydrochloride, AZD-3199, GSK-159802; GSK-597901, GSK-678007, GSK-961081; 4-[2-[3-(1H-Benzimidazol-1-yl)-1,1-dimethylpropylamino]-1-hydroxyethyl]-2-(4-methoxybenzylamino)phenol, 1-[2H-5-hydroxy-3-oxo-4H-1,4-benzoxazin-8-yl]-2-[3-(4-N,N-dimethylaminophenyl)-2-methyl-2-propylamino]ethanol, 1-[2H-5-hydroxy-3-oxo-4H-1,4-benzoxazin-8-yl]-2-[3-(4-domethoxyphenyl)-2-methyl-2-propylamino]ethanol, 1-[2H-5-hydroxy-3-oxo-4H-1,4-benzoxazin-8-yl]-2-[3-(4-n-butyloxyhenyl)-2-methyl-2-propylamino]ethanol, KUL-1248, HOKU-81, SM-110444, RP-58802B, LAS 100977 and compounds described in PCT patent applications Nos. WO 2007/124898, WO 2006/122788A1, WO 2008/046598, WO 2008095720, WO 2009/068177 and WO 2010/072354.

Specific examples of suitable Phosphosdiesterase IV (PDE IV) inhibitors that can be combined with the JAK inhibitors of the present invention are benafentrine dimaleate, etazolate, denbufylline, rolipram, cipamfylline, zardaverine, arofylline, filaminast, tipelukast, tofimilast, piclamilast, tolafentrine, mesopram, drotaverine hydrochloride, lirimilast, roflumilast, cilomilast, oglemilast, apremilast, tetomilast, filaminast, (R)-(+)-4-[2-(3-Cyclopentyloxy-4-methoxyphenyl)-2-phenylethyl]pyridine (CDP-840), N-(3,5-Dichloro-4-pyridinyl)-2-[1-(4-fluorobenzyl)-5-hydroxy-1H-indol-3-yl]-2-oxoacetamide (GSK-842470), 9-(2-Fluorobenzyl)-N6-methyl-2-(trifluoromethyl)adenine (NCS-613), N-(3,5-Dichloro-4-pyridinyl)-8-methoxyquinoline-5-carboxamide (D-4418), 3-[3-(Cyclopentyloxy)-4-methoxybenzyl]-6-(ethylamino)-8-isopropyl-3H-purine hydrochloride (V-11294A), 6-[3-(N,N-Dimethylcarbamoyl)phenylsulfonyl]-4-(3-methoxyphenylamino)-8-methylquinoline-3-carboxamide hydrochloride (GSK-256066), 4-[6,7-Diethoxy-2,3-bis(hydroxymethyl)naphthalen-1-yl]-1-(2-methoxyethyl)pyridin-2(1H)-one (T-440), (-)-trans-2-[3'-[3-(N-Cyclopropylcarbamoyl)-4-oxo-1,4-dihydro-1,8-naphthyridin-1-yl]-3-fluorobiphenyl-4-yl]cyclopropanecarboxylic acid, MK-0873, CDC-801, UK-500001, BLX-914, 2-carbomethoxy-4-cyano-4-(3-cyclopropylmethoxy-4-difluroromethoxyphenyl)cyclohexan1-one, cis [4-cyano-4-(3-cyclopropylmethoxy-4-difluoromethoxyphenyl)cyclohexan-1-ol, 5(S)-[3-(Cyclopentyloxy)-4-methoxyphenyl]-3(S)-(3-methylbenzyl)piperidin-2-one (IPL-455903), ONO-6126 (Eur Respir J 2003, 22(Suppl. 45): Abst 2557) and the compounds claimed in the PCT patent applications number WO 03/097613, WO 2004/058729, WO 2005/049581, WO 2005/123693, WO 2005/123692, and WO 2010/069504.

Examples of suitable Phosphoinositide 3-Kinases (PI3Ks) inhibitors that can be combined with the JAK inhibitors of the present invention are 2-Methyl-2-[4-[3-methyl-2-oxo-8-(3-quinolinyl)-2,3-dihydro-1H-imidazo[4,5-c]quinolin-1-yl]phenyl]propanenitrile (BEZ-235 from Novartis), CAL-101 (from Calistoga Pharmaceuticals) and N-Ethyl-N'-[3-(3,4,5-trimethoxyphenylamino)pyrido[2,3-b]pyrazin-6-yl]thiourea (AEZS-126 from Aeterna Zentaris).

The compounds of formula (I) and the combinations of the invention may be used in the treatment of myeloproliferative disorders, leukemia, lymphoid malignancies and solid tumors; bone marrow and organ transplant rejection; immune-mediated diseases and inflammatory diseases, wherein the use of a JAK inhibitor is expected to have a beneficial effect, for example rheumatoid arthritis, multiple sclerosis, inflammatory bowel disease (such as ulcerative colitis or Crohn's disease), dry eye, uveitis, allergic conjunctivitis, allergic rhinitis, asthma, chronic obstructive pulmonary disease (COPD), atopic dermatitis and psoriasis.

The active compounds in the combination product may be administered together in the same pharmaceutical composition or in different compositions intended for separate, simultaneous, concomitant or sequential administration by the same or a different route.

It is contemplated that all active agents would be administered at the same time, or very close in time. Alternatively, one or two actives could be administered in the morning and the other (s) later in the day. Or in another scenario, one or two actives could be administered twice daily and the other (s) once daily, either at the same time as one of the twice-a-day dosing occurred, or separately. Preferably at least two, and more preferably all, of the actives would be administered together at the same time. Preferably, at least two, and more preferably all actives would be administered as an admixture.

The invention is also directed to a combination product of the compounds of the invention together with one or more other therapeutic agents for use in the treatment of a pathological condition or disease susceptible to amelioration by inhibiton of Janus Kinases (JAK), in particular wherein the pathological condition or disease is selected from myeloproliferative disorders, leukemia, lymphoid malignancies and solid tumors; bone marrow and organ transplant rejection; immune-mediated diseases and inflammatory diseases, more in particular wherein the pathological condition or disease is selected from rheumatoid arthritis, multiple sclerosis, inflammatory bowel disease, dry eye, uveitis, allergic conjunctivitis, allergic rhinitis, asthma, chronic obstructive pulmonary disease (COPD), atopic dermatitis and psoriasis.

The invention also encompasses the use of a combination of the compounds of the invention together with one or more other therapeutic agents for the manufacture of a formulation or medicament for treating these diseases.

The invention also provides a method of treatment of a pathological condition or disease susceptible to amelioration by inhibiton of Janus Kinases (JAK), in particular wherein the pathological condition or disease is selected from myeloproliferative disorders, leukemia, lymphoid malignancies and solid tumors; bone marrow and organ transplant rejection; immune-mediated diseases and inflammatory diseases, more in particular wherein the pathological condition or disease is selected from rheumatoid arthritis, multiple sclerosis, inflammatory bowel disease, dry eye, uveitis, allergic conjunctivitis, allergic rhinitis, asthma, chronic obstructive pulmonary disease (COPD), atopic dermatitis and psoriasis; comprising administering a therapeutically effective amount of a combination of the compounds of the invention together with one or more other therapeutic agents.

The active compounds in the combinations of the invention may be administered by any suitable route, depending on the nature of the disorder to be treated, e.g. orally (as syrups, tablets, capsules, lozenges, controlled-release preparations, fast-dissolving preparations, etc); topically (as creams, ointments, lotions, nasal sprays or aerosols, etc); by injection (subcutaneous, intradermic, intramuscular, intravenous, etc.) or by inhalation (as a dry powder, a solution, a dispersion, etc).

The active compounds in the combination, i.e. the pyridin-2(1 H)-one derivatives of the invention, and the other optional active compounds may be administered together in the same pharmaceutical composition or in different compositions intended for separate, simultaneous, concomitant or sequential administration by the same or a different route.

One execution of the present invention consists of a kit of parts comprising a pyridin-2(1 H)-one derivative of the invention together with instructions for simultaneous, concurrent, separate or sequential use in combination with another active compound useful in the treatment of myeloproliferative disorders, leukemia, lymphoid malignancies and solid tumors; bone marrow and organ transplant rejection; immune-mediated diseases and inflammatory diseases, more in particular useful in the treatment of rheumatoid arthritis, multiple sclerosis, inflammatory bowel disease, dry eye, uveitis, allergic conjunctivitis, allergic rhinitis, asthma, chronic obstructive pulmonary disease (COPD), atopic dermatitis and psoriasis.

Another execution of the present invention consists of a package comprising a pyridin-2(1 H)-one derivative of the invention and another active compound useful in the treatment of myeloproliferative disorders, leukemia, lymphoid malignancies and solid tumors; bone marrow and organ transplant rejection; immune-mediated diseases and inflammatory diseases, more in particular useful in the treatment of rheumatoid arthritis, multiple sclerosis, inflammatory bowel disease, dry eye, uveitis, allergic conjunctivitis, allergic rhinitis, asthma, chronic obstructive pulmonary disease (COPD), atopic dermatitis and psoriasis.

### Pharmaceutical Compositions

Pharmaceutical compositions according to the present invention comprise the compounds of the invention in association with a pharmaceutically acceptable diluent or carrier.

As used herein, the term pharmaceutical composition refers to a mixture of one or more of the compounds described herein, or physiologically/pharmaceutically acceptable salts, solvates, N-oxides, stereoisomers, deuterated derivatives thereof or prodrugs thereof, with other chemical components, such as physiologically/pharmaceutically acceptable carriers and excipients. The purpose of a pharmaceutical composition is to facilitate administration of a compound to an organism.

As used herein, a physiologically/pharmaceutically acceptable diluent or carrier refers to a carrier or diluent that does not cause significant irritation to an organism and does not abrogate the biological activity and properties of the administered compound.

The invention further provides pharmaceutical compositions comprising the compounds of the invention in association with a pharmaceutically acceptable diluent or carrier together with one or more other therapeutic agents for use in the treatment of a pathological condition or disease susceptible to amelioration by inhibiton of Janus Kinases (JAK), such as the ones previously described.

The invention is also directed to pharmaceutical compositions of the invention for use in the treatment of a pathological condition or disease susceptible to amelioration by inhibiton of Janus Kinases (JAK), in particular wherein the pathological condition or disease is selected from myeloproliferative disorders, leukemia, lymphoid malignancies and solid tumors; bone marrow and organ transplant rejection; immune-mediated diseases and inflammatory diseases, more in particular wherein the pathological condition or disease is selected from rheumatoid arthritis, multiple sclerosis, inflammatory bowel disease, dry eye, uveitis, allergic conjunctivitis, allergic rhinitis, asthma, chronic obstructive pulmonary disease (COPD), atopic dermatitis and psoriasis. The invention also encompasses the use of a pharmaceutical composition of the invention for the manufacture of a medicament for treating these diseases.

The invention also provides a method of treatment of a pathological condition or disease susceptible to amelioration by inhibiton of Janus Kinases (JAK), in particular wherein the pathological condition or disease is selected from myeloproliferative disorders, leukemia, lymphoid malignancies and solid tumors; bone marrow and organ transplant rejection; immune-mediated diseases and inflammatory diseases, more in particular wherein the pathological condition or disease is selected from rheumatoid arthritis, multiple sclerosis, inflammatory bowel disease, dry eye, uveitis, allergic conjunctivitis, allergic rhinitis, asthma, chronic obstructive pulmonary disease (COPD), atopic dermatitis and psoriasis, comprising administering a therapeutically effective amount of a pharmaceutical composition of the invention.

The present invention also provides pharmaceutical compositions which comprise, as an active ingredient, at least a compound of formula (I) or a pharmaceutically acceptable salt thereof in association with a pharmaceutically acceptable excipient such as a carrier or diluent. The active ingredient may comprise 0.001% to 99% by weight, preferably 0.01% to 90% by weight, of the composition depending upon the nature of the formulation and whether further dilution is to be made prior to application. Preferably the compositions are made up in a form suitable for oral, inhalation, topical, nasal, rectal, percutaneous or injectable administration.

Pharmaceutical compositions suitable for the delivery of compounds of the invention and methods for their preparation will be readily apparent to those skilled in the art. Such compositions and methods for their preparation can be found, for example, in Remington: The Science and Practice of Pharmacy, 21 st Edition, Lippincott Williams & Wilkins, Philadelphia, Pa., 2001.

The pharmaceutically acceptable excipients which are admixed with the active compound or salts of such compound, to form the compositions of this invention are well-known per se and the actual excipients used depend inter alia on the intended method of administering the compositions. Examples, without limitation, of excipients include calcium carbonate, calcium phosphate, various sugars and types of starch, cellulose derivatives, gelatin, vegetable oils and polyethylene glycols.

Additional suitable carriers for formulations of the compounds of the present invention can be found in Remington: The Science and Practice of Pharmacy, 21 st Edition, Lippincott Williams & Wilkins, Philadelphia, Pa., 2001.

### i) Oral Administration

The compounds of the invention may be administered orally (peroral administration; *per* os (latin)). Oral administration involve swallowing, so that the compound is absorbed from the gut and delivered to the liver via the portal circulation (hepatic first pass metabolism) and finally enters the gastrointestinal (GI) tract.

Compositions for oral administration may take the form of tablets, retard tablets, sublingual tablets, capsules, inhalation aerosols, inhalation solutions, dry powder inhalation, or liquid preparations, such as mixtures, solutions, elixirs, syrups or suspensions, all containing the compound of the invention; such preparations may be made by methods well-known in the art. The active ingredient may also be presented as a bolus, electuary or paste.

Where the composition is in the form of a tablet, any pharmaceutical carrier routinely used for preparing solid formulations may be used. Examples of such carriers include magnesium stearate, talc, gelatine, acacia, stearic acid, starch, lactose and sucrose.

A tablet may be made by compression or moulding, optionally with one or more accessory ingredients. Compressed tablets may be prepared by compressing in a suitable machine the active ingredient in a free-flowing form such as a powder or granules, optionally mixed with a binder, lubricant, inert diluent, lubricating, surface active or dispersing agent.

Moulded tablets may be made by moulding in a suitable machine a mixture of the powdered compound moistened with an inert liquid diluent. The tablets may optionally be coated or scored and may be formulated so as to provide slow or controlled release of the active ingredient therein.

For tablet dosage forms, depending on dose, the drug may make up from 1 wt% to 80 wt% of the dosage form, more typically from 5 wt% to 60 wt% of the dosage form. In addition to the drug, tablets generally contain a disintegrant. Examples of disintegrants include sodium starch glycolate, sodium carboxymethyl cellulose, calcium carboxymethyl cellulose, croscarmellose sodium, crospovidone, polyvinylpyrrolidone, methyl cellulose, microcrystalline cellulose, lower alkyl- substituted hydroxypropyl cellulose, starch, pregelatinized starch and sodium alginate. Generally, the disintegrant will comprise from 1 wt% to 25 wt%, preferably from 5 wt% to 20 wt% of the dosage form.

Binders are generally used to impart cohesive qualities to a tablet formulation. Suitable binders include microcrystalline cellulose, gelatin, sugars, polyethylene glycol, natural and synthetic gums, polyvinylpyrrolidone, pregelatinized starch, hydroxypropyl cellulose and hydroxypropyl methylcellulose. Tablets may also contain diluents, such as lactose (monohydrate, spray-dried monohydrate, anhydrous and the like), mannitol, xylitol, dextrose, sucrose, sorbitol, microcrystalline cellulose, starch and dibasic calcium phosphate dihydrate. Tablets may also optionally include surface active agents, such as sodium lauryl sulfate and polysorbate 80, and glidants such as silicon dioxide and talc. When present, surface active agents are typically in amounts of from 0.2 wt% to 5 wt% of the tablet, and glidants typically from 0.2 wt% to 1 wt% of the tablet.

Tablets also generally contain lubricants such as magnesium stearate, calcium stearate, zinc stearate, sodium stearyl fumarate, and mixtures of magnesium stearate with sodium lauryl sulphate. Lubricants generally are present in amounts from 0.25 wt% to 10 wt%, preferably from 0.5 wt% to 3 wt% of the tablet. Other conventional ingredients include anti-oxidants, colorants, flavoring agents, preservatives and taste-masking agents.

Exemplary tablets contain up to about 80 wt% drug, from about 10 wt% to about 90 wt% binder, from about 0 wt% to about 85 wt% diluent, from about 2 wt% to about 10 wt% disintegrant, and from about 0.25 wt% to about 10 wt% lubricant. Tablet blends may be compressed directly or by roller to form tablets. Tablet blends or portions of blends may alternatively be wet-, dry-, or melt-granulated, melt congealed, or extruded before tabletting. The final formulation may include one or more layers and may be coated or uncoated; or encapsulated.

The formulation of tablets is discussed in detail in "Pharmaceutical Dosage Forms: Tablets, Vol. 1 ", by H. Lieberman and L. Lachman, Marcel Dekker, N.Y., 1980.

Where the composition is in the form of a capsule, any routine encapsulation is suitable, for example using the aforementioned carriers in a hard gelatine capsule. Where the composition is in the form of a soft gelatine capsule any pharmaceutical carrier routinely used for preparing dispersions or suspensions may be considered, for example aqueous gums, celluloses, silicates or oils, and are incorporated in a soft gelatine capsule.

Solid formulations for oral administration may be formulated to be immediate and/or modified release. Modified release formulations include delayed-, sustained-, pulsed-, controlled-, targeted and programmed release.

Suitable modified release formulations are described in U.S. Patent No. 6,106,864. Details of other suitable release technologies such as high energy dispersions and osmotic and coated particles can be found in Verma et al, Pharmaceutical Technology On-line, 25(2), 1-14 (2001). The use of chewing gum to achieve controlled release is described in WO 00/35298. The disclosures of these references are incorporated herein by reference in their entireties.

Liquid formulations include suspensions, solutions, syrups and elixirs. Such formulations may be used as fillers in soft or hard capsules and typically include a carrier, for example, water, ethanol, polyethylene glycol, propylene glycol, methylcellulose, or a suitable oil, and one or more emulsifying agents and/or suspending agents. The solutions may be aqueous solutions of a soluble salt or other derivative of the active compound in association with, for example, sucrose to form a syrup. The suspensions may comprise an insoluble active compound of the invention or a pharmaceutically acceptable salt thereof in association with water, together with a suspending agent or flavouring agent. Liquid formulations may also be prepared by the reconstitution of a solid, for example, from a sachet.

### ii) Oral mucosal administration

The compounds of the invention can also be administered via the oral mucosal. Within the oral mucosal cavity, delivery of drugs is classified into three categories: (a) sublingual delivery, which is systemic delivery of drugs through the mucosal membranes lining the floor of the mouth, (b) buccal delivery, which is drug administration through the mucosal membranes lining the cheeks (buccal mucosa), and (c) local delivery, which is drug delivery into the oral cavity.

Pharmaceutical products to be administered via the oral mucosal can be designed using mucoadhesive, quick dissolve tablets and solid lozenge formulations, which are formulated with one or more mucoadhesive (bioadhesive) polymers (such as hydroxy propyl cellulose, polyvinyl pyrrolidone, sodium carboxymethyl cellulose, hydroxy propyl methyl cellulose, hydroxy ethyl cellulose, polyvinyl alcohol, polyisobutylene or polyisoprene); and oral mucosal permeation enhancers (such as butanol, butyric acid, propranolol, sodium lauryl sulphate and others)

### iii) Inhaled administration

The compounds of the invention can also be administered by inhalation, typically in the form of a dry powder (either alone, as a mixture, for example, in a dry blend with lactose, or as a mixed component particle, for example, mixed with phospholipids, such as phosphatidylcholine) from a dry powder inhaler or as an aerosol spray from a pressurized container, pump, spray, atomizer (preferably an atomizer using electrohydrodynamics to produce a fine mist), or nebulizer, with or without the use of a suitable propellant, such as 1 ,1 ,1 ,2-tetrafluoroethane or 1 ,1 ,1 ,2,3,3,3-heptafluoropropane. For intranasal use, the powder may include a bioadhesive agent, for example, chitosan or cyclodextrin.

Dry powder compositions for topical delivery to the lung by inhalation may, for example, be presented in capsules and cartridges of for example gelatine or blisters of for example laminated aluminium foil, for use in an inhaler or insufflator. Formulations generally contain a powder mix for inhalation of the compound of the invention and a suitable powder base (carrier substance) such as lactose or starch. Use of lactose is preferred. Each capsule or cartridge may generally contain between 0.001-50 mg, more preferably 0.01-5 mg of active ingredient or the equivalent amount of a pharmaceutically acceptable salt thereof. Alternatively, the active ingredient (s) may be presented without excipients.

Packaging of the formulation may be suitable for unit dose or multi-dose delivery. In the case of multi- dose delivery, the formulation can be pre-metered or metered in use. Dry powder inhalers are thus classified into three groups: (a) single dose, (b) multiple unit dose and (c) multi dose devices.

For inhalers of the first type, single doses have been weighed by the manufacturer into small containers, which are mostly hard gelatine capsules. A capsule has to be taken from a separate box or container and inserted into a receptacle area of the inhaler. Next, the capsule has to be opened or perforated with pins or cutting blades in order to allow part of the inspiratory air stream to pass through the capsule for powder entrainment or to discharge the powder from the capsule through these perforations by means of centrifugal force during inhalation. After inhalation, the emptied capsule has to be removed from the inhaler again. Mostly, disassembling of the inhaler is necessary for inserting and removing the capsule, which is an operation that can be difficult and burdensome for some patients.

Other drawbacks related to the use of hard gelatine capsules for inhalation powders are (a) poor protection against moisture uptake from the ambient air, (b) problems with opening or perforation after the capsules have been exposed previously to extreme relative humidity, which causes fragmentation or indenture, and (c) possible inhalation of capsule fragments. Moreover, for a number of capsule inhalers, incomplete expulsion has been reported (e. g. Nielsen et al, 1997).

Some capsule inhalers have a magazine from which individual capsules can be transferred to a receiving chamber, in which perforation and emptying takes place, as described in WO 92/03175. Other capsule inhalers have revolving magazines with capsule chambers that can be brought in line with the air conduit for dose discharge (e. g. WO91/02558 and GB 2242134). They comprise the type of multiple unit dose inhalers together with blister inhalers, which have a limited number of unit doses in supply on a disk or on a strip.

Blister inhalers provide better moisture protection of the medicament than capsule inhalers. Access to the powder is obtained by perforating the cover as well as the blister foil, or by peeling off the cover foil. When a blister strip is used instead of a disk, the number of doses can be increased, but it is inconvenient for the patient to replace an empty strip. Therefore, such devices are often disposable with the incorporated dose system, including the technique used to transport the strip and open the blister pockets.

Multi-dose inhalers do not contain pre-measured quantities of the powder formulation. They consist of a relatively large container and a dose measuring principle that has to be operated by the patient. The container bears multiple doses that are isolated individually from the bulk of powder by volumetric displacement. Various dose measuring principles exist, including rotatable membranes (Ex. EP0069715) or disks (Ex. GB 2041763; EP 0424790; DE 4239402 and EP 0674533), rotatable cylinders (Ex. EP 0166294; GB 2165159 and WO 92/09322) and rotatable frustums (Ex. WO 92/00771), all having cavities which have to be filled with powder from the container. Other multi dose devices have measuring slides (Ex. US 5201308 and WO 97/00703) or measuring plungers with a local or circumferential recess to displace a certain volume of powder from the container to a delivery chamber or an air conduit (Ex. EP 0505321, WO 92/04068 and WO 92/04928), or measuring slides such as the Genuair® (formerly known as Novolizer SD2FL), which is described the following patent applications Nos: WO97/000703, WO03/000325 and WO2006/008027.

Reproducible dose measuring is one of the major concerns for multi dose inhaler devices.

The powder formulation has to exhibit good and stable flow properties, because filling of the dose measuring cups or cavities is mostly under the influence of the force of gravity.

For reloaded single dose and multiple unit dose inhalers, the dose measuring accuracy and reproducibility can be guaranteed by the manufacturer. Multi dose inhalers on the other hand, can contain a much higher number of doses, whereas the number of handlings to prime a dose is generally lower.

Because the inspiratory air stream in multi-dose devices is often straight across the dose measuring cavity, and because the massive and rigid dose measuring systems of multi dose inhalers can not be agitated by this inspiratory air stream, the powder mass is simply entrained from the cavity and little de-agglomeration is obtained during discharge.

Consequently, separate disintegration means are necessary. However in practice, they are not always part of the inhaler design. Because of the high number of doses in multi-dose devices, powder adhesion onto the inner walls of the air conduits and the de-agglomeration means must be minimized and/or regular cleaning of these parts must be possible, without affecting the residual doses in the device. Some multi dose inhalers have disposable drug containers that can be replaced after the prescribed number of doses has been taken (Ex. WO 97/000703). For such semi-permanent multi dose inhalers with disposable drug containers, the requirements to prevent drug accumulation are even more strict.

Apart from applications through dry powder inhalers the compositions of the invention can be administered in aerosols which operate via propellant gases or by means of socalled atomisers, via which solutions of pharmacologically-active substances can be sprayed under high pressure so that a mist of inhalable particles results. The advantage of these atomisers is that the use of propellant gases can be completely dispensed with. Such atomiser is the Respimat® which is described, for example, in PCT Patent Applications Nos. W0 91/14468 and WO 97/12687, reference here is being made to the contents thereof.

Spray compositions for topical delivery to the lung by inhalation may for example be formulated as aqueous solutions or suspensions or as aerosols delivered from pressurised packs, such as a metered dose inhaler, with the use of a suitable liquefied propellant. Aerosol compositions suitable for inhalation can be either a suspension or a solution and generally contain the active ingredient (s) and a suitable propellant such as a fluorocarbon or hydrogen-containing chlorofluorocarbon or mixtures thereof, particularly hydrofluoroalkanes, e. g. dichlorodifluoromethane, trichlorofluoromethane, dichlorotetra-fluoroethane, especially 1,1, 1, 2-tetrafluoroethane, 1,1, 1,2, 3,3, 3-heptafluoro-n-propane or a mixture thereof. Carbon dioxide or other suitable gas may also be used as propellant.

The aerosol composition may be excipient free or may optionally contain additional formulation excipients well known in the art such as surfactants (eg oleic acid or lecithin) and cosolvens (eg ethanol). Pressurised formulations will generally be retained in a canister (eg an aluminium canister) closed with a valve (eg a metering valve) and fitted into an actuator provided with a mouthpiece.

Medicaments for administration by inhalation desirably have a controlled particle size. The optimum particle size for inhalation into the bronchial system is usually 1-10 µm, preferably 2-5 µm. Particles having a size above 20 µm are generally too large when inhaled to reach the small airways. To achieve these particle sizes the particles of the active ingredient as produced may be size reduced by conventional means eg by micronisation. The desired fraction may be separated out by air classification or sieving. Preferably, the particles will be crystalline.

Achieving high dose reproducibility with micronised powders is difficult because of their poor flowability and extreme agglomeration tendency. To improve the efficiency of dry powder compositions, the particles should be large while in the inhaler, but small when discharged into the respiratory tract. Thus, an excipient such as lactose or glucose is generally employed. The particle size of the excipient will usually be much greater than the inhaled medicament within the present invention. When the excipient is lactose it will typically be present as milled lactose, preferably crystalline alpha lactose monohydrate.

Pressurized aerosol compositions will generally be filled into canisters fitted with a valve, especially a metering valve. Canisters may optionally be coated with a plastics material e. g. a fluorocarbon polymer as described in W096/32150. Canisters will be fitted into an actuator adapted for buccal delivery.

### iv) Nasal mucosal administration

The compounds of the invention may also be administered via the nasal mucosal. Typical compositions for nasal mucosa administration are typically applied by a metering, atomizing spray pump and are in the form of a solution or suspension in an inert vehicle such as water optionally in combination with conventional excipients such as buffers, anti-microbials, tonicity modifying agents and viscosity modifying agents.

### v) Parenteral Administration

The compounds of the invention may also be administered directly into the blood stream, into muscle, or into an internal organ. Suitable means for parenteral administration include intravenous, intraarterial, intraperitoneal, intrathecal, intraventricular, intraurethral, intrasternal, intracranial, intramuscular and subcutaneous. Suitable devices for parenteral administration include needle (including microneedle) injectors, needle-free injectors and infusion techniques.

Parenteral formulations are typically aqueous solutions which may contain excipients such as salts, carbohydrates and buffering agents (preferably to a pH of from 3 to 9), but, for some applications, they may be more suitably formulated as a sterile nonaqueous solution or as a dried form to be used in conjunction with a suitable vehicle such as sterile, pyrogen-free water.

The preparation of parenteral formulations under sterile conditions, for example, by lyophilization, may readily be accomplished using standard pharmaceutical techniques well known to those skilled in the art. The solubility of compounds of the invention used in the preparation of parenteral solutions may be increased by the use of appropriate formulation techniques, such as the incorporation of solubility-enhancing agents.

Formulations for parenteral administration may be formulated to be immediate and/or modified release. Modified release formulations include delayed-, sustained-, pulsed-, controlled-, targeted and programmed release. Thus compounds of the invention may be formulated as a solid, semi-solid, or thixotropic liquid for administration as an implanted depot providing modified release of the active compound. Examples of such formulations include drug-coated stents and PGLA microspheres.

### vi) Topical Administration

The compounds of the invention may also be administered topically to the skin or mucosa, that is, dermally or transdermally. Typical formulations for this purpose include gels, hydrogels, lotions, solutions, creams, ointments, dusting powders, dressings, foams, films, skin patches, wafers, implants, sponges, fibers, bandages and microemulsions. Liposomes may also be used. Typical carriers include alcohol, water, mineral oil, liquid petrolatum, white petrolatum, glycerin, polyethylene glycol and propylene glycol. Penetration enhancers may be incorporated; see, for example, J Pharm Sci, 88 (10), 955-958 by Finnin and Morgan (October 1999). Other means of topical administration include delivery by electroporation, iontophoresis, phonophoresis, sonophoresis and microneedle or needle-free injection.

Formulations for topical administration may be formulated to be immediate and/or modified release. Modified release formulations include delayed-, sustained-, pulsed-, controlled-, targeted and programmed release.

### vii) Rectal/Intravaginal Administration

Compounds of the invention may be administered rectally or vaginally, for example, in the form of a suppository, pessary, or enema. Cocoa butter is a traditional suppository base, but various alternatives may be used as appropriate. Formulations for rectal/vaginal administration may be formulated to be immediate and/or modified release. Modified release formulations include delayed-, sustained-, pulsed-, controlled-, targeted and programmed release.

### viii) Ocular Administration

Compounds of the invention may also be administered directly to the eye or ear, typically in the form of drops of a micronized suspension or solution in isotonic, pH-adjusted, sterile saline. Other formulations suitable for ocular and aural administration include ointments, biodegradable (e.g. absorbable gel sponges, collagen) and nonbiodegradable (e.g. silicone) implants, wafers, lenses and particulate or vesicular systems, such as niosomes or liposomes. A polymer such as crossed-linked polyacrylic acid, polyvinylalcohol, hyaluronic acid, a cellulosic polymer, for example, hydroxypropylmethylcellulose, hydroxyethylcellulose, or methyl cellulose, or a heteropolysaccharide polymer, for example, gelan gum, may be incorporated together with a preservative, such as benzalkonium chloride. Such formulations may also be delivered by iontophoresis.

Formulations for ocular/aural administration may be formulated to be immediate and/or modified release. Modified release formulations include delayed-, sustained-, pulsed-, controlled-, targeted, or programmed release.

### ix) Other Technologies

Compounds of the invention may be combined with soluble macromolecular entities, such as cyclodextrin and suitable derivatives thereof or polyethylene glycol-containing polymers, in order to improve their solubility, dissolution rate, taste-masking, bioavailability and/or stability for use in any of the aforementioned modes of administration.

The amount of the active compound administered will be dependent on the subject being treated, the severity of the disorder or condition, the rate of administration, the disposition of the compound and the discretion of the prescribing physician. However, an effective dosage is typically in the range of 0.01-3000 mg, more preferably 0.5-1000 mg of active ingredient or the equivalent amount of a pharmaceutically acceptable salt thereof per day. Daily dosage may be administered in one or more treatments, preferably from 1 to 4 treatments, per day.

Preferably, the the pharmaceutical compositions of the invention are made up in a form suitable for oral, inhalation or topical administration, being particularly preferred oral or inhalation administration.

The pharmaceutical formulations may conveniently be presented in unit dosage form and may be prepared by any of the methods well known in the art of pharmacy. Preferably the composition is in unit dosage form, for example a tablet, capsule or metered aerosol dose, so that the patient may administer a single dose.

The amount of each active which is required to achieve a therapeutic effect will, of course, vary with the particular active, the route of administration, the subject under treatment, and the particular disorder or disease being treated.

The following preparations forms are cited as formulation examples:

### Formulation Examples

### Formulation Example 1 (Oral suspension)

| **Ingredient** | **Amount** |
|---|---|
| Active Compound | 3 mg |
| Citric acid | 0,5 g |
| Sodium chloride | 2,0 g |
| Methyl paraben | 0,1 g |
| Granulated sugar | 25 g |
| Sorbitol (70% solution) | 11 g |
| Veegum K | 1,0 g |
| Flavoring | 0,02 g |
| Dye | 0,5 mg |
| Distilled water | q.s. to 100 mL |

### Formulation Example 2 (Hard gelatine capsule for oral administration)

| **Ingredient** | **Amount** |
|---|---|
| Active Compound | 1 mg |
| Lactose | 150 mg |
| Magnesium stearate | 3 mg |

### Formulation Example 3 (Gelatin cartridge for inhalation)

| **Ingredient** | **Amount** |
|---|---|
| Active Compound (micronized) | 0,2 mg |
| Lactose | 25 mg |

### Formulation Example 4 (Formulation for inhalation with a DPI)

| **Ingredient** | **Amount** |
|---|---|
| Active Compound (micronized) | 15 mg |
| Lactose | 3000 mg |

### Formulation Example 5 (Formulation for a MDI)

| **Ingredient** | **Amount** |
|---|---|
| Active Compound (micronized) | 10 g |
| 1,1,1,2,3,3,3-heptafluoro-n-propane | q.s. to 200 mL |

Modifications, which do not affect, alter, change or modify the essential aspects of the compounds, combinations or pharmaceutical compositions described, are included within the scope of the present invention.

Modifications, which do not affect, alter, change or modify the essential aspects of the compounds, combinations or pharmaceutical compositions described, are included within the scope of the present invention.

## Claims

1. A compound of formula (I), or a pharmaceutically acceptable salt, or solvate, or N-oxide, or stereoisomer or deuterated derivative thereof: 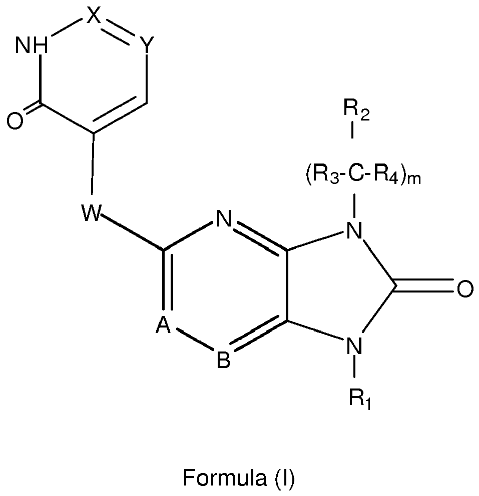 wherein,
m is 0 or an integer from 1 to 3;
X and Y each independently represent a nitrogen atom or a -CR₅ group, wherein at least one of X and Y represents a -CR₅ group;
A and B each independently represent a nitrogen atom or a -CR₆ group, wherein at least one of A and B represents a -CR₆ group;
W represents a linker selected from a -NR₇- group, a -(CR₈R₉)- group, -O- or -S-;
R₁ represents a hydrogen atom, a linear or branched C₁-C₆ alkyl group, a C₁-C₄ haloalkyl group, a C₁-C₄ hydroxyalkyl group, a C₁-C₄ alkoxy group, a C₃-C₁₀ cycloalkyl group, a C₃-C₁₀ cycloalkenyl group, a monocyclic or bicyclic C₆-C₁₄ aryl group, a 5- to 14- membered heteroaryl group containing at least one heteroatom selected from O, S and N, or a 5- to 14- membered heterocyclyl group containing at least one heteroatom selected from O, S and N,
wherein the cycloalkyl, cycloalkenyl, aryl, heteroaryl, and heterocyclyl groups are unsubstituted or substituted by one or more substituents selected from a halogen atom, a cyano group, a linear or branched C₁-C₆ alkyl group, a C₁-C₄ haloalkyl group, a C₁-C₄ hydroxyalkyl group, a C₁-C₄ alkylsulfonyl group, a C₃-C₇ cycloalkyl group, a phenyl group, a pyridyl group, a pyrimidinyl group or a piperidyl group;
R₂ and R₆ each independently represent a hydrogen atom, a halogen atom, a cyano group, a linear or branched C₁-C₆ alkyl group, a C₁-C₄ haloalkyl group, a C₁-C₄ hydroxyalkyl group, a C₃-C₁₀ cycloalkyl group, a C₃-C₁₀ cycloalkenyl group, a monocyclic or bicyclic C₆-C₁₄ aryl group, a 5- to 14- membered heteroaryl group containing at least one heteroatom selected from O, S and N, a 5- to 14- membered heterocyclyl group containing at least one heteroatom selected from O, S and N, or a bicyclyl group which is a monocyclic C₆-C₉ aryl or 5- to 9- membered heteroaryl group fused to a 5- to 9- membered cycloalkyl or heterocyclyl group, said heteroaryl or heterocyclyl group containing at least one heteroatom selected from O, S and N,
wherein the cycloalkyl, cycloalkenyl, aryl, heteroaryl, and heterocyclyl groups, and the bicyclyl group which is a monocyclic C₆-C₉ aryl or 5- to 9- membered heteroaryl group fused to a 5- to 9- membered cycloalkyl or heterocyclyl group are unsubstituted or substituted by one or more substituents selected from a halogen atom, a cyano group, a linear or branched C₁-C₆ alkyl group, a C₁-C₄ haloalkyl group, a C₁-C₄ hydroxyalkyl group, a C₃-C₇ cycloalkyl group, a monocyclic or bicyclic C₆-C₁₄ aryl group, a 5- to 14- membered heteroaryl group containing at least one heteroatom selected from O, S and N, a 5- to 14-membered heterocyclyl group containing at least one heteroatom selected from O, S and N, a -(CH₂)₁₋₃CN group, a -(CH₂)ₙOR₁₁ group, a -NR₁₀R₁₁ group, a -NR₁₀C(O)-(CH₂)ₙ-R₁₁ group, a -NR₁₀C(O)-(CH₂)ₙ-NR₁₁R₁₂ group, a -C(O)-(CH₂)₁₋₃-CN group, a -C(O)-(CH₂)ₙ-R₁₁ group, a -C(O)-(CH₂)ₙ-NR₁₀R₁₁ group, a -S(O)₂(CH₂)ₙR₁₁ group, a -S(O)₂(CH₂)ₙNR₁₀R₁₁ group, or a -NR₁₀S(O)₂(CH₂)ₙNR₁₁R₁₂ group; wherein each n is 0, 1 or 2;
R₃ and R₄ each independently represent a hydrogen atom, a C₁-C₄ haloalkyl group, a C₁-C₄ hydroxyalkyl group or a linear or branched C₁-C₆ alkyl group, which alkyl group is unsubstituted or substituted by one or more substituents selected from a C₁-C₄ alkoxy group, a cyano group, a C₃-C₇ cycloalkyl group, a phenyl group, a pyridyl group, a pyrimidinyl group or a piperidyl group;
R₅ represents a hydrogen atom, a halogen atom, a cyano group, a linear or branched C₁-C₆ alkyl group, a C₁-C₄ haloalkyl group, a C₁-C₄ hydroxyalkyl group, a C₃-C₁₀ cycloalkyl group, a C₃-C₁₀ cycloalkenyl group, a monocyclic or bicyclic C₆-C₁₄ aryl group, a 5- to 14- membered heteroaryl group containing at least one heteroatom selected from O, S and N, or a 5- to 14- membered heterocyclyl group containing at least one heteroatom selected from O, S and N,
wherein the cycloalkyl, cycloalkenyl, aryl, heteroaryl, and heterocyclyl groups are unsubstituted or substituted by one or more substituents selected from a halogen atom, a cyano group, a linear or branched C₁-C₆ alkyl group, a C₁-C₄ haloalkyl group, a C₁-C₄ hydroxyalkyl group, a C₃-C₇ cycloalkyl, a phenyl group, a pyridyl group, a pyrimidinyl group, a piperidyl group or a -C(O)-(CH₂)ₙ-NR₁₀R₁₁ group; wherein n is 0, 1 or 2;
R₇, R₈ and R₉ each independently represent a hydrogen atom, a C₁-C₄ haloalkyl group, a C₁-C₄ hydroxyalkyl group or a linear or branched C₁-C₆ alkyl group, which alkyl group is unsubstituted or substituted by one or more substituents selected from a C₁-C₄ alkoxy group, a cyano group, a C₃-C₇ cycloalkyl group, a phenyl group, a pyridyl group, a pyrimidinyl group or a piperidyl group;
R₁₀, R₁₁ and R₁₂ each independently represent a hydrogen atom, a C₁-C₄ haloalkyl group, a C₁-C₄ hydroxyalkyl group or a linear or branched C₁-C₆ alkyl group, which alkyl group is unsubstituted or substituted by one or more substituents selected from a C₁-C₄ alkoxy group, a cyano group, a C₃-C₇ cycloalkyl group, a phenyl group, a pyridyl group, a pyrimidinyl group or a piperidyl group.

2. A compound according to claim 1, wherein W represents a linker selected from a -NR₇- group or a -(CR₈R₉)- group wherein R₇, R₈ and R₉ are as defined in claim 1; and wherein preferably W represents a -NR₇- group wherein R₇ is as defined in claim 1.

3. A compound according to claim 1 or claim 2, wherein R₁ represents a hydrogen atom, a linear or branched C₁-C₆ alkyl group, a C₁-C₄ haloalkyl group, a C₁-C₄ hydroxyalkyl group, a C₃-C₇ cycloalkyl group, a phenyl group, a pyridyl group, a pyrimidinyl group or a piperidinyl group;
and
wherein R₁ preferably represents a hydrogen atom, a linear or branched C₁-C₄ alkyl group, a C₁-C₄ haloalkyl group, a C₁-C₄ hydroxyalkyl group, a C₃-C₇ cycloalkyl group, a phenyl group or a pyridyl group;
and
wherein R₁ more preferably represents a hydrogen atom, a linear or branched C₁-C₃ alkyl group, a C₁-C₃ haloalkyl group or a C₁-C₃ hydroxyalkyl group.

4. A compound according to any one of the preceding claims, wherein R₂ represents a linear or branched C₁-C₆ alkyl group, a C₁-C₄ haloalkyl group, a C₁-C₄ hydroxyalkyl group, a C₃-C₇ cycloalkyl group, a monocyclic or bicyclic C₆-C₁₄aryl group, a 5- to 7- membered heteroaryl group containing one, two or three heteroatoms selected from O, S and N, a 5- to 7- membered heterocyclyl group containing one, two or three heteroatoms selected from O, S and N, or a bicyclyl group which is a monocyclic C₆-C₉ aryl or 5- to 9- membered heteroaryl group bonded fused to a 5- to 9- membered cycloalkyl or heterocyclyl group, said heteroaryl or heterocyclyl group containing one, two or three heteroatoms selected from O, S and N,
wherein the cycloalkyl, cycloalkenyl, aryl, heteroaryl, and heterocyclyl groups and bicyclyl group which is a monocyclic C₆-C₉ aryl or 5- to 9- membered heteroaryl group fused to a 5- to 9- membered cycloalkyl or heterocyclyl group are unsubstituted or substituted by one or more substituents selected from a halogen atom, a cyano group, a linear or branched C₁-C₆ alkyl group, a C₁-C₄ haloalkyl group, a C₁-C₄ hydroxyalkyl group, a C₃-C₇ cycloalkyl group, a monocyclic or bicyclic C₆-C₁₄ aryl group, a 5- to 14- membered heteroaryl group containing at least one heteroatom selected from O, S and N, a 5- to 14-membered heterocyclyl group containing at least one heteroatom selected from O, S and N, a -(CH₂)₁₋₃CN group, a -(CH₂)ₙOR₁₁ group, a -NR₁₀R₁₁ group, a -NR₁₀C(O)-(CH₂)ₙ-R₁₁ group, a -NR₁₀C(O)-(CH₂)ₙ-NR₁₁R₁₂ group, a -C(O)-(CH₂)₁₋₃-CN group, a -C(O)-(CH₂)ₙ-R₁₁ group, a -C(O)-(CH₂)ₙ-NR₁₀R₁₁ group, a -S(O)₂(CH₂)ₙR₁₁ group, a -S(O)₂(CH₂)ₙNR₁₀R₁₁ group, or a -NR₁₀S(O)₂(CH₂)ₙNR₁₁R₁₂ group; wherein each n is 0, 1 or 2; and wherein R₁₀, R₁₁ and R₁₂ are as defined in claim 1.

5. A compound according to claim 4, wherein R₂ represents a linear or branched C₁-C₆ alkyl group, a C₁-C₄ haloalkyl group, a C₁-C₄ hydroxyalkyl group, a C₃-C₇ cycloalkyl group, a phenyl group, a pyridyl group, a pyrimidinyl group, a pyrrolidinyl group, a piperidyl group, a tetrahydropyranyl group, a morpholinyl group, or a tetrahydronaphthalenyl group,
wherein the cycloalkyl, phenyl, pyridyl, pyrimidinyl, pyrrolidinyl, piperidyl, tetrahydropyranyl, morpholinyl or tetrahydronaphthalenyl groups are unsubstituted or substituted by one, two or three substituents selected from a halogen atom, a cyano group, a linear or branched C₁-C₆ alkyl group, a C₁-C₄ haloalkyl group, a C₁-C₄ hydroxyalkyl group, a C₃-C₇ cycloalkyl group, a phenyl group, a pyridyl group, a pyrimidinyl group, a piperidyl group, a -(CH₂)₁₋₃CN group, a -(CH₂)ₙOR₁₁ group, a -NR₁₀R₁₁ group, a -NR₁₀C(O)-(CH₂)ₙ-R₁₁ group, a -NR₁₀C(O)-(CH₂)ₙ-NR₁₁R₁₂ group, a -C(O)-(CH₂)₁₋₃-CN group, a -C(O)-(CH₂)ₙ-R₁₁ group, a -C(O)-(CH₂)ₙ-NR₁₀R₁₁ group, a -S(O)₂(CH₂)ₙR₁₁ group, a -S(O)(CH₂)ₙNR₁₀R₁₁ group, or a -NR₁₀S(O)(CH₂)ₙNR₁₁R₁₂ group; wherein each n is 0, 1 or 2; and wherein R₁₀, R₁₁ and R₁₂ each independently represent a hydrogen atom or a linear or branched C₁-C₃ alkyl group;

6. A compound according to any one of the preceding claims, wherein R₃ and R₄ each independently represent a hydrogen atom or a linear or branched C₁-C₆ alkyl group, which alkyl group is unsubstituted or substituted by a C₁-C₂ alkoxy group;
and
wherein R₃ and R₄ preferably each independently represent a hydrogen atom or a linear or branched C₁-C₃ alkyl group;
and
wherein R₃ and R₄ more preferably each independently represent a hydrogen atom or a methyl group.

7. A compound according to any one of the preceding claims, wherein R₅ represents a hydrogen atom, a halogen atom, a cyano group, a linear or branched C₁-C₄ alkyl group, a C₁-C₄ haloalkyl group, a C₁-C₄ hydroxyalkyl group, a C₃-C₇ cycloalkyl group, a phenyl group, a pyridyl group, a pyrimidinyl group, a pyrrolidinyl group, a piperidyl group, a tetrahydropyranyl group or a morpholinyl group,
wherein the phenyl, pyridyl, pyrimidinyl, pyrrolidinyl, piperidyl, tetrahydropyranyl or morpholinyl groups are unsubstituted or substituted by one, two or three substituents selected from a halogen atom, a cyano group, a linear or branched C₁-C₆ alkyl group, a C₁-C₄ haloalkyl group, a C₁-C₄ hydroxyalkyl group, a C₃-C₇ cycloalkyl, a phenyl group, a pyridyl group, a pyrimidinyl group, a piperidyl group, or a -C(O)-(CH₂)ₙ-NR₁₀R₁₁ group; wherein n is 0, 1 or 2; and wherein R₁₀ and R₁₁ are as defined in claim 1.

8. A compound according to any one of the preceding claims, wherein R₆ represents a hydrogen atom, a halogen atom, a cyano group, a linear or branched C₁-C₄ alkyl group, a C₁-C₄ haloalkyl group, a C₁-C₄ hydroxyalkyl group, a C₃-C₇ cycloalkyl group, a phenyl group, a pyridyl group, a pyrimidinyl group, a pyrrolidinyl group, a piperidyl group, a tetrahydropyranyl group, a morpholinyl group, or a tetrahydronaphthalenyl group,
wherein the cycloalkyl, phenyl, pyridyl, pyrimidinyl, pyrrolidinyl, piperidyl, tetrahydropyranyl, morpholinyl or tetrahydronaphthalenyl groups are unsubstituted or substituted by one, two or three substituents selected from a halogen atom, a cyano group, a linear or branched C₁-C₆ alkyl group, a C₁-C₄ haloalkyl group, a C₁-C₄ hydroxyalkyl group, a C₃-C₇ cycloalkyl group, a phenyl group, a pyridyl group, a pyrimidinyl group, a piperidyl group, a -(CH₂)₁₋₃CN group, a -(CH₂)ₙOR₁₁ group, a -NR₁₀R₁₁ group, a -NR₁₀C(O)-(CH₂)ₙ-R₁₁ group, a -NR₁₀C(O)-(CH₂)ₙ-NR₁₁R₁₂ group, a -C(O)-(CH₂)₁₋₃-CN group, a -C(O)-(CH₂)ₙ-R₁₁ group, a -C(O)-(CH₂)ₙ-NR₁₀R₁₁ group, a -S(O)₂(CH₂)ₙR₁₁ group, a -S(O)₂(CH₂)ₙNR₁₀R₁₁ group, or a -NR₁₀S(O)₂(CH₂)ₙNR₁₁R₁₂ group; wherein each n is 0, 1 or 2; and wherein R₁₀, R₁₁ and R₁₂ each independently represent a hydrogen atom or a linear or branched C₁-C₃ alkyl group.

9. A compound according to any one of the preceding claims, wherein R₇ represents a hydrogen atom or a linear or branched C₁-C₆ alkyl group, which alkyl group is unsubstituted or substituted by a C₁-C₂ alkoxy group;
and
wherein R₇ preferably represents a hydrogen atom or a linear or branched C₁-C₃ alkyl group;
and
wherein R₇ more preferably represents a hydrogen atom or a methyl group.

10. A compound according to claim 1, wherein:
m is 0 or 1;
X is a nitrogen atom and Y is a -CR₅ group; or Y is a nitrogen atom and X is a -CR₅ group; or both X and Y are a -CR₅ group;
A is a nitrogen atom and B is a -CR₆ group; or B is a nitrogen atom and A is a -CR₆ group; or both A and B are a -CR₆ group;
W represents a linker selected from a -NR₇- group, a -(CR₈R₉)- group, -O- or -S-;
R₁ represents a hydrogen atom, a linear or branched C₁-C₄ alkyl group, a C₁-C₄ haloalkyl group, a C₁-C₄ hydroxyalkyl group, a C₃-C₇ cycloalkyl group, a phenyl group or a pyridyl group;
R₂ represents a linear or branched C₁-C₆ alkyl group, a C₁-C₄ haloalkyl group, a C₁-C₄ hydroxyalkyl group, a C₃-C₇ cycloalkyl group, a monocyclic or bicyclic C₆-C₁₄ aryl group, a 5- to 9- membered heteroaryl group containing one, two or three heteroatoms selected from O, S and N, a 5- to 9- membered heterocyclyl group containing one, two or three heteroatoms selected from O, S and N, or a bicyclyl group which is a monocyclic C₆-C₉ aryl or 5- to 9- membered heteroaryl group fused to a 5- to 9-membered cycloalkyl or heterocyclyl group, said heteroaryl or heterocyclyl groups containing one, two or three heteroatoms selected from O, S and N,
wherein the cycloalkyl, aryl, heteroaryl, and heterocyclyl groups and bicyclyl group which is a monocyclic C₆-C₉ aryl or 5- to 9- membered heteroaryl group fused to a 5- to 9- membered cycloalkyl or heterocyclyl group are unsubstituted or substituted by one or more substituents selected from a halogen atom, a cyano group, a linear or branched C₁-C₆ alkyl group, a C₁-C₄ haloalkyl group, a C₁-C₄ hydroxyalkyl group, a C₃-C₇ cycloalkyl group, a monocyclic or bicyclic C₆-C₁₄ aryl group, a 5- to 14- membered heteroaryl group containing at least one heteroatom selected from O, S and N, a 5- to 14- membered heterocyclyl group containing at least one heteroatom selected from O, S and N, a -(CH₂)₁₋₃CN group, a -(CH₂)ₙOR₁₁ group, a -NR₁₀R₁₁ group, a -NR₁₀C(O)-(CH₂)ₙ-R₁₁ group, a -NR₁₀C(O)-(CH₂)ₙ₋NR₁₁R₁₂ group, a -C(O)-(CH₂)₁₋₃-CN group, a -C(O)-(CH₂)ₙ-R₁₁ group, a -C(O)-(CH₂)ₙ-NR₁₀R₁₁ group, a -S(O)₂(CH₂)ₙR₁₁ group, a -S(O)₂(CH₂)ₙNR₁₀R₁₁ group, or a -NR₁₀S(O)₂(CH₂)ₙNR₁₁R₁₂ group; wherein each n is 0, 1 or 2;
R₃ and R₄ each independently represent a hydrogen atom or a linear or branched C₁-C₃ alkyl group;
R₅ represent a hydrogen atom, a halogen atom, a cyano group, a linear or branched C₁-C₄ alkyl group, a C₁-C₄ haloalkyl group, a C₁-C₄ hydroxyalkyl group, a C₃-C₇ cycloalkyl group, a monocyclic or bicyclic C₆-C₁₄aryl group, a 5- to 9- membered heteroaryl group containing one, two or three heteroatoms selected from O, S and N, a 5- to 9- membered heterocyclyl group containing one, two or three heteroatoms selected from O, S and N,
wherein the cycloalkyl, aryl, heteroaryl, and heterocyclyl groups are unsubstituted or substituted by one, two or three substituents selected from a halogen atom, a cyano group, a linear or branched C₁-C₆ alkyl group, a C₁-C₄ haloalkyl group, a C₁-C₄ hydroxyalkyl group, a C₃-C₇ cycloalkyl, a phenyl group, a pyridyl group, a pyrimidinyl group, a piperidyl group, or a -C(O)-(CH₂)ₙ-NR₁₀R₁₁ group; wherein n is 0, 1 or 2;
R₆ represents a hydrogen atom, a halogen atom, a cyano group, a linear or branched C₁-C₄ alkyl group, a C₁-C₄ haloalkyl group, a C₁-C₄ hydroxyalkyl group, a C₃-C₇ cycloalkyl group, a monocyclic or bicyclic C₆-C₁₄ aryl group, a 5- to 9- membered heteroaryl group containing one, two or three heteroatoms selected from O, S and N, a 5- to 9- membered heterocyclyl group containing one, two or three heteroatoms selected from O, S and N,
wherein the cycloalkyl, aryl, heteroaryl, and heterocyclyl groups are unsubstituted or substituted by one, two or three substituents selected from a halogen atom, a cyano group, a linear or branched C₁-C₆ alkyl group, a C₁-C₄ haloalkyl group, a C₁-C₄ hydroxyalkyl group, a C₃-C₇ cycloalkyl group, a phenyl group, a pyridyl group, a pyrimidinyl group, a piperidyl group, a -(CH₂)₁₋₃CN group, a -(CH₂)ₙOR₁₁ group, a -NR₁₀R₁₁ group, a -NR₁₀C(O)-(CH₂)ₙ-R₁₁ group, a -NR₁₀C(O)-(CH₂)ₙ-NR₁₁ R₁₂ group, a -C(O)-(CH₂)₁₋₃-CN group, a -C(O)-(CH₂)ₙ-R₁₁ group, a -C(O)-(CH₂)ₙ-NR₁₀R₁₁ group, a -S(O)₂(CH₂)ₙR₁₁ group, a -S(O)₂(CH₂)ₙNR₁₀R₁₁ group, or a -NR₁₀S(O)₂(CH₂)ₙNR₁₁R₁₂ group; wherein each n is 0, 1 or 2;
R₇ represents a hydrogen atom or a linear or branched C₁-C₆ alkyl group, which alkyl group is unsubstituted or substituted by a C₁-C₂ alkoxy group;
R₈ and R₉ each independently represent a hydrogen atom or a linear or branched C₁-C₃ alkyl group;
R₁₀, R₁₁ and R₁₂ each independently represent a hydrogen atom or a linear or branched C₁-C₃ alkyl group.

11. A compound according to claim 10, wherein:
m is 0 or 1;
X is a nitrogen atom and Y is a -CR₅ group; or Y is a nitrogen atom and X is a -CR₅ group; or both X and Y are a -CR₅ group;
A is a nitrogen atom and B is a -CR₆ group; or B is a nitrogen atom and A is a -CR₆ group; or both A and B are a -CR₆ group;
W represents a linker selected from a -NR₇- group or a -(CR₈R₉)- group;
R₁ represents a hydrogen atom, a linear or branched C₁-C₃ alkyl group, a C₁-C₃ haloalkyl group or a C₁-C₃ hydroxyalkyl group;
R₂ represents a linear or branched C₁-C₄ alkyl group, a C₁-C₄ haloalkyl group, a C₁-C₄ hydroxyalkyl group, a C₃-C₇ cycloalkyl group, a phenyl group, a pyridyl group, a pyrimidinyl group, a pyrrolidinyl group, a piperidyl group, a tetrahydropyranyl group or a morpholinyl group,
wherein the cycloalkyl, phenyl, pyridyl, pyrimidinyl, pyrrolidinyl, piperidinyl , tetrahydropyranyl or morpholinyl groups are unsubstituted or substituted by one, two or three substituents selected from a halogen atom, a cyano group, a linear or branched C₁-C₃ alkyl group, a C₁-C₄ haloalkyl group, a C₁-C₄ hydroxyalkyl group, a C₃-C₇ cycloalkyl, a -(CH₂)₁₋₃CN group, a -(CH₂)ₙOR₁₁ group, a -NR₁₀R₁₁ group, a -C(O)-(CH₂)₁₋₃-CN group, a -C(O)-(CH₂)ₙ-R₁₀ group, a -C(O)-(CH₂)ₙ-NR₁₀R₁₁ group, a -S(O)₂(CH₂)ₙR₁₁ group or a - S(O)(CH₂)ₙNR₁₀R₁₁ group; wherein each n is 0, 1 or 2; and wherein R₁₀ is a hydrogen atom, a cyano group, a linear or branched C₁-C₃ alkyl group, a C₁-C₄ haloalkyl group or a C₃-C₇ cycloalkyl group and wherein R₁₁ is a hydrogen atom or a linear or branched C₁-C₃ alkyl group;
R₃ and R₄ each independently represent a hydrogen atom or a linear or branched C₁-C₃ alkyl group;
R₅ represents a hydrogen atom, a halogen atom, a cyano group, a linear or branched C₁-C₃ alkyl group, a C₁-C₄ haloalkyl group, a C₁-C₄ hydroxyalkyl group, a C₃-C₇ cycloalkyl group, a phenyl group, a pyridyl group, a pyrimidinyl group, a pyrrolidinyl group, a piperidyl group, a tetrahydropyranyl group or a morpholinyl group;
R₆ represents a hydrogen atom, a halogen atom, a cyano group, a linear or branched C₁-C₃ alkyl group, a C₁-C₄ haloalkyl group, a C₁-C₄ hydroxyalkyl group, a C₃-C₇ cycloalkyl group, a phenyl group, a pyridyl group, a pyrimidinyl group, a pyrrolidinyl group, a piperidyl group, a tetrahydropyranyl group or a morpholinyl group;
R₇ represents a hydrogen atom or a linear or branched C₁-C₃ alkyl group;
R₈ and R₉ each independently represent a hydrogen atom or a linear or branched C₁-C₃ alkyl group;

12. A compound according to claim 1, wherein:
m is 0 or 1;
X is a nitrogen atom and Y is a -CR₅ group; or Y is a nitrogen atom and X is a -CR₅ group; or both X and Y are a -CR₅ group;
A is a nitrogen atom and B is a -CR₆ group; or B is a nitrogen atom and A is a -CR₆ group; or both A and B are a -CR₆ group;
W represents a -NR₇- group;
R₁ represents a hydrogen atom, a C₁-C₃ haloalkyl group, a C₁-C₃ hydroxyalkyl group or a linear or branched C₁-C₃ alkyl group;
R₂ represents a C₃-C₇ cycloalkyl group, a phenyl group, a pyridyl group, a pyrimidinyl group, a tetrahydropyranyl group, or a tetrahydronaphthalenyl group,
wherein the cycloalkyl, phenyl, pyridyl, pyrimidinyl,tetrahydropyranyl and tetrahydronaphthalenyl groups are unsubstituted or substituted by one, two or three substituents selected from a halogen atom, a linear or branched C₁-C₃ alkyl group, a C₁-C₃ haloalkyl group, a -(CH₂)₁₋₃CN group or a -(CH₂)ₙOR₁₁ group; wherein each n is 0, 1 or 2;
R₃ and R₄ each independently represent a hydrogen atom or a methyl group;
R₅ represents a hydrogen atom, a halogen atom, a linear or branched C₁-C₃ alkyl group, a C₁-C₃ haloalkyl group or a C₃-C₇ cycloalkyl group;
R₆ represents a hydrogen atom, a halogen atom, a linear or branched C₁-C₃ alkyl group, a C₁-C₃ haloalkyl group, a C₃-C₇ cycloalkyl group or a morpholinyl group;
R₇ represents a hydrogen atom or a methyl group;
R₁₁ represents a hydrogen atom or a methyl group.

13. A compound according to claim 1 which is one of:
2-(2-Oxo-1,2-dihydropyridin-3-ylamino)-9-(tetrahydro-2H-pyran-4-yl)-7H-purin-8(9H)-one;
2-(5-chloro-2-oxo-1,2-dihydropyridin-3-ylamino)-9-(tetrahydro-2H-pyran-4-yl)-7H-purin-8(9H)-one;
2-(5-fluoro-2-oxo-1,2-dihydropyridin-3-ylamino)-9-(tetrahydro-2H-pyran-4-yl)-7H-purin-8(9H)-one;
2-(5-methyl-2-oxo-1,2-dihydropyridin-3-ylamino)-9-(tetrahydro-2H-pyran-4-yl)-7H-purin-8(9H)-one;
2-(5-(difluoromethyl)-2-oxo-1,2-dihydropyridin-3-ylamino)-9-(tetrahydro-2H-pyran-4-yl)-7H-purin-8(9H)-one;
(R)-9-(1-(5-Fluoropyridin-2-yl)ethyl)-2-(2-oxo-1,2-dihydropyridin-3-ylamino)-7H-purin-8(9H)-one;
2-((1r,4r)-4-(8-Oxo-2-(2-oxo-1,2-dihydropyridin-3-ylamino)-7H-purin-9(8H)-yl) cyclohexyl)acetonitrile;
7-methyl-2-(2-oxo-1,2-dihydropyridin-3-ylamino)-9-(tetrahydro-2H-pyran-4-yl)-7H-purin-8(9H)-one;
7-Methyl-2-(methyl(2-oxo-1,2-dihydropyridin-3-yl)amino)-9-(tetrahydro-2H-pyran-4-yl)-7H-purin-8(9H)-one;
7-(2-hydroxyethyl)-2-(2-oxo-1,2-dihydropyridin-3-ylamino)-9-(tetrahydro-2H-pyran-4-yl)-7H-purin-8(9H)-one;
5-(2-Oxo-1,2-dihydropyridin-3-ylamino)-3-(tetrahydro-2H-pyran-4-yl)-1H-imidazo [4,5-b]pyridin-2(3H)-one;
6-fluoro-5-(2-oxo-1,2-dihydropyridin-3-ylamino)-3-(tetrahydro-2H-pyran-4-yl)-1H-imidazo[4,5-b]pyridin-2(3H)-one;
6-morpholino-2-(2-oxo-1,2-dihydropyridin-3-ylamino)-9-(tetrahydro-2H-pyran-4-yl)-7H-purin-8(9H)-one;
2-(6-Oxo-1,6-dihydropyrimidin-5-ylamino)-9-(tetrahydro-2H-pyran-4-yl)-7H-purin-8(9H)-one;
(R)-9-(1-(5-fluoropyrimidin-2-yl)ethyl)-2-(6-oxo-1,6-dihydropyrimidin-5-ylamino)-7H-purin-8(9H)-one;
2-(3-oxo-2,3-dihydropyridazin-4-ylamino)-9-(tetrahydro-2H-pyran-4-yl)-7H-purin-8(9H)-one;
2-(6-chloro-3-oxo-2,3-dihydropyridazin-4-ylamino)-9-(tetrahydro-2H-pyran-4-yl)-7H-purin-8(9H)-one;
(2-(1r,4r)-4-{2-[(5-Chloro-2-oxo-1,2-dihydropyridin-3-yl)amino]-8-oxo-7,8-dihydro-9H-purin-9-yl}cyclohexyl)acetonitrile;
(R)-2-(5-fluoro-2-oxo-1,2-dihydropyridin-3-ylamino)-9-(1-(5-fluoropyridin-2-yl)ethyl)-7H-purin-8(9H)-one;
(R)-9-(1-(5-fluoropyridin-2-yl)ethyl)-2-(5-methyl-2-oxo-1,2-dihydropyridin-3-ylamino)-7H-purin-8(9H)-one;
(R)-2-(5-chloro-2-oxo-1,2-dihydropyridin-3-ylamino)-9-(1-(5-fluoropyridin-2-yl)ethyl)-7H-purin-8(9H)-one;
2-(5-fluoro-2-oxo-1,2-dihydropyridin-3-ylamino)-9-((1r,4r)-4-hydroxycyclohexyl)-7H-purin-8(9H)-one;
9-((1R,4R)-5,7-difluoro-4-hydroxy-1,2,3,4-tetrahydronaphtha!en-1-yl)-2-(2-oxo-1,2-dihydropyridin-3-ylamino)-7H-purin-8(9H)-one;
9-((1S,2R)-2-methylcyclohexyl)-2-(2-oxo-1,2-dihydropyridin-3-ylamino)-7H-purin-8(9H)-one;
9-((1r,4r)-4-(hydroxymethyl)cyclohexyl)-2-(2-oxo-1,2-dihydropyridin-3-ylamino)-7H-purin-8(9H)-one;
or a pharmaceutically acceptable salt, or solvate, or N-oxide, or stereoisomer or deuterated derivative thereof.

14. A compound according to any one of claims 1 to 13, for use in the treatment of a pathological condition or disease susceptible to amelioration by inhibition of Janus Kinases.

15. A compound according to claim 14, wherein the pathological condition or disease is selected from myeloproliferative disorders, leukemia, lymphoid malignancies and solid tumors; bone marrow and organ transplant rejection; immune-mediated diseases and inflammatory diseases.

16. A compound according to claims 14 or 15, wherein the pathological condition or disease is selected from rheumatoid arthritis, multiple sclerosis, inflammatory bowel disease, dry eye, uveitis, allergic conjunctivitis, allergic rhinitis, asthma, chronic obstructive pulmonary disease (COPD), atopic dermatitis and psoriasis.

17. A pharmaceutical composition comprising a compound as defined in any one of claims 1 to 13 in association with a pharmaceutically acceptable diluent or carrier.

18. Use of a compound as defined in any one of claims 1 to 13, for the manufacture of a medicament for the treatment of a pathological condition or disease as defined in any one of claims 14 to 16.

19. A method for treating a subject afflicted with a pathological condition or disease as defined in any one of claims 14 to 16, which comprises administering to said subject a therapeutically effective amount of a compound as defined in any one of claims 1 to 13, or a pharmaceutical composition as defined in claim 17.

20. A combination product comprising (i) a compound as defined in any one of claims 1 to 13; and (ii) another compound selected from:
a) Dyhydrofolate reductase inhibitors, such as Methotrexate or CH-1504;
b) Dihydroorotate dehydrogenase (DHODH) inhibitors such as leflunomide, teriflunomide, or the compounds described in the International Patent Application Nos. WO2008/077639 and WO2009/021696;
c) Immunomodulators such as Glatiramer acetate (Copaxone), Laquinimod or Imiquimod;
d) Inhibitors of DNA synthesis and repair, such as Mitoxantrone or Cladribine;
e) Immunosuppressants, such as Imuran (azathioprine) or Purinethol (6-mercaptopurine or 6-MP);
f) Anti-alpha 4 integrin antibodies, such as Natalizumab (Tysabri) ;
g) Alpha 4 integrin antagonists such as R-1295 , TBC-4746, CDP-323, ELND-002, Firategrast or TMC-2003;
*h)* Corticoids and glucocorticoids such as prednisone or methylprednisolone, fluticasone, mometasone, budesonide, ciclesonide or beta-metasone;
*i)* Fumaric acid esters, such as *BG-12*;
j) Anti-tumor necrosis factor-alpha (Anti-TNF-alpha), such as Infliximab, Adalimumab, or Certolizumab pegol,
k) Soluble Tumor necrosis factor-alpha (TNF-alpha) receptors such as Ethanercept,
I) Anti-CD20 (lymphocyte protein) monoclonal antibodies such as Rituximab, Ocrelizumab Ofatumumab or TRU-015,
m) Anti-CD52 (lymphocyte protein) monoclonal antibodies such as alemtuzumab;
n) Anti-CD25 (lymphocyte protein) such as daclizumab;
o) Anti-CD88 (lymphocyte protein), such as eculizumab or pexilizumab;
p) Anti-Interleukin 6 Receptor (IL-6R), such as tocilizumab;
q) Anti-Interleukin 12 Receptor (IL-12R) / Interleukin 23 Receptor (IL-23R), such as ustekinumab;
r) Calcineurin inhibitors such as cyclosporine A or tacrolimus;
s) Inosine-monophosphate dehydrogenase (IMPDH) inhibitors, such as mycophenolate mophetyl, ribavirin, mizoribine or mycophenolic acid;
t) Cannabinoid receptor agonists such as Sativex;
u) Chemokine CCR1 antagonists such as MLN-3897 or PS-031291;
v) Chemokine CCR2 antagonists such as INCB-8696;
w) Necrosis factor-kappaB (NF-kappaB or NFKB) Activation Inhibitors such as Sulfasalazine, Iguratimod or MLN-0415;
x) Adenosine A_{2A} agonists, such as ATL-313, ATL-146e, CGS-21680, Regadenoson or UK-432,097;
y) Sphingosine-1 (S1 P) phosphate receptor agonists such as fingolimod, BAF-312, or ACT128800;
z) Sphingosine-1 (S1 P) liase inhibitors such as LX2931;
aa) Spleen tyrosine kinase (Syk) inhibitors, such as R-112;
bb) Protein Kinase Inhibitors (PKC) inhibitors, such as NVP-AEB071; cc) Anti-cholinergic agents such as tiotropium or aclidinium;
dd) Beta adrenergic agonists such as formoterol, indacaterol or LAS100977;
ee) Compounds having bifunctional Muscarinic Antagonist-Beta2 Agonist activity (MABAs);
ff) Histamine 1 (H1) receptor antagonists, such as azelastine or ebastine;
gg) Chemoattractant receptor homologous molecule expressed on TH₂ cells (CRTH2) inhibitors, such as OC-459, AZD-1981, ACT-129968, QAV-680;
hh) Vitamin D derivatives like calcipotriol (Daivonex) ;
ii) Anti-inflammatory agents, such as non-steroidal anti-inflammatory drugs (NSAIDs) or selective cyclooxygenase-2 (COX-2) inhibitors such as aceclofenac, diclofenac, ibuprofen, naproxen, apricoxib, celecoxib, cimicoxib, deracoxib, etoricoxib, lumiracoxib, parecoxib sodium, rofecoxib, selenocoxib-1 or valdecoxib;
jj) Anti-allergic agents;
kk) Anti-viral agents;
II) Phosphodiestearase (PDE) III inhibitors;
mm) Phosphosdiesterase (PDE) IV inhibitors such as roflumilast or GRC-4039;
nn) Dual Phosphodiestearase (PDE) III/IV inhibitors;
oo) Xanthine derivatives, such as theophylline or theobromine;
pp) p38 Mitogen-Activated Protein Kinase (p38 MAPK) Inhibitors such as ARRY-797;
qq) Mitogen-activated extracellular signal regulated kinase kinase (MEK) inhibitor, such as ARRY-142886 or ARRY-438162;
rr) Phosphoinositide 3-Kinases (PI3Ks) inhibitors;
ss) Interferons comprising Interferon beta 1 a such as Avonex from Biogen Idec, CinnoVex from CinnaGen and Rebif from EMD Serono, and Interferon beta 1 b such as Betaferon from Schering and Betaseron from Berlex; and
tt) Interferon alpha such as Sumiferon MP;
for simultaneous, separate or sequential use in the treatment of the human or animal body.
